# EUROPEAN PATENT APPLICATION

(11) **EP 4 029 520 A1**
(43) Date of publication of application: **20.07.2022**
(21) Application number: 20863189.5
(22) Date of filing: 09.09.2020
(51) Int. Cl.: A61K 48/00, A61P 1/00, A61P 1/16, A61P 3/04, A61P 3/10, A61P 7/06, A61P 9/00, A61P 9/10, A61P 11/00, A61P 13/12, A61P 27/02, A61P 31/04, A61P 35/00, A61P 37/02, A61P 43/00, C12N 5/10, A61K 47/54, C12N 15/113, C12N 15/87, A61K 31/7088

(54) **GALNAC-OLIGONUCLEOTIDE CONJUGATE FOR LIVER-TARGETED DELIVERY USE, AND METHOD FOR PRODUCING SAME**

(30) Priority: 10.09.2019 JP 2019164564
(71) Applicant: Daiichi Sankyo Company, Limited, Tokyo 103-8426 (JP)
(72) Inventor: KOIZUMI Makoto, Tokyo 103-8426 (JP); IWAMOTO Mitsuhiro, Tokyo 103-8426 (JP); SEKIGUCHI Yukiko, Tokyo 103-8426 (JP); ITO Kentaro, Tokyo 103-8426 (JP)
(74) Representative: Nieuwenhuys, William Francis
(86) International application number: PCT/JP2020/034022
(87) International publication number: WO 2021/049504

(57) **Abstract**

The present invention provides a conjugate of an oligonucleotide having a nucleic acid sequence expected to have a pharmacological effect in hepatic parenchymal cells with a biantennary GalNAc unit, or a pharmaceutically acceptable salt thereof, and a medicament or the like containing the same as an active component.

## Description

### Technical Field

The present invention relates to a biantennary N-acetyl-D-galactosamine (GalNAc) ligand-oligonucleotide conjugate, and a method for producing the conjugate.

### Background Art

Conjugates of nucleic acid therapeutics (such as antisense and siRNA) covalently bound to N-acetyl-D-galactosamine (GalNAc) or the like as a ligand capable of binding to an asialoglycoprotein receptor (ASGPR) have been reported as methods for delivering oligonucleotides to hepatic parenchymal cells of the liver (Non Patent Literature 1 and Patent Literatures 1 to 9). In those reports, one to three GalNAcs are bound to one oligonucleotide. Further, Non Patent Literature 2 describes an example in which two GalNAcs are bound.

In the case of using reverse-phase HPLC in a purification step after solid-phase synthesis of an oligonucleotide, the retention time of oligomers with a short chain length generated in the solid phase synthesis step often approximates the retention time of the target oligomer, and it is very difficult to obtain high-purity refined products on a large scale. In the solid phase synthesis of an oligonucleotide, a nucleic acid unit in which the 5'-hydroxyl group of a nucleoside is protected with a 4,4'-dimethoxytrityl (DMTr) group is generally used, and therefore the 5'-hydroxyl group of the nucleoside located at the 5' end of the oligonucleotide to be synthesized has a DMTr group. Non Patent Literature 3 and 4 describe examples of reverse phase column purification (hereinafter, referred to also as DMTr-on reverse phase column purification; "DMTr-on" indicates that an oligonucleotide with a DMTr group introduced into its 5' end is used) using the high hydrophobicity of the DMTr group and ion exchange column purification (hereinafter, referred to also as DMTr-on ion exchange column purification) using DMTr-on. However, since a GalNAc unit is generally introduced into the hydroxyl group at the 5' end of the oligonucleotide by deprotecting the DMTr group, a conjugate to be synthesized has no DMTr groups. Therefore, for separation/purification from oligonucleotides with no GalNAc units conjugated, purification by HPLC has been needed, which is laborious and time-consuming (Non Patent Literature 5). No examples using methods such as DMTr-on reverse phase column purification or DMTr-on ion exchange column purification for GalNAc unit-oligonucleotide conjugates have been reported so far.

Thus, a GalNAc unit capable of efficiently delivering an oligonucleotide to hepatic parenchymal cells and having an advantageous structure in view of mass production, and a conjugate of the GalNAc unit with an oligonucleotide having a therapeutic effect on a disease have been desired to be developed, and an efficient production method thereof is desired.

### Citation List

### Patent Literature

Patent Literature 1: International Publication No. WO 2009/073809
Patent Literature 2: International Publication No. WO 2014/076196
Patent Literature 3: International Publication No. WO 2014/179620
Patent Literature 4: International Publication No. WO 2015/006740
Patent Literature 5: International Publication No. WO 2015/105083
Patent Literature 6: International Publication No. WO 2016/055601
Patent Literature 7: International Publication No. WO 2017/023817
Patent Literature 8: International Publication No. WO 2017/084987
Patent Literature 9: International Publication No. WO 2017/131236

### Non Patent Literature

Non Patent Literature 1: Methods in Enzymology, 1999, Vol. 313, pp. 297 to 321
Non Patent Literature 2: Bioorganic & Medicinal Chemistry Letters 26 (2016) 3690-3693
Non Patent Literature 3: AKTAdesign Purification-User Manual 18-1124-23 Edition AD
Non Patent Literature 4: Organic Process Research & Development 2005, 9, 212-215
Non Patent Literature 5: Molecules 2017, 22, 1356.

### Summary of Invention

### Technical Problem

The linkers conventionally used in studies to deliver synthetic nucleic acids to the liver using GalNAc units have emphasized keeping ligand sites away from nucleic acids, have adopted a structure having a long linear alkyl and a ring structure in a very long skeleton, and have furthermore had a complex structure with three or more branches in many cases. However, the studies of the inventors have newly revealed a problem that, when conjugates using such conventional long chain alkyl linkers are concentrated, micelles are formed in high-concentration solutions, and preparation of appropriate doses is difficult. Further, a method for column separation of oligonucleotides to which GalNAc units are bound has not been established in the conventional methods, and mass production has been difficult.

### Solution to Problem

As a result of dedicated studies in order to solve the aforementioned problems, the inventors have found that a conjugate of a biantennary GalNAc unit having the linker structure of the present invention with an oligonucleotide exhibits good solubility even in a high-dose solution while maintaining the ability of efficient delivery to hepatic parenchymal cells. Further, they have found that an oligonucleotide bound to a GalNAc unit having a highly hydrophobic protecting group (such as 4,4'-dimethoxytrityl (DMTr) group) on the hydroxyl group at position 6 of GalNAc can be purified with better efficiency by hydrophobic resin columns, thereby accomplishing the present invention. Thereby, ion exchange column purification or DMTr-on reverse phase column purification of the conjugate on a large scale can be expected to be easy.

The gist of the present invention is as follows.
(A1) A conjugate of an oligonucleotide having a nucleic acid sequence expected to have a pharmacological effect in hepatic parenchymal cells with a biantennary GalNAc unit, or a pharmaceutically acceptable salt thereof, wherein, in the conjugate, the biantennary GalNAc unit is represented by the following formula and is bound to the 5' end or the 3' end of the oligonucleotide, and wherein the conjugate is delivered specifically to hepatic parenchymal cells in vivo: 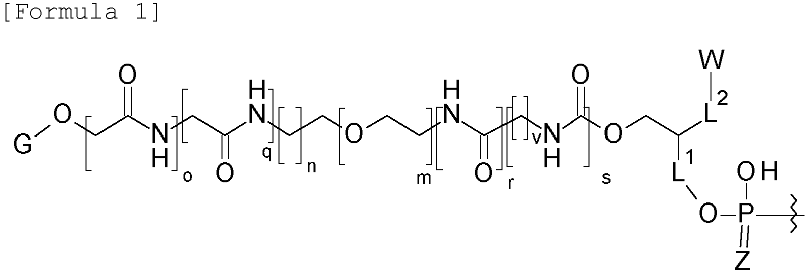 wherein W is a group represented by the following formula: 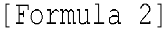 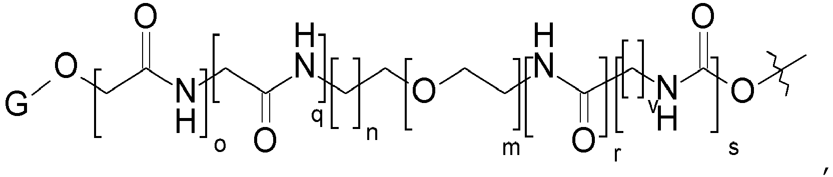 G represents a 5-acetoamido-2-hydroxymethyl-3,4-dihydroxytetrahydropyran-6-yl group (GalNAc); Z represents an oxygen atom or a sulfur atom; one of L¹ and L² represents a methylene group (CH₂), and the other represents no atoms interposed; o, q, r, and s each independently represent 0 or 1; n represents an integer of 1 to 15; m represents an integer of 0 to 5; and v represents 1 to 7, provided that when n is 1, then m is an integer of 0 to 5, and when n is an integer of 2 to 15, then m is 0.
(A2) The conjugate or a pharmaceutically acceptable salt thereof according to (A1), wherein o and q are each 0 in the formula of the biantennary GalNAc unit.
(A3) The conjugate or a pharmaceutically acceptable salt thereof according to (A1) or (A2), wherein the biantennary GalNAc unit is bound to the 5' end of the oligonucleotide.
(A4) The conjugate or a pharmaceutically acceptable salt thereof according to (A1), wherein the biantennary GalNAc unit is a group represented by any of the following formulas: 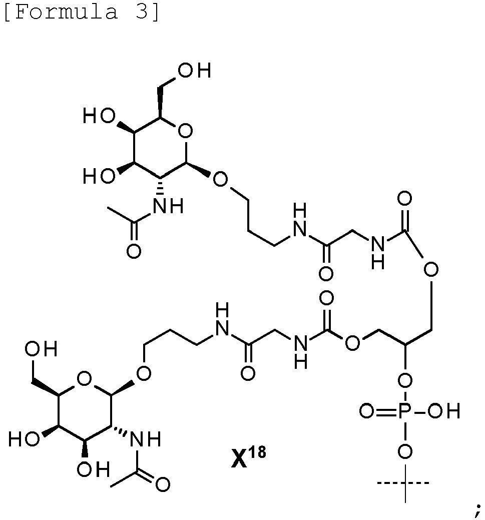 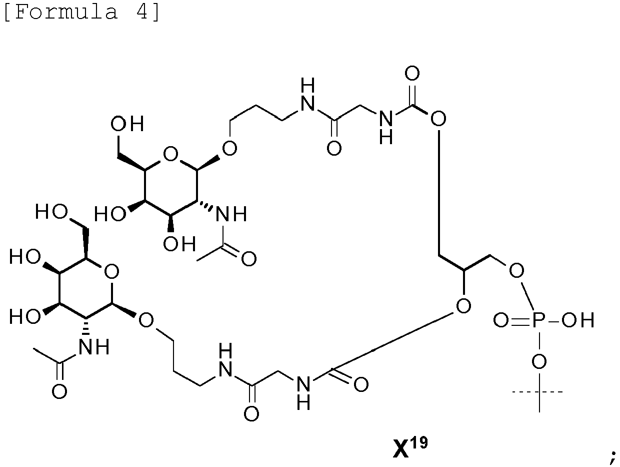 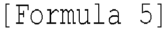 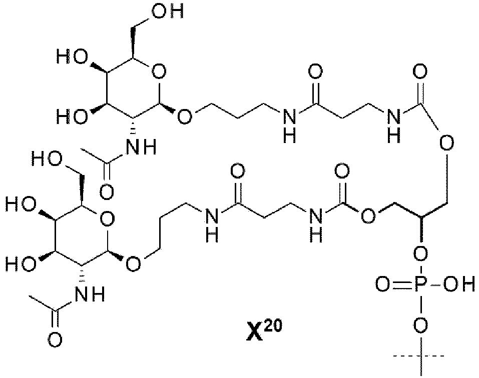 ; 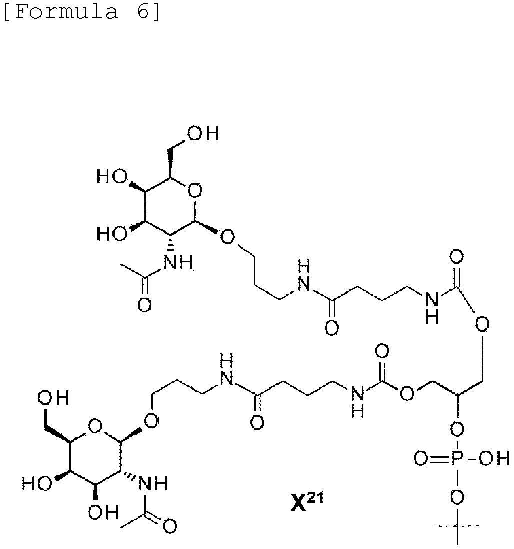 ; and 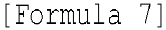 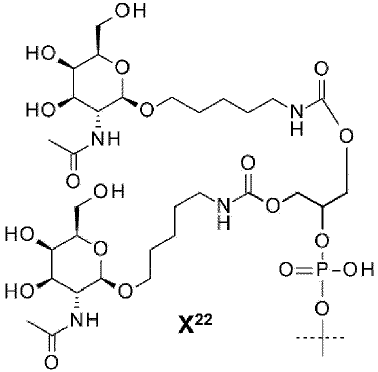
(A5) A medicament comprising the conjugate or a pharmaceutically acceptable salt thereof according to any of (A1) to (A4).

Further, the present invention provides the following production method and a compound or a conjugate comprising a hydrophobic protecting group-containing GalNac unit to be used as an intermediate in the production method.
(B1) A method for producing a conjugate of an oligonucleotide with a GalNAc unit, comprising the steps of:
   a: synthesizing a conjugate of a structure comprising a hydrophobic protecting group-containing GalNAc unit-phosphate group represented by the following formula bound to the 5' end or the 3' end of the oligonucleotide: 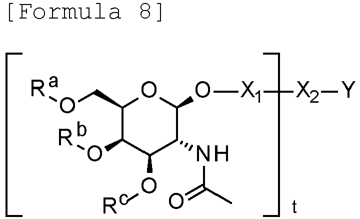 wherein at least one of R^{a}, R^{b}, and R^{c} is a hydrophobic protecting group, and the others are hydrogen atoms or protecting groups to be removed under release conditions; X₁ and X₂ each independently represent a linker structure, which may be branched between X₁ and X₂; t is the number of branched chains in the linker structure and is an integer of 1 to 10; and Y represents a bond which is bound to the oligonucleotide via a phosphate group or a thiophosphate group;
   b: passing a solution containing the product of step a through a column filled with a resin having adsorptivity due to hydrophobic interaction, and passing an aqueous washing solution through the column; and
   c: a step of eluting the oligonucleotide from the column after step b.
(B2) The production method according to (B1), wherein the hydrophobic protecting group is a protecting group to be removed under basic conditions or acidic conditions, preferably a hydrophobic protecting group to be removed under acidic conditions, more preferably an optionally substituted trityl group or an optionally substituted silyl group.
(B3) The production method according to (B1), wherein the structure comprising the hydrophobic protecting group-containing GalNAc unit-phosphate group is bound to the hydroxyl group at position 5 of the nucleoside located at the 5' end of the oligonucleotide.
(B4) The production method according to (B1), wherein R^{a} is a hydrophobic protecting group, R^{b} and R^{c} are each a hydrogen atom, and t is an integer of 1 to 3 in the structure comprising a hydrophobic protecting group-containing GalNAc unit-phosphate group.
(B5) The production method according to (B1), wherein the resin having adsorptivity due to hydrophobic interaction is any one selected from styrene-divinylbenzene adsorption resins, styrene-divinylbenzene modified adsorption resins, methacrylic adsorption resins, and phenolic adsorption resins.
(B6) The production method according to (B1), wherein the column filled with a resin having adsorptivity due to hydrophobic interaction is an ion exchange column or a reverse phase column.
(B7) The production method according to (B1), wherein step a comprises the following steps of:
   a1: synthesizing a desired oligonucleotide on a support capable of extending the oligonucleotide in the direction from the 3' end to the 5' end by repeating deprotection of the protecting group of the 5'-hydroxyl group of the nucleoside located at the 5' end and condensation of the nucleoside using the support to extend the chain of the oligonucleotide, wherein the protecting groups selected other than the protecting group of the 5'-hydroxyl group of the nucleoside used are protecting groups that undergo deprotection under conditions that allow cleavage of the binding between the 3' end of the oligonucleotide and the support (hereinafter referred to as "release conditions") are selected as the protecting groups other than the protecting group of the 5'-hydroxyl group of the nucleoside used, and a protecting group that does not undergo deprotection under the release conditions is selected as the protecting group of the 5'-hydroxyl group of the nucleoside;
   a2: introducing a hydrophobic protecting group-containing GalNAc unit into the 5'-hydroxyl group of the nucleoside located at the 5' end of the oligonucleotide synthesized on the support, wherein a hydrophobic protecting group that does not undergo deprotection under the release conditions is selected; and
   a3: treating the support obtained in step a2 with a solution under the release conditions to obtain a solution of a conjugate of a hydrophobic protecting group-containing GalNAc unit with an oligonucleotide.
(B8) The production method according to (B1), comprising, after step b and prior to step c, subjecting the hydrophobic protecting group in the conjugate adsorbed on the column to deprotection.
(B9) The production method according to (B1), wherein the release conditions are basic, and the hydrophobic protecting group is a protecting group to be removed under acidic conditions.
(B10) The production method according to (B1), wherein the structure comprising a hydrophobic protecting group-containing GalNAc unit-phosphate group is represented by any one of the following formulas: 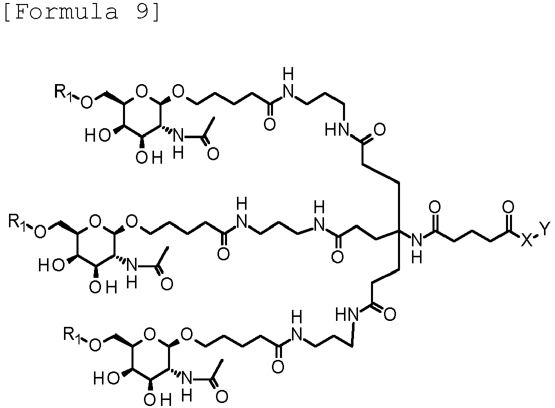 wherein R₁ represents a hydrophobic protecting group to be removed under acidic conditions, X represents a linker structure, Y represents an atomic bond that binds to the oligonucleotide via a phosphate group or a thiophosphate group, and the same applies below: 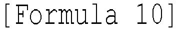 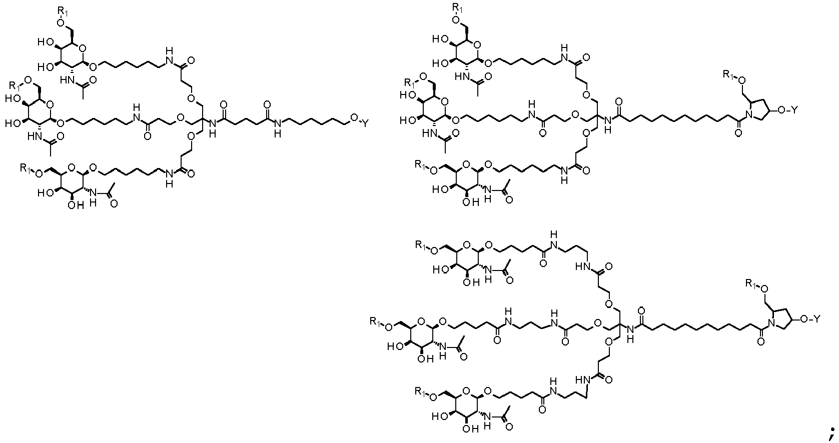 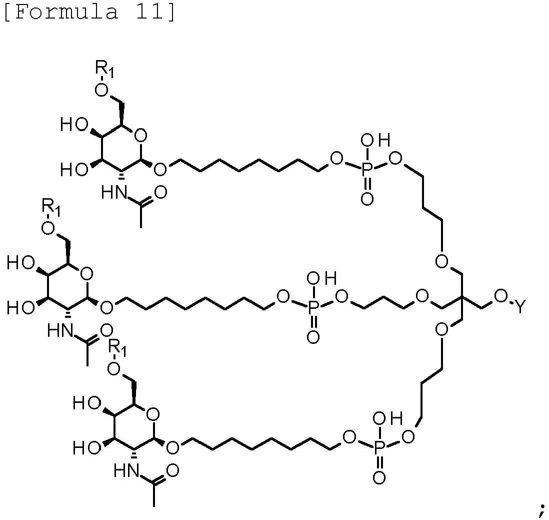 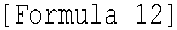 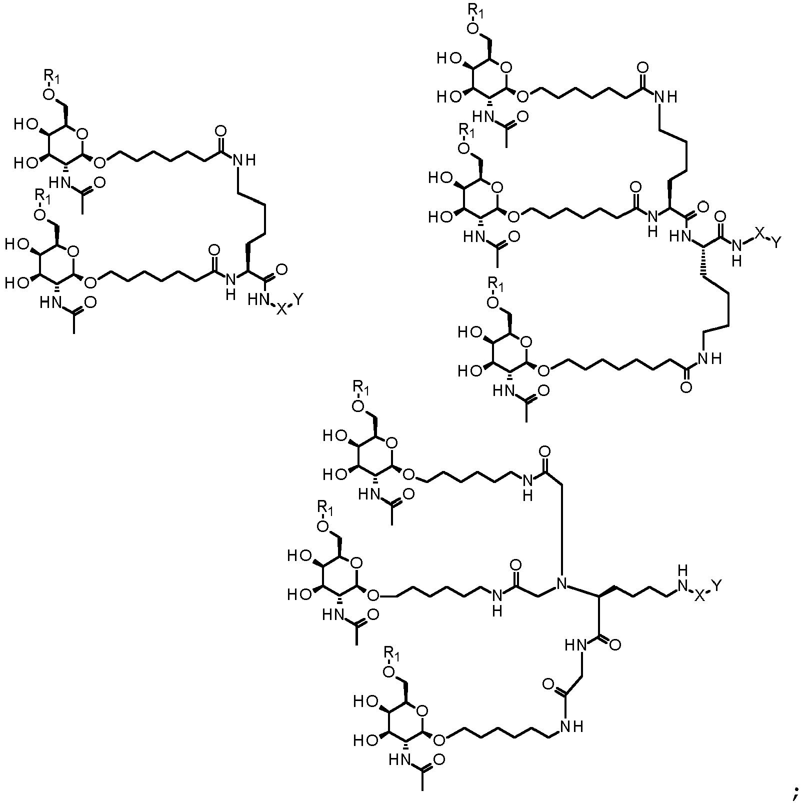 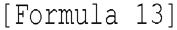 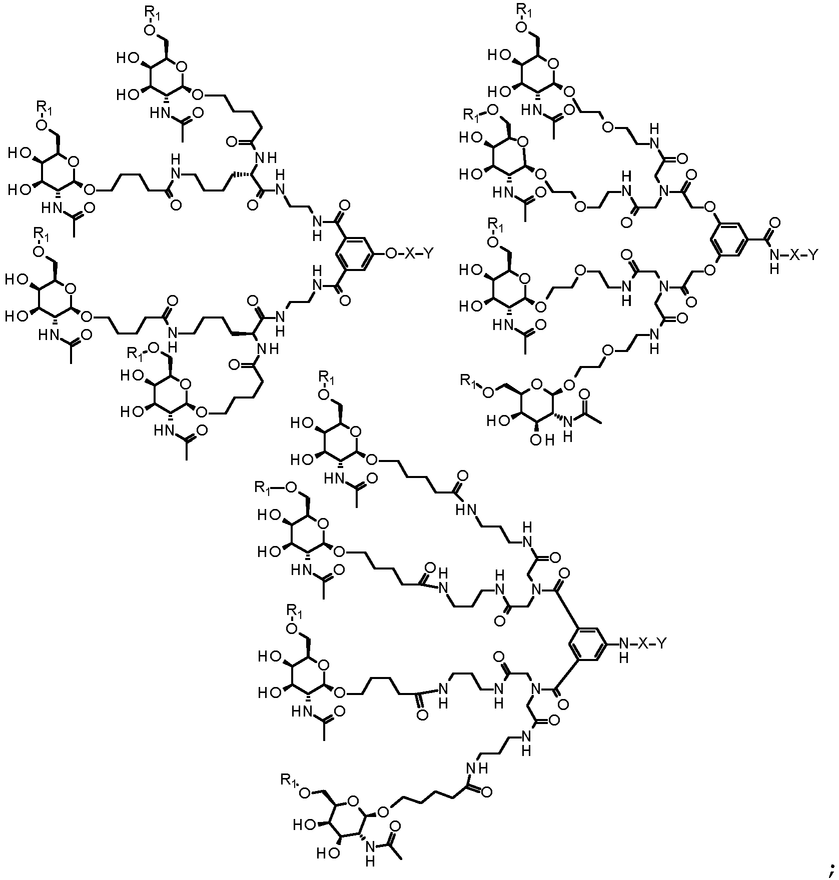 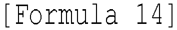 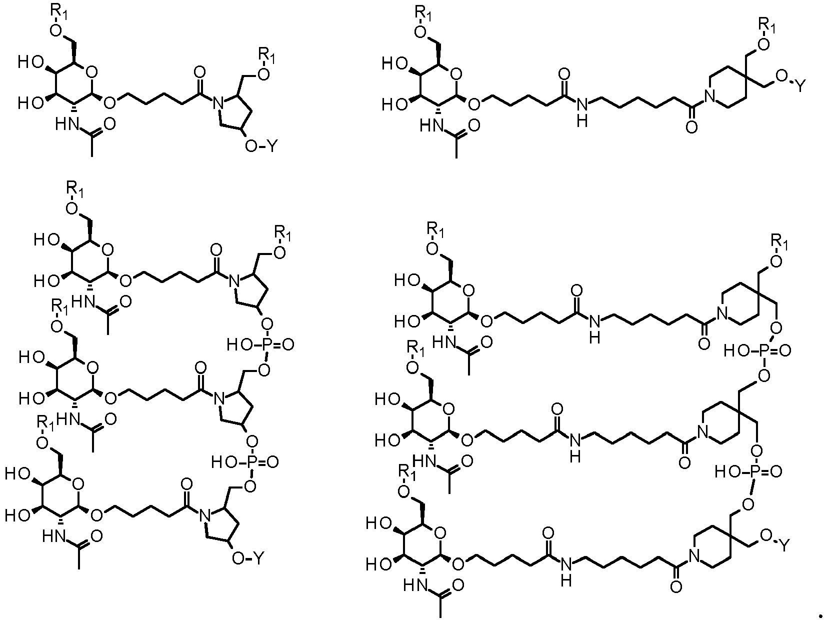 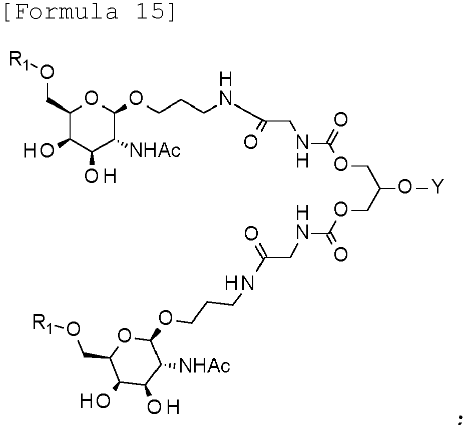 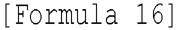 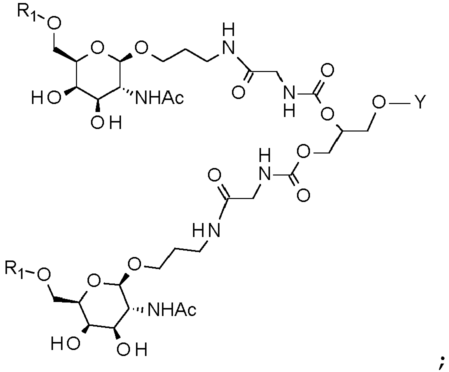 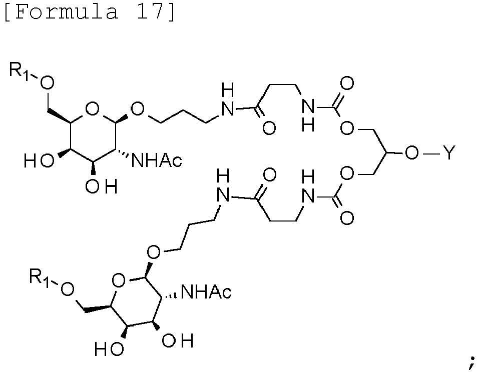 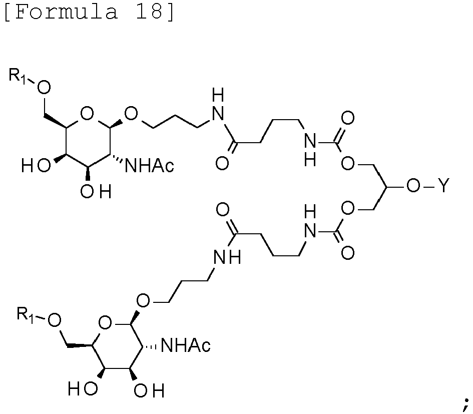 and 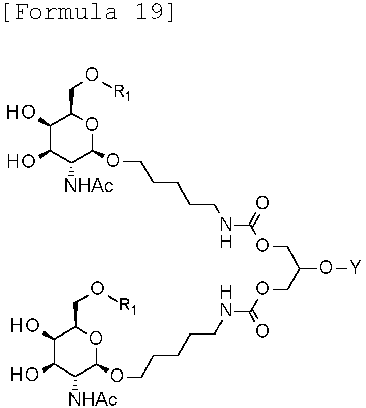
(B11) A compound represented by the following formula: 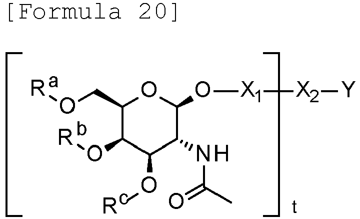 wherein at least one of R^{a}, R^{b}, and R^{c} is a hydrophobic protecting group, and the others are protecting groups to be removed under release conditions; X₁ and X₂ each independently represent a linker structure, which may be branched between X₁ and X₂; t is the number of branches in the linker structure and is an integer of 1 to 10; and
   Y represents a phosphoramidite group (preferably, a group represented by -P(OCH₂CH₂CN)N(CH(CH₃)₂)₂ or-P(OCH₃)N(CH(CH₃)₂)₂) or an H-phosphonate group (-PH(=O)(OH)); or a salt thereof.
(B12) The compound or a salt thereof according to (B11), wherein R^{a} is a hydrophobic protecting group to be removed under acidic conditions, R^{b} and R^{c} are each an acetyl group, Y represents a phosphoramidite group or an H-phosphonate group, and t is an integer of 1 to 3.
(B13) The compound or a salt thereof according to (B11) or (B12), represented by any one of the following formulas: 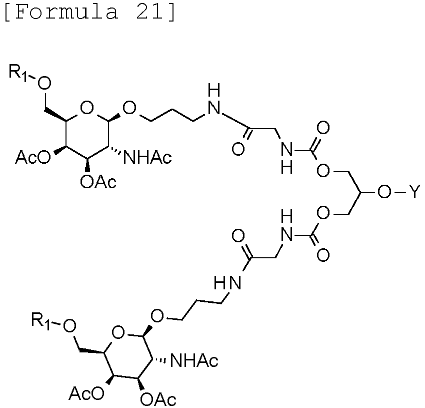 wherein R₁ is a hydrophobic protecting group to be removed under acidic conditions, and Y represents a phosphoramidite group or an H-phosphonate group, and the same applies below: 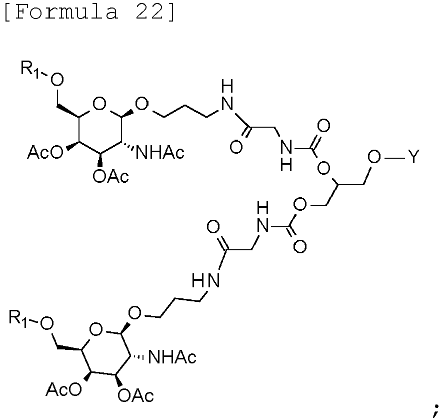 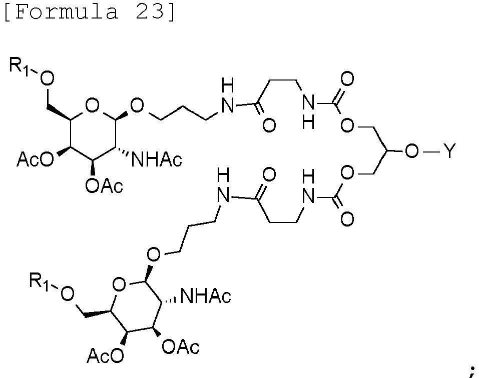 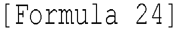 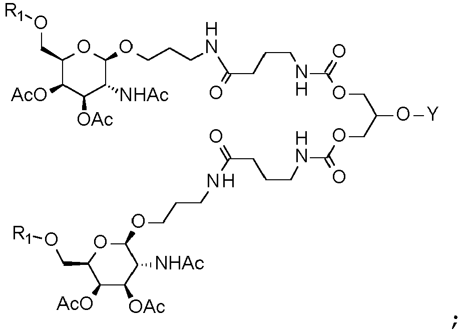 and 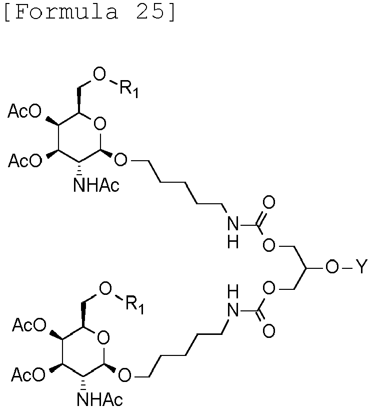
(B14) The compound or a salt thereof according to (B11) or (B12), represented by any one of the following formulas: 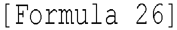 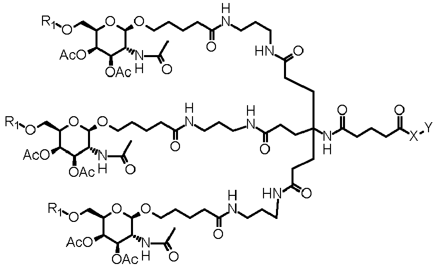 wherein R₁ represents a hydrophobic protecting group to be removed under acidic conditions, X represents a linker structure, and Y represents a phosphoramidite group or an H-phosphonate group, and the same applies below: 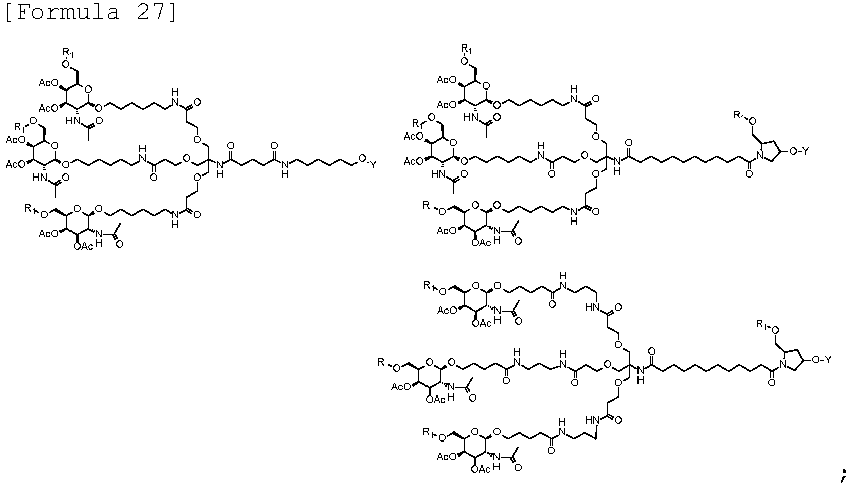 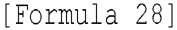 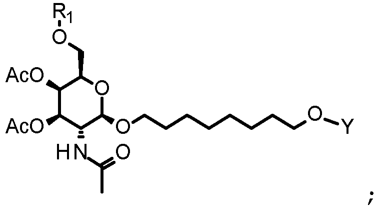 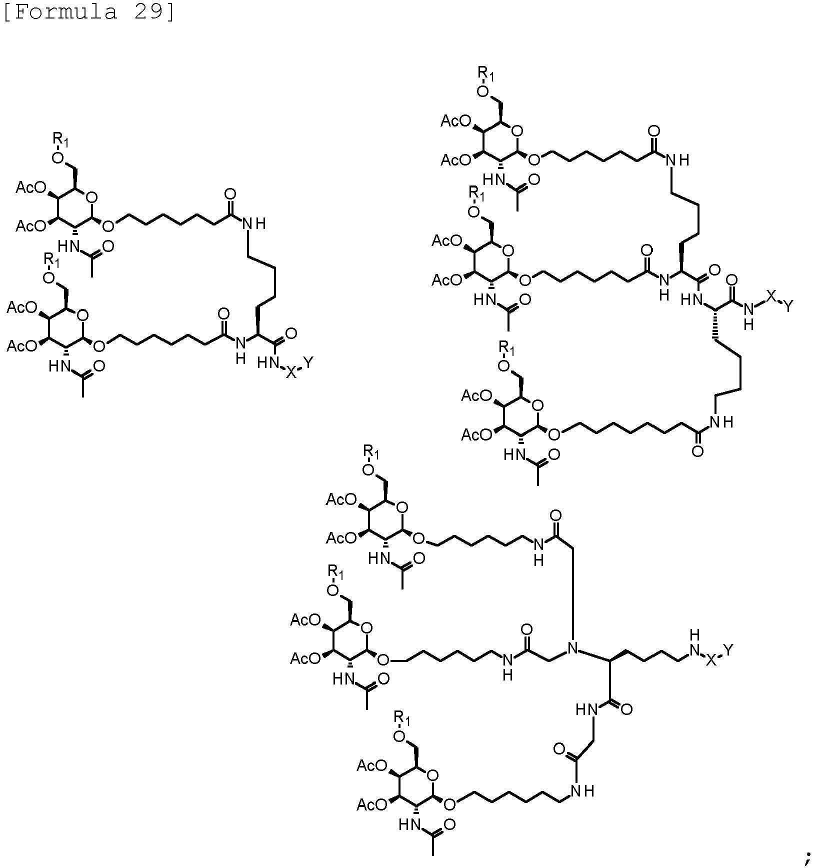 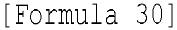 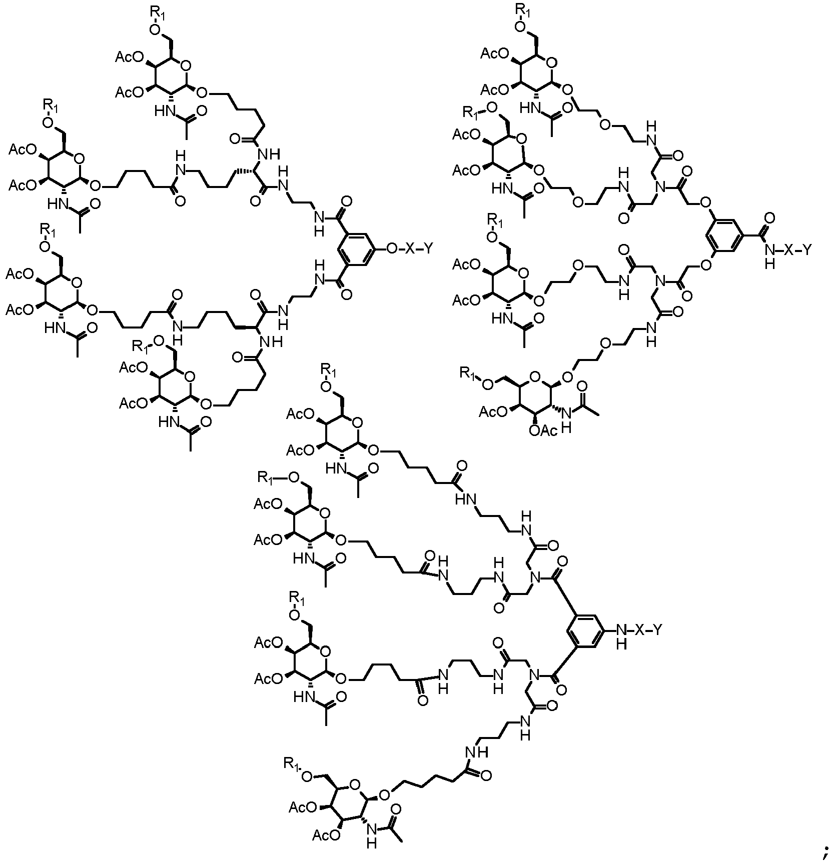 and 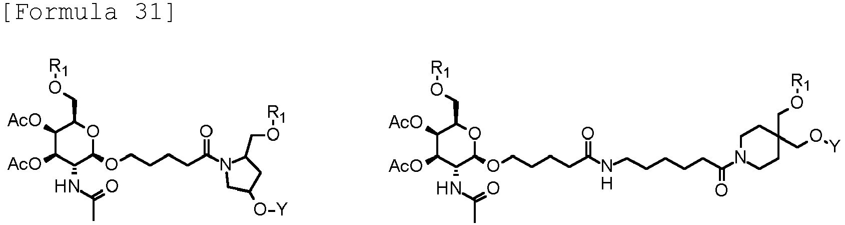
(B15) The compound or a salt thereof according to any one of (B11) to (B14), wherein the hydrophobic protecting group is an optionally substituted trityl group, and the phosphoramidite group is -P(OCH₂CH₂CN)N(CH(CH₃)₂)₂ or-P(OCH₃)N(CH(CH₃)₂)₂.
(B16) A conjugate of a structure comprising a hydrophobic protecting group-containing GalNAc unit-phosphate group represented by the following formula bound to the 5' end or the 3' end of an oligonucleotide: 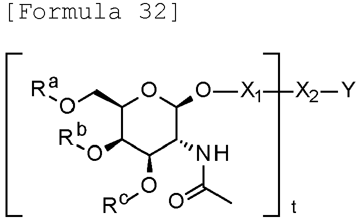 wherein at least one of R^{a}, R^{b}, and R^{c} is a hydrophobic protecting group, and the others are hydrogen atoms or protecting groups to be removed under release conditions; X₁ and X₂ each independently represent a linker structure, which may be branched between X₁ and X₂; t is the number of branched chains in the linker structure and is an integer of 1 to 10; and Y represents a bond which is bound to the oligonucleotide via a phosphate group or a thiophosphate group, or
   a salt thereof.
(B17) The conjugate or a salt thereof according to (B16), wherein the structure comprising a hydrophobic protecting group-containing GalNAc unit-phosphate group is represented by any one of the following formulas: 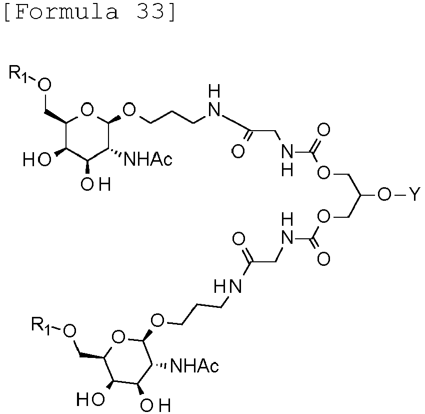 wherein R₁ represents a hydrophobic protecting group to be removed under acidic conditions, Y represents an atomic bond that binds to the oligonucleotide via a phosphate group or a thiophosphate group, and the same applies below: 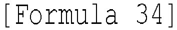 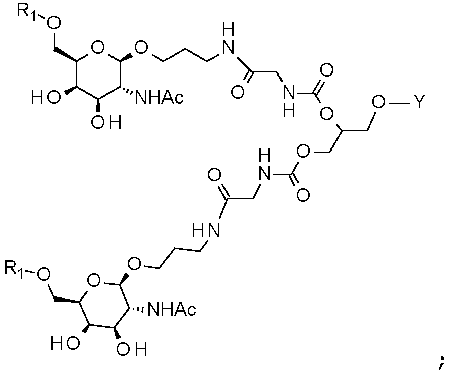 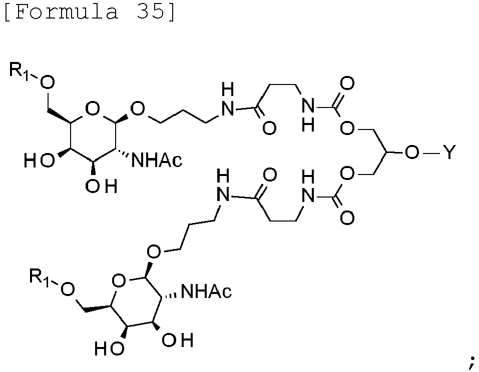 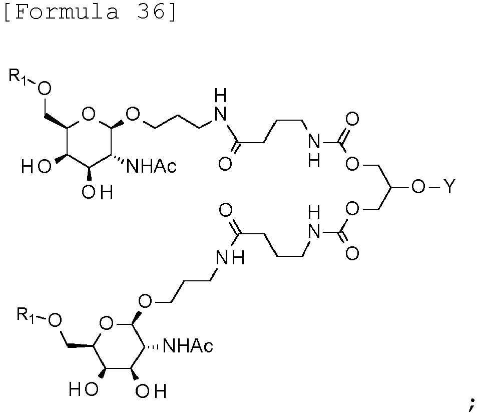 and 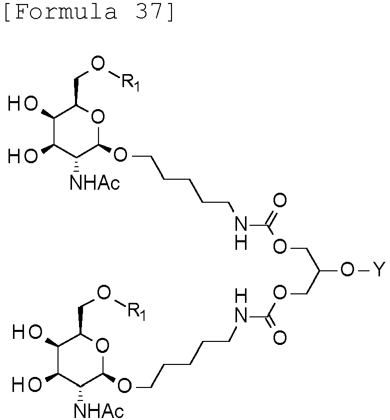
(B18) The conjugate or a salt thereof according to (B16), wherein the structure comprising a hydrophobic protecting group-containing GalNAc unit-phosphate group is represented by any one of the following formulas: 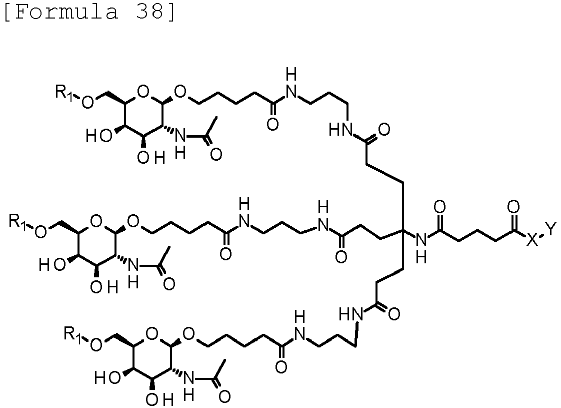 wherein R₁ represents a hydrophobic protecting group to be removed under acidic conditions, X represents a linker structure, Y represents an atomic bond that binds to the oligonucleotide via a phosphate group or a thiophosphate group, and the same applies below: 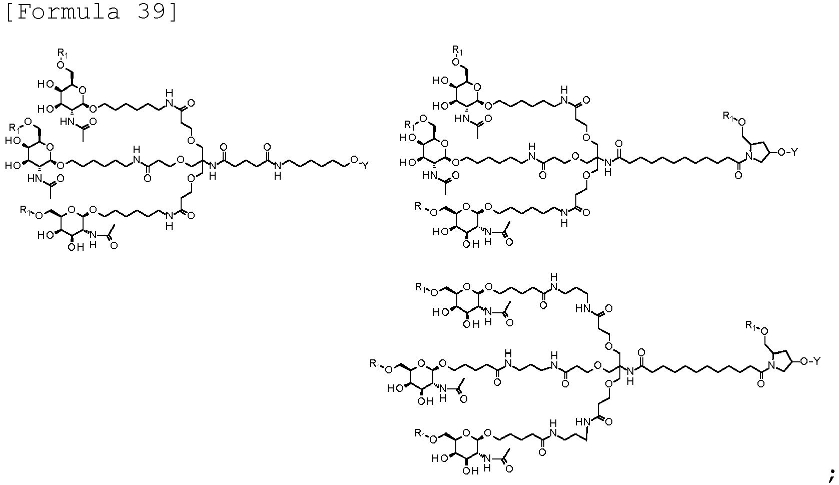 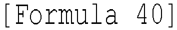 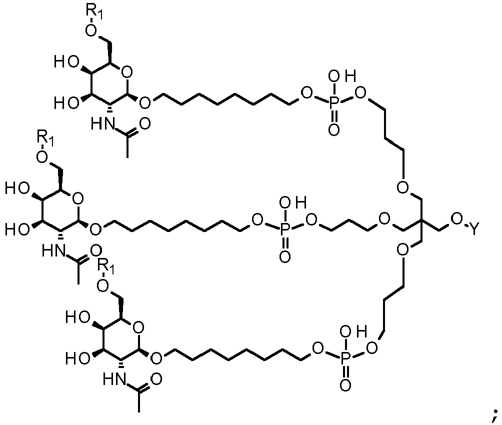 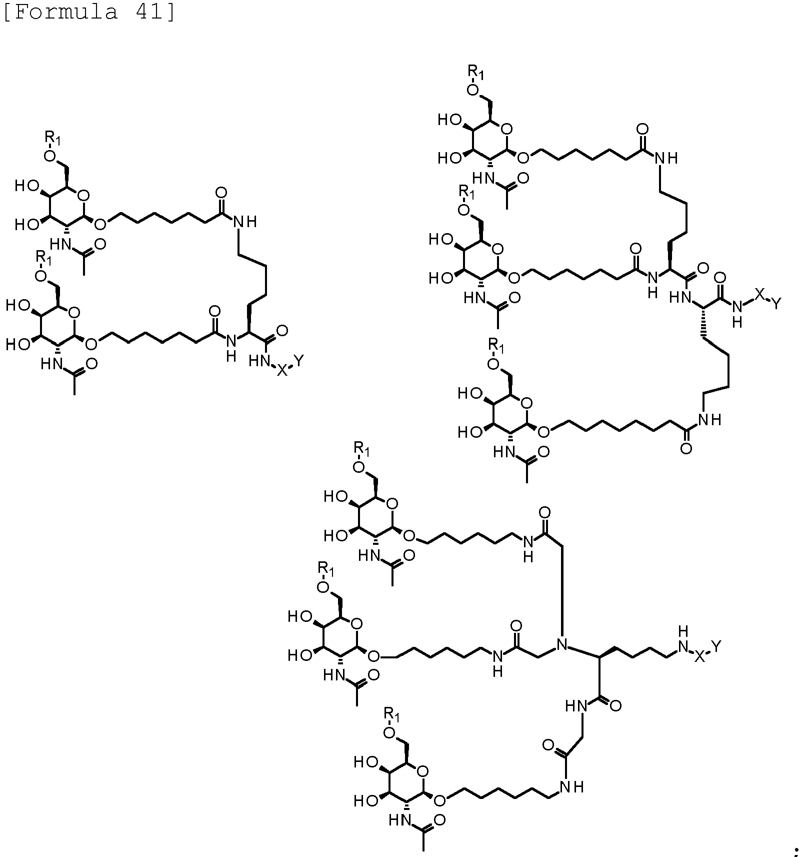 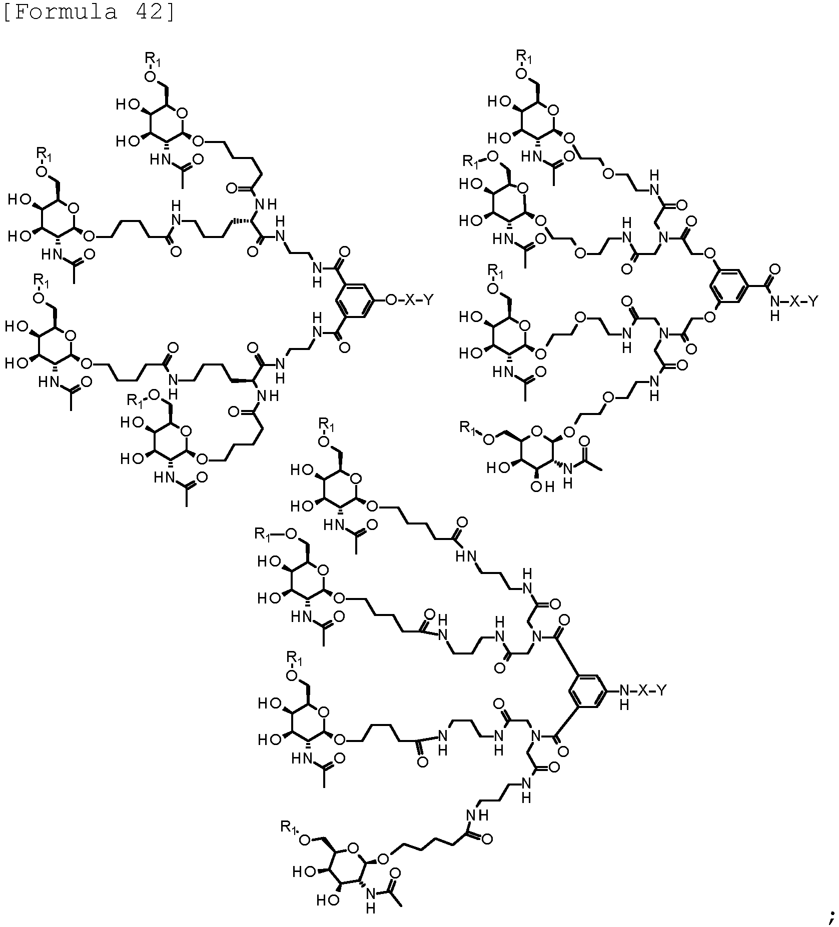 and 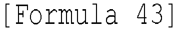 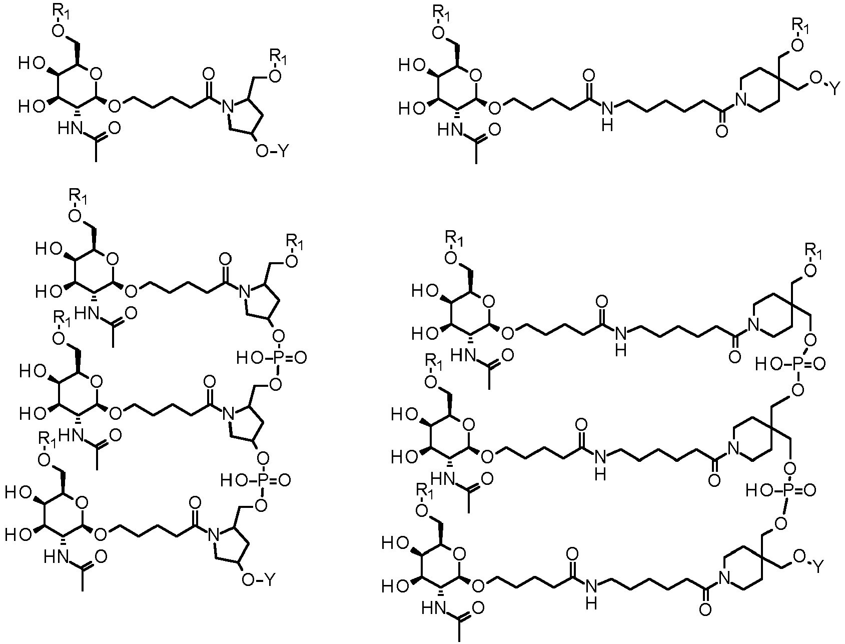
(B19) The conjugate or a salt thereof according to any one of (B16) to (B18), wherein the hydrophobic protecting group is an optionally substituted trityl group, and Y represents an atomic bond that binds to the 5'-hydroxyl group of the nucleoside located at the 5' end of the oligonucleotide via a phosphodiester bond or a phosphorothioate bond.

### Advantageous Effects of Invention

The conjugate of the present invention has achieved appropriate delivery of various oligonucleotides to the liver and high water solubility thereof. Further, it has enabled large scale production by establishing a method for DMTr-on column purification of a conjugate of a GalNAc unit with an oligonucleotide. These have made it possible to increase the dose to enhance the pharmacological effect and to prepare a high-concentration chemical solution for applying a long-term continuous formulation, thereby enabling product design of nucleic acid therapeutics corresponding to various needs.

### Brief Description of Drawings

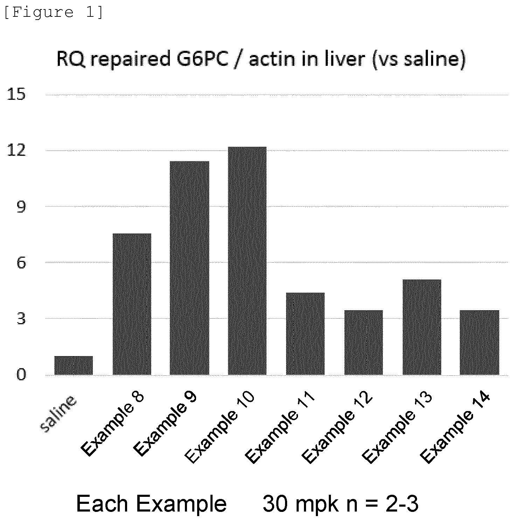

[Figure 1] Figure 1 is a graph showing corrective effects of abnormal splicing of G6PC mRNA in the liver when the compounds of Examples 8 to 14 are administered to hetero knock-in mice. The amounts of G6PC mRNA with abnormal splicing in the livers of the hetero knock-in mice corrected are quantitatively evaluated relatively by qRT-PCR (corrected by the amount of actin mRNA). The Y axis represents relative values of the expression level of normal G6PC mRNA in the administration groups when the saline administration group is taken as 1 (RQ: Relative Quantification; mpk: mg/kg).

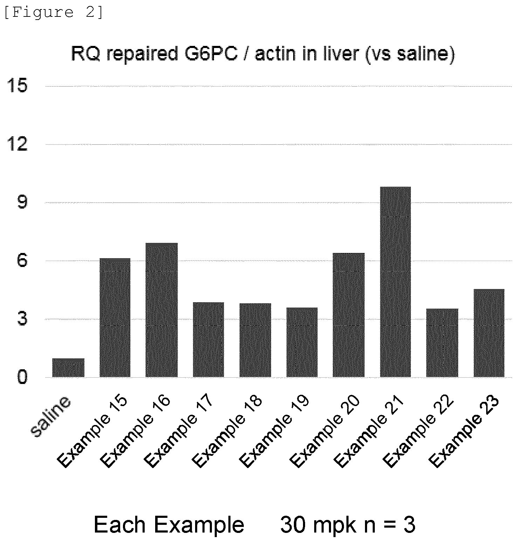

[Figure 2] Figure 2 is a graph showing corrective effects of abnormal splicing of G6PC mRNA in the liver when the compounds of Examples 15 to 23 are administered to hetero knock-in mice. The amounts of G6PC mRNA with abnormal splicing in the liver of the hetero knock-in mice corrected are quantitatively evaluated by qRT-PCR (corrected by the amount of actin mRNA). The Y axis is the same as in Figure 1. RQ: Relative Quantification; mpk: mg/kg

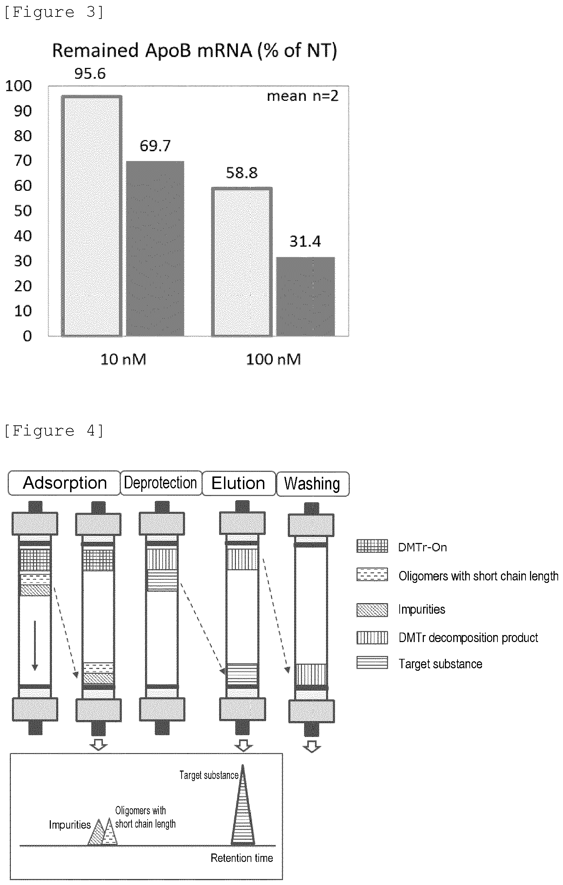

[Figure 3] Figure 3 is a graph showing degradation effects of ApoB mRNA in human primary cultured hepatocytes when ApoB-ASO (white column) and an ApoB-ASO conjugate (filled column) are added to the cells. The amounts of ApoB mRNA in the cells are quantitatively evaluated relatively by qRT-PCR (corrected by the amount of actin mRNA). The Y axis represents relative values (%) in the addition groups when the amount of ApoB mRNA in untreated human primary cultured hepatocytes is taken as 100%.
[Figure 4] Figure 4 is a correlation diagram of steps in DMTr-on ion exchange column purification and chromatocharts.

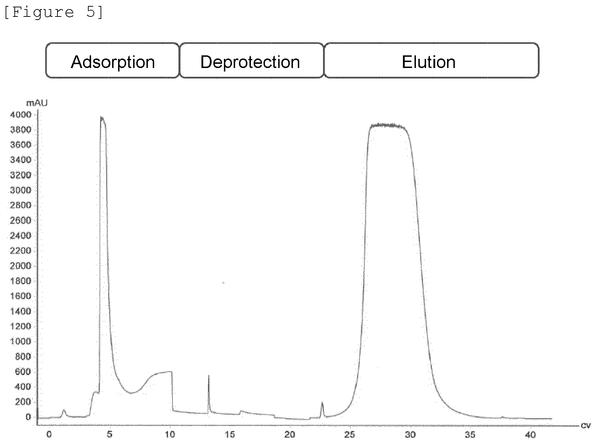

[Figure 5] Figure 5 is a chromatogram of a conjugate having a DMTr group on a hydroxyl group of GalNAc in DMTr-on ion exchange column purification (detection wavelength: 260 nm).

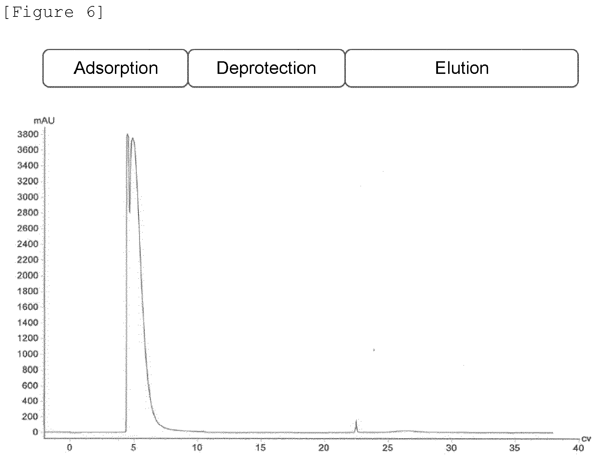

[Figure 6] Figure 6 is a chromatogram of a conjugate having a DMTr group near an oligonucleotide in DMTr-on ion exchange column purification (detection wavelength: 260 nm).

### Description of Embodiments

Hereinafter, the embodiments of the present invention will be described in detail. In this description, the term "conjugate" means a compound in which a GalNAc unit is bound to the 5' end and/or the 3' end of an oligonucleotide.

In this description, the term "GalNAc unit" means a partial structure containing N-acetyl-D-galactosamine (GalNAc) capable of binding to an asialoglycoprotein receptor (ASGPR) on hepatic parenchymal cells of the liver. The GalNAc unit contains a phosphate group or a thiophosphate group for binding a linear or branched linker structure to an oligonucleotide. Such a structural unit containing one phosphate group or a thiophosphate group for binding may be referred to as "GalNAc unit". There is no limitation on the number of GalNacs contained in one GalNac unit, and those known and disclosed in this description can be adopted, unless otherwise specified. The structure of GalNAc may be modified as long as the ability to bind to ASGPR is maintained. Further, GalNAcs with a protecting group introduced during the production process are also included.

In this description, the biantennary GalNAc unit means the GalNAc unit represented by formula (A1) above, unless otherwise stated.

In this description, the term "oligonucleotide" refers to a synthesizable nucleic acid having a chain length of 100 bases or less, which may be single-stranded or double-stranded. As will be described later, DNA, RNA, natural nucleic acids, modified nucleic acids, or the like may be selectively used as the nucleic acid to be adopted according to the purpose, and these nucleic acids may be mixed in one oligonucleotide.

In this description, the term "release conditions" mean treatment conditions to be adopted when the binding between the support and the oligonucleotide is cleaved to release the oligonucleotide from the support in the synthesis process of the oligonucleotide. In general solid-phase synthesis, the release conditions are basic.

In the present invention, the "hydrophobic protecting group" is a protecting group that can be used as a protecting group for a hydroxyl group, has a highly hydrophobic structure, and is not removed under the release conditions. For example, when the release conditions are basic, a protecting group that is removed under acidic conditions is preferred, whereas when the release conditions are acidic, a protecting group that is removed under basic conditions is preferred.

In this description, the term "phosphoramidite group" is a functional group to be adopted for an amidite reagent in nucleic acid synthesis represented by-P(OR)N(CH(CH₃)₂)₂ (R herein is a C1-C3 alkyl group or a cyano-C1-C3 alkylene group). Preferably, examples thereof include -P(OCH₂CH₂CN)N(CH(CH₃)₂)₂ and-P(OCH₃)N(CH(CH₃)₂)₂, but there is no limitation to these examples.

In this description, the "H-phosphonate group" is a functional group to be adopted in nucleic acid synthesis represented by -PH(=O)(OH) or a salt thereof. Compounds having an H-phosphonate group can form a salt together with -PH(=O)(O⁻). Examples of such a salt can include metal salts including alkali metal salts such as sodium salt, potassium salt, and lithium salt, alkaline earth metal salts such as calcium salt and magnesium salt, aluminum salt, iron salt, zinc salt, copper salt, nickel salt, and cobalt salt; amine salts including inorganic salts such as ammonium salt, and organic salts such as t-octylamine salt, dibenzylamine salt, morpholine salt, glucosamine salt, phenylglycine alkyl ester salt, ethylenediamine salt, N-methylglucamine salt, guanidine salt, diethylamine salt, triethylamine salt, dicyclohexylamine salt, N,N'-dibenzylethylenediamine salt, chloroprocaine salt, procaine salt, diethanolamine salt, N-benzyl-phenethylamine salt, piperazine salt, tetramethyl ammonium salt, and tris(hydroxymethyl)aminomethane salt; inorganic acid salts including hydrohalides such as hydrofluorate, hydrochloride, hydrobromide, and hydroiodide, nitrate, perchlorate, sulfate, and phosphate; organic acid salts including lower alkane sulfonates such as methanesulfonate, trifluoromethanesulfonate, and ethanesulfonate, aryl sulfonates such as benzenesulfonate and p-toluenesulfonate, acetate, malate, fumarate, succinate, citrate, tartrate, oxalate, and maleate; and amino acid salts such as glycine salt, lysine salt, arginine salt, ornithine salt, glutamate, and aspartate, preferably organic salts, more preferably triethylamine salt. These salts can be produced by known methods.

In this description, terms such as "binding via a phosphate group" and "structure containing a phosphate group" in the binding between nucleotides or the structure of a nucleotide and a conjugate mean that two structural units are bound in a binding mode commonly used in nucleic acid synthesis, such as a phosphodiester bond or a modified form thereof (e.g., phosphorothioate bond), or a structure containing such a binding mode.

### <1. Oligonucleotide>

In this description, the term "nucleic acid", "oligonucleotide", or "polynucleotide" refers to a polymer containing at least two deoxyribonucleotides or ribonucleotides in any of single-stranded, double-stranded, and triple-stranded forms.

Unless otherwise indicated, specific nucleic acid sequences implicitly include conservatively modified mutants (e.g., degenerate codon substitutions), alleles, orthologs, SNPs, and complementary sequences, and also include explicitly indicated sequences.

### 1-1. Types of nucleic acids

The nucleic acid of the present invention can include any form known to those skilled in the art. Specific examples of the form of the nucleic acid can include a single-stranded DNA, a single-stranded RNA, and a single-stranded oligonucleotide in which DNA and RNA are mixed. Specific examples of other forms of the nucleic acid can include a double-stranded DNA, a double-stranded RNA, a DNA-RNA hybrid polynucleotide, and a double-stranded polynucleotide consisting of two oligonucleotides in which DNA and RNA are mixed.

Examples of nucleosides or nucleotides constituting the nucleic acid of the present invention include modified nucleosides or modified nucleotides that are chemically modified, in addition to natural ones. Examples of the modified nucleosides/nucleotides include sugar-modified nucleosides/nucleotides, nucleobase-modified nucleosides/nucleotides, skeleton-modified nucleosides/nucleotides, or combinations thereof (e.g., see Nucleic Acid Research, 1997, Vol. 25, No. 22, 4429-4443) .

In this description, "natural nucleosides" refer to 2'-deoxynucleosides such as 2'-deoxyadenosine, 2'-deoxyguanosine, 2'-deoxycytidine, 2'-deoxy-5-methylcytidine, and thymidine, and ribonucleosides such as adenosine, guanosine, cytidine, 5-methylcytidine, and uridine. Further, an "oligonucleotide" refers to an oligonucleotide composed of a compound in which the sugar moiety of a nucleoside forms an ester with phosphoric acid. In this description, an oligonucleotide and a polynucleotide are used with the same meaning.

In this description, 2'-deoxyadenosine may be referred to as A^{t}, 2'-deoxyguanosine may be referred to as G^{t}, 2'-deoxycytidine may be referred to as C^{t}, 2'-deoxy-5-methylcytidine may be referred to as 5meC^{t}, thymidine may be referred to as T^{t}, 2'-deoxyuridine may be referred to as U^{t}, adenosine may be referred to as A^{rt}, guanosine may be referred to as G^{rt}, cytidine may be referred to as C^{rt}, 5-methylcytidine may be referred to as 5meC^{rt}, and uridine may be referred to as U^{rt}. In this description, 2'-deoxyadenosine nucleotide may be referred to as A^{p}, 2'-deoxyguanosine nucleotide may be referred to as G^{p}, 2'-deoxycytidine nucleotide may be referred to as C^{p}, 2'-deoxy-5-methylcytidine nucleotide may be referred to as 5meC^{p}, thymidine nucleotide may be referred to as T^{p}, 2'-deoxyuridine nucleotide may be referred to as U^{p}, adenosine nucleotide may be referred to as A^{rp}, guanosine nucleotide may be referred to as G^{rp}, cytidine nucleotide may be referred to as C^{rp}, 5-methylcytidine nucleotide may be referred to as 5meC^{rp}, and uracil nucleotide may be referred to as U^{rp}.

In this description, phosphorothioate esters contained instead of the phosphate esters in nucleotides corresponding to AP may be referred to as A^{s}, those corresponding to G^{p} may be referred to as G^{s}, those corresponding to C^{p} may be referred to as C^{s}, those corresponding to 5meC^{p} may be referred to as 5meC^{s}, those corresponding to TP may be referred to as T^{s}, those corresponding to U^{p} may be referred to as U^{s}, those corresponding to A^{rp} may be referred to as A^{rs}, those corresponding to G^{rp} may be referred to as G^{rs}, those corresponding to C^{rp} may be referred to as C^{rs}, those corresponding to 5meC^{rp} may be referred to as 5meC^{rs}, and those corresponding to U^{rp} may be referred to as U^{rs}.

In this description, a "sugar-modified nucleoside" refers to a nucleoside in which the sugar moiety of the nucleoside is modified. Examples of the sugar-modified nucleoside include 2'-O-methylnucleoside, 2'-O,4'-C-ethylene nucleoside (ENA), and 2'-O,4'-C-methylene nucleotide (BNA/LNA).

Among them, examples of the 2'-O-methyl modification include 2'-O-methylnucleoside and 2'-O-methyl nucleotide. Those corresponding to A^{rt} may be referred to as A^{m1t}, those corresponding to G^{rt} may be referred to as G^{m1t}, those corresponding to C^{rt} may be referred to as C^{m1t}, those corresponding to 5meC^{rt} may be referred to as 5meC^{m1t}, those corresponding to U^{rt} may be referred to as U^{m1t}, those corresponding to A^{rp} may be referred to as A^{m1p}, those corresponding to G^{rp} may be referred to as G^{m1p}, those corresponding to C^{rp} may be referred to as C^{m1p}, those corresponding to 5meC^{rp} may be referred to as 5meC^{m1p}, those corresponding to U^{rp} may be referred to as U^{m1p}, those corresponding to A^{rs} may be referred to as A^{m1s}, those corresponding to G^{rs} may be referred to as G^{m1s}, those corresponding to C^{rs} may be referred to as C^{m1s}, those corresponding to 5meC^{s} may be referred to as 5meC^{m1s}, and those corresponding to U^{rs} may be referred to as U^{m1s}.

In the sequence listing attached to this description, "cm" represents 2'-O-methylcytidine, "um" represents 2'-O-methyluridine, and "gm" represents 2'-O-methylguanosine, in item <223> of each sequence.

In this description, a 2'-O,4'-C-ethylene nucleotide unit and an "ENA unit" refer to each of the aforementioned nucleoside and nucleotide having ENA. The nucleosides and the nucleotides having an ENA unit may be expressed, e.g., those corresponding to A^{t} may be referred to as A^{2t}, those corresponding to A^{p} may be referred to as A^{e2p}, those corresponding to A^{s} may be referred to as A^{e2s}, those corresponding to G^{t} may be referred to as G^{2t}, those corresponding to G^{p} may be referred to as G^{e2p}, those corresponding to G^{s} may be referred to as G^{e2s}, those corresponding to 5meC^{t} may be referred to as C^{2t}, those corresponding to 5meC^{p} may be referred to as C^{e2p}, those corresponding to 5meC^{s} may be referred to as C^{e2s}, those corresponding to T^{t} may be referred to as T^{2t}, those corresponding to T^{p} may be referred to as T^{e2p}, and those corresponding to T^{s} may be referred to as T^{e2s}.

In this description, a 2'-O,4'-C-methylene nucleotide unit and a "2',4'-BNA/LNA unit" refer to each of the aforementioned nucleoside and nucleotide having 2',4'-BNA/LNA. The nucleosides and the nucleotides having a 2',4'-BNA/LNA unit may be expressed, e.g., those corresponding to A^{t} may be referred to as A^{1t}, those corresponding to A^{p} may be referred to as A^{e1p}, those corresponding to A^{s} may be referred to as A^{e1s}, those corresponding to G^{t} may be referred to as G^{1t}, those corresponding to G^{p} may be referred to as G^{e1p}, those corresponding to G^{s} may be referred to as G^{e1s}, those corresponding to 5meC^{t} may be referred to as C^{1t}, those corresponding to 5meC^{p} may be referred to as C^{e1p}, those corresponding to 5meC^{s} may be referred to as C^{e1s}, those corresponding to T^{t} may be referred to as T^{1t}, those corresponding to T^{p} may be referred to as T^{e1p}, and those corresponding to T^{s} may be referred to as T^{e1s}.

### 1-2. Target gene

In this description, the "target gene" is not particularly limited, as long as it is RNA in a cell, a tissue, or an individual into which the target gene is introduced (hereinafter, which may be referred to as "introduction subject") and may be mRNA to be translated into a protein or non-coding RNA not to be translated into a protein. Examples of the non-coding RNA include functional RNAs, such as untranslated regions of mRNA, tRNA, rRNA, mRNA-type ncRNA (mRNA-like non-coding RNA), long-chain ncRNA (long non-coding RNA), snRNA (small nuclear RNA), snoRNA (small nucleolar RNA), and miRNA (microRNA). Specifically, the target gene may be endogenous to the introduction subject or may be exogenous so as to be introduced by a technique such as gene transfer. Further, it may be a gene existing on the chromosome or a gene outside the chromosome. Examples of an exogenous gene include those derived from viruses, bacteria, fungi, or protozoa capable of infecting the introduction subject, but there is no limitation to these examples. The functions of the gene may be known or unknown.

Examples of the target gene can include genes with specifically increased expression in the liver and/or genes specifically mutated in patients with specific diseases in the liver. Examples of such diseases can include cardiovascular diseases (e.g., hypertension, cardiac hypertrophy, angina, arteriosclerosis, and hypercholesterolemia), cancers (e.g., liver cancer), respiratory diseases (e.g., pneumonia, bronchitis, asthma, chronic obstructive pulmonary disease, and pulmonary fibrosis), diabetes, diabetic retinopathy, diabetic nephropathy, anemia (e.g., anemia associated with chronic diseases and iron refractory iron-deficiency anemia), age-related macular degeneration, immune system diseases (e.g., autoimmune disease, immunodeficiency, and leukemia), liver/gallbladder diseases (e.g., nonalcoholic steatohepatitis, liver cirrhosis, hepatitis, liver failure, cholestasis, and calculus), digestive tract diseases (e.g., ulcer, enteritis, and absorption failure), infection, obesity, and fibrosis (e.g., liver fibrosis).

Examples of the causative genes of these diseases can include kinesin spindle protein (KSP), vascular endothelial growth factor (VEGF), transthyretin (TTR), proprotein convertase subtilisn/kexin type 9 (PCSK9), polo-like kinase 1 (PLK-1), ApoB-100, ribonucleotide reductase M2 subunit (RRM2), clusterin, heat shock protein 27 (Hsp27), survivin, eukaryotic initiation factor-4E (eIF-4E), alpha subunit of the interleukin 4 receptor (IL-4R-alpha), Factor XI, Factor VII, N-ras, H-ras, K-ras, bcl-2, bcl-xL, Her-1, Her-2, Her-3, Her-4, MDR-1, human β-catenin gene, DDX3 (DEAD (Asp-Glu-Ala-Asp) box polypeptide 3, X-linked), Myeloid Cell Leukemia Sequence 1 (MCL1) gene, PKR (Eif2ak2), Hsp47 (Serpinh1), Hepcidin, activated protein C(APC), signal tranducer and activator of transcription (STAT3), Collagen, type I, alpha 1 (Col1A1), ApoC-III, angiopoietin-like 3 protein (ANGPTL3), growth hormone receptor (GHr), prekallikrein (PKK), transmembrane protease, serine 6 (TMPRSS6), lipoprotein (a), glucagon receptors, or diacylglycerol acyltransferase 2 (DGAT2), angiotensinogen, complement factor B (FB), aminolevulinic acid synthase 1 (ALAS1), antithrombin (AT), Primary Hyperoxaluria Type 1 (PH1), C5 component of the complement, alpha-1 antitrypsin (AAT), and hepatitis B virus (HBV) genome, but there is no limitation to these examples.

### 1-3. Double-stranded oligonucleotide

In the case where the nucleic acid of the present invention is a nucleic acid having an RNA interference action on the target gene, the structure and chemical modifications thereof are not limited as long as it has the RNA interference action of the nucleic acid. Examples thereof can include siRNA (e.g., see International Publication No. WO 2002/044321, Current Opinion in Chemical Biology 570-579), AtuRNAi consisting of an oligonucleotide in which RNA and 2'-OMeRNA are alternately bound (e.g., see International Publication No. WO 2004/015107), a double-stranded oligonucleotide in which nucleic acids with different types of a sense strand and an antisense strand form a double strand with a Watson-Crick base pair in an oligonucleotide in which DNA and 2'-OMeRNA are alternately bound, which will be described below in the section 3-4-1 (e.g., see International Publication No. WO 2010/001909), a nucleic acid with an end of the oligonucleotide modified, which will be described below in the section 3-4-2 (e.g., see International Publication No. WO 2010/052715), and a single-stranded oligonucleotide having a single strand by binding between the 5' end of an antisense strand polynucleotide and the 3' end of a sense strand oligonucleotide via a linker and having a double-stranded structure by forming a Watson-Crick base pair in a molecule, which will be described in the section 3-4-3 (e.g., see International Publication No. WO 2012/074038).

In this description, the phrase "consisting of the same nucleotide sequence as that of the target gene" refers to consisting of the same sequence as at least a part of the nucleotide sequence of the target gene, including not only completely the same sequence but also substantially the same sequence, as long as an RNA interference action and/or a gene expression inhibitory action on the target gene is exerted. The phrase "consisting of a nucleotide sequence complementary to the target gene" refers to consisting of a sequence complementary to at least a part of the nucleotide sequence of the target gene, including not only a completely complementary sequence but also substantially the same sequence, as long as an RNA interference action and/or a gene expression inhibitory action on the target gene is exerted. Further, in the case where SNPs and the like are known in the target gene, sequences having such mutations are also included in the same nucleotide sequence. In this description, a polynucleotide containing a nucleotide sequence complementary to the target gene and having an RNA interference action and/or a gene expression inhibitory action on the target gene is called an oligonucleotide for the target gene.

The nucleotide sequence of the nucleic acid of the present invention is not particularly limited, as long as it has an RNA interference action and/or a gene expression inhibitory action on the target gene but can be determined, for example, by determining the sequences of the sense strand and the antisense strand based on a sequence expected to have an RNA interference action on the target gene using computer software (e.g., GENETYX (R): available from GENETYX COORPORATION) or further confirming the RNA interference action and/or the gene expression inhibitory action of an oligonucleotide produced based on a selected sequence.

The chain lengths of the sense strand and the antisense strand of the double-stranded oligonucleotide having an RNA interference action each may be any length from 10 nucleotides to the full-length of the open reading frame (ORF) of the target gene, as long as an RNA interference action and/or a gene expression inhibitory action is exerted, and it is preferably any length from 18 nucleotides to the full-length of the open reading frame (ORF) of the target gene, further preferably 10 to 100 nucleotides, further preferably 15 to 30 nucleotides.

In the case of using a double-stranded oligonucleotide in which nucleic acids with different types of a sense strand and an antisense strand form Watson-Crick binding in an oligonucleotide in which DNA and 2'-OMeRNA are alternately bound (International Publication No. WO 2010/001909) as a double-stranded oligonucleotide having an RNA interference action, the sense strand preferably has a chain length of 18 to 21, further preferably 18 to 19. The antisense strand preferably has a chain length of 19 to 21, further preferably 21. Further, the whole structure does not necessarily have a double-stranded structure, and those partially protruding at the 5' and/or the 3' end are also included. The protruding ends are composed of 1 to 5 nucleotides, preferably 1 to 3 nucleotides, further preferably 2 nucleotides. Further, the most preferable example is a polynucleotide having a structure in which the 3' end of the oligonucleotide of the antisense strand protrudes by 2 nucleotides (overhang structure) and forms 18 base pairs.

### 1-3-1. Oligonucleotide with DNA and 2'-OMeRNA alternately bound

As an example of the nucleic acid of the present invention to be contained in nucleic acid lipid particles, a double-stranded oligonucleotide in which nucleic acids with different types of a sense strand and an antisense strand form Watson-Crick binding in an oligonucleotide in which DNA and 2'-OMeRNA are alternately bound can be mentioned.

Specific examples of such a double-stranded oligonucleotide include double-stranded oligonucleotides disclosed in International Publication No. WO 2010/001909, International Publication No. WO 2010/001909, and the like.

### 1-3-2. Modified double-stranded oligonucleotide

In the case of using a nucleic acid having an RNA interference action as a nucleic acid to be contained in nucleic acid lipid particles, a nucleic acid in which an end of an oligonucleotide is modified can be mentioned as an example thereof, as long as it has an RNA interference action. For example, a double-stranded oligonucleotide in which a phosphate group at the 5' end of an antisense strand is modified into a 5'-aryl phosphate group (e.g., see International Publication No. WO 2010/052715) in a double-stranded oligonucleotide having an RNA interference action, such as a double-stranded oligonucleotide in which nucleic acids with different types of a sense strand and an antisense strand form Watson-Crick binding in an oligonucleotide in which siRNA, AtuRNAi, or DNA and 2'-OMeRNA are alternately bound (e.g., see International Publication No. WO 2010/001909) can be mentioned.

Specific examples of such a modified double-stranded oligonucleotide include the modified double-stranded oligonucleotides disclosed in International Publication No. WO 2010/052715 and International Publication No. WO 2015/005253.

### 1-4. Single-stranded oligonucleotide

### 1-4-1. RNA interfering single-stranded nucleotide

Examples of the nucleic acid to be used in the present invention also include an oligonucleotide having a sense strand oligonucleotide for the target gene and an antisense strand oligonucleotide with a nucleotide sequence complementary to the sense strand and having a single-stranded structure bound by a linker forming a phosphodiester structure at each of the 5' end of the antisense strand oligonucleotide and the 3' end of the sense strand oligonucleotide (e.g., see International Publication No. WO 2012/074038), as long as it has an RNA interference action.

Specific examples of such a compound can include those described in International Publication No. WO 2012/074038 and International Publication No. WO 2015/005253.

### 1-4-2. Antisense nucleotide

In the treatment of a disease caused by a gene mutation, an antisense nucleotide is applied in order to treat the disease that can be treated by normalizing or suppressing the expression of the mutation gene. In the case of suppressing the gene expression, a nucleotide which has a sequence complementary to a part of the mRNA of the target gene and in which a nucleic acid in the middle of the sequence is DNA is used. Such an antisense nucleotide for suppressing expression forms a double strand with the mRNA of the target gene. Since the RNA/DNA double-stranded structure in the double strand formed is specifically decomposed by RNaseH in vivo, the mRNA of the target gene is specifically destroyed, thereby suppressing the expression of the target gene.

Examples of the antisense nucleotide intended to normalize the gene expression include those suppressing abnormal splicing and those restoring mutation sites on the gene into a normal sequence. In the case of restoring mutations on the gene into a normal sequence, an antisense sequence for the 5' side region of the mutation sites in the sense strand of the target gene is included on the 3' side of the antisense nucleotide, and a sequence constituting a hairpin structure with which nuclease associates (a sequence in which sequences complementary to each other at two sites are connected by an intermediate linker/loop sequence) is included on the 5' side thereof. With such a nucleotide, the antisense region on the 3' side functions as a guide RNA that guides the nuclease to the mutation sites of the target gene (a sense strand of mRNA or genome DNA). This phenomenon is similar to the genome editing technology known as the CRISPR-Cas system and is widely known as being applicable to various gene editing.

The mechanism by which abnormal splicing occurs due to gene mutation is as follows. In the splicing process from pre-mRNA to mature mRNA, the mutation causes a splicing factor to accidentally recognize and bind to a site to which it does not bind in normal pre-mRNA, so that mRNA subjected to abnormal splicing is expressed, thereby causing function failure or expression failure in the protein expressed by the target gene. When an antisense nucleotide binds to the region misrecognized by the splicing factor on the mutated pre-mRNA, the splicing factor cannot recognize the region, and thus abnormal splicing is suppressed. Examples of diseases caused by abnormal splicing due to gene mutation include an antisense oligonucleotide suppressing abnormal splicing caused by a c.648G>T mutation in the G6PC gene of glycogen storage disease type 1a described below in detail, and other antisense oligonucleotides for treating various known diseases can be mentioned.

The present invention provides an oligonucleotide capable of repairing the G6PC gene having the c.648G>T mutation in patients with glycogen storage disease type Ia at the mRNA level and expressing normal G6PC protein, or a pharmaceutically acceptable salt thereof. The oligonucleotide of the present invention is an oligonucleotide with 15 to 30 bases consisting of a nucleotide sequence complementary to the cDNA of the G6PC gene having the c.648G>T mutation and consists of a sequence complementary to a region containing any of the 82nd to the 92nd sites from the 5' end of exon 5 of the G6PC gene having the c.648G>T mutation.

Among patients with glycogen storage disease type Ia, patients with a c.648G>T mutation can be targeted in the present invention. Not only homozygous patients with a c.648G>T mutation but also compound heterozygous patients with a c.648G>T mutation and other mutations can be targeted.

The c.648G>T mutation of the G6PC gene is a mutation of nucleotide 728 from G to T in Homo sapiens glucose-6-phosphatase catalytic subunit (G6PC), transcript variant 1, mRNA (NCBI-GenBank accession No. NM_000151.3). It is located at the 86th position from the 5' end of exon 5. The oligonucleotide of the present invention may consist of a sequence complementary to a region containing any of the 82nd to the 92nd sites from the 5' end of exon 5 of the G6PC gene having the c.648G>T mutation, preferably a sequence complementary to a region containing the 92nd site.

Examples of the oligonucleotide with 15 to 30 bases consisting of a nucleotide sequence complementary to the cDNA of the G6PC gene having a c.648G>T mutation and consisting of a sequence complementary to a region containing any of the 82nd to the 92nd sites from the 5' end of exon 5 of the G6PC gene having a c.648G>T mutation can include those containing all or a part of any sequence of SEQ ID Nos: 1 to 32, 40 to 42, and 44 to 48 (provided that t or T may be u or U, or u or U may be t or T, in the sequence). In the present invention, the term "a part of a sequence" is generally 80 % or more, preferably 85%, further preferably 90%, most preferably 94% or more, in the entire sequence. The oligonucleotide of the present invention suitably has 15 to 30, preferably 15 to 21, more preferably 15 to 18 bases.

Nucleotides constituting the oligonucleotide of the present invention may be any of natural DNA, natural RNA, DNA/RNA chimera, and modified forms of these, but at least one of the nucleotides is preferably a modified nucleotide.

Examples of the modified nucleotide in the present invention can include those in which a sugar is modified (e.g., those in which D-ribofuranose is 2'-O-alkylated, those in which D-ribofuranose is 2'-O,4'-C-alkylenated, and those in which D-ribofuranose is 2'-,4'-crosslinked), those in which a phosphodiester bond is modified (e.g., thioated), those in which a base is modified, and combinations of them. Those in which at least one D-ribofuranose constituting the antisense oligonucleotide is 2'-O-alkylated or 2'-O,4'-C-alkylenated can be expected to have a higher therapeutic effect than natural nucleotides (that is, oligo DNA and oligo RNA) due to their high binding to RNA and high resistance to nucleases. Further, those in which at least one phosphodiester bond constituting the oligonucleotide is thioated can also be expected to have a higher therapeutic effect than natural nucleotides (that is, oligo DNA and oligo RNA) due to their high resistance to nucleases. An oligonucleotide containing both of a modified sugar as above and a modified phosphodiester bond has a higher resistance to nucleases and thus can be expected to have a still higher therapeutic effect.

Examples of sugar modification in the oligonucleotide of the present invention can include 2'-O-alkylation of D-ribofuranose (e.g., 2'-O-methylation, 2'-O-aminoethylation, 2'-O-propylation, 2'-O-allylation, 2'-O-methoxyethylation, 2'-O-butylation, 2'-O-pentylation, and 2'-O-propargylation), 2'-O,4'-C-alkylenation of D-ribofuranose (e.g., 2'-O,4'-C-ethylenation, 2'-O,4'-C-methylenation, 2'-O,4'-C-propylenation, 2'-O,4'-C-tetramethylenation, and 2'-O,4'-C-pentamethylenation), 2'-deoxy-2'-C,4'-C-methyleneoxymethylenation of D-ribofuranose, S-cEt (2',4'-constrained ethyl), AmNA (Amide-bridged nucleic acid), 3'-deoxy-3'-amino-2'-deoxy-D-ribofuranose, and 3'-deoxy-3'-amino-2'-deoxy-2'-fluoro-D-ribofuranose.

Examples of sugar 2'-,4'-crosslinking modification in the oligonucleotide of the present invention can include 2'-O,4'-C-alkylenation of D-ribofuranose (e.g., 2'-O,4'-C-ethylenation, 2'-O,4'-C-methylenation, 2'-O,4'-C-propylenation, 2'-O,4'-C-tetramethylenation, and 2'-O,4'-C-pentamethylenation), 2'-deoxy-2'-C,4'-C-methyleneoxymethylenation of D-ribofuranose, S-cEt (2',4'-constrained ethyl), and AmNA.

Examples of modification of the phosphodiester bond in the oligonucleotide of the present invention can include phosphorothioate bond, methylphosphonate bond, methylthiophosphonate bond, phosphorodithioate bond, and phosphoramidate bond.

In the present invention, examples of base modification can include 5-methylation, 5-fluorination, 5-bromination, 5-iodination, and N4-methylation of cytosine, 5-demethylation (uracil), 5-fluorination, 5-bromination, and 5-iodination of thymine, N6-methylation and 8-bromination of adenine, and N2-methylation and 8-bromination of guanine.

The oligonucleotide of the present invention can be synthesized according to the method described in the literature (Nucleic Acids Research, 12, 4539 (1984)) using a commercially available synthesizer (e.g., model 392, available from PerkinElmer, Inc., by the phosphoramidide method). As a nucleoside phosphoramidite reagent to be used therein, a commercially available reagent can be used for natural nucleosides and 2'-O-methylnucleosides (that is, 2'-O-methylguanosine, 2'-O-methyladenosine, 2'-O-methylcytidine, and 2'-O-methyluridine). 2'-O-Alkylguanosine with an alkyl group having 2 to 6 carbon atoms, adenosine, cytidine, and uridine can be synthesized as follows.

2'-O-Aminoethylguanosine, 2'-O-aminoethyladenosine, 2'-O-aminoethylcytidine, and 2'-O-aminoethyluridine can be synthesized according to the literature (Blommers et al. Biochemistry (1998), 37, 17714-17725.).

2'-O-Propylguanosine, 2'-O-propyladenosine, 2'-O-propylcytidine, and 2'-O-propyluridine can be synthesized according to the literature (Lesnik, E. A. et al. Biochemistry (1993), 32, 7832-7838.).

Commercially available reagents can be used for 2'-O-allylguanosine, 2'-O-allyladenosine, 2'-O-allylcytidine, and 2'-O-allyluridine.

2'-O-Methoxyethylguanosine, 2'-O-methoxyethyladenosine, 2'-O-methoxyethylcytidine, and 2'-O-methoxyethyluridine can be synthesized according to a patent (US6261840) or the literature (Martin, P. Helv. Chim. Acta. (1995) 78, 486-504.).

2'-O-Butylguanosine, 2'-O-butyladenosine, 2'-O-butylcytidine, and 2'-O-butyluridine can be synthesized according to the literature (Lesnik,E.A. et al. Biochemistry (1993), 32, 7832-7838.).

2'-O-Pentylguanosine, 2'-O-pentyladenosine, 2'-O-pentylcytidine, and 2'-O-pentyluridine can be synthesized according to the literature (Lesnik,E.A. et al. Biochemistry (1993), 32, 7832-7838.).

Commercially available reagents can be used for 2'-O-propargylguanosine, 2'-O-propargyladenosine, 2'-O-propargylcytidine, and 2'-O-propargyluridine.

2'-O,4'-C-Methyleneguanosine, 2'-O,4'-C-methyleneadenosine, 2'-O,4'-C-methylenecytidine, 2'-O,4'-C-methylene-5-methylcytidine, and 2'-O,4'-C-methylenethymidine can be produced according to the method described in International Publication No. WO 99/14226, and 2'-O,4'-C-alkyleneguanosine with an alkylene group having 2 to 5 carbon atoms, 2'-O,4'-C-alkyleneadenosine, 2'-O,4'-C-alkylenecytidine, 2'-O,4'-C-alkylene-5-methylcytidine, and 2'-O,4'-C-alkylenethymidine can be produced according to the method described in International Publication No. WO 00/47599.

A nucleoside in which D-ribofuranose is 2'-deoxy-2'-C,4'-C-methyleneoxymethylenated can be synthesized according to the literature (Wang, G. et al. Tetrahedron (1999), 55, 7707-7724.).

S-cEt (constrained ethyl) can be synthesized according to the literature (Seth, P. P. et al. J. Org. Chem (2010), 75, 1569-1581.).

AmNA can be synthesized according to the literature (Yahara, A. et al. ChemBioChem (2012), 13, 2513-2516.) or International Publication No. WO 2014/109384.

In the present invention, adenine can be described as (A) or (a), guanine can be described as (G) or (g), cytosine can be described as (C) or (c), thymine can be described as (T) or (t), and uracil can be described as (U) or (u), respectively, in nucleic acid nucleotide sequences. Instead of cytosine, 5-methylcytosine can be used. In the nucleobases, uracil (U) or (u) and thymine (T) or (t) are compatible with each other. Both of uracil (U) or (u) and thymine (T) or (t) can be used for forming a base pair with adenine (A) or (a) of the complementary strand.

An antisense oligonucleotide with a phosphorothioate bond can be synthesized by coupling a nucleoside phosphoramidite reagent and then reacting sulfur, tetraethylthiuram disulfide (TETD, Applied Biosystems), Beaucage reagent (Glen Research), or a reagent such as xanthane hydride (Tetrahedron Letters, 32, 3005 (1991), J. Am. Chem. Soc. 112, 1253 (1990), PCT/International Publication No. WO 98/54198).

As controlled pore glass (CPG) to be used in a synthesizer, commercially available products with 2'-O-methylnucleoside bound can be used. Further, for 2'-O,4'-C-methyleneguanosine, 2'-O,4'-C-methyleneadenosine, 2'-O,4'-C-methylene-5-methylcytidine, and 2'-O,4'-C-methylenethymidine, nucleosides produced according to the method described in International Publication No. WO 99/14226 can be bound to CPG, and for 2'-O,4'-C-alkyleneguanosine with an alkylene group having 2 to 5 carbon atoms, 2'-O,4'-C-alkyleneadenosine, 2'-O,4'-C-alkylene-5-methylcytidine, and 2'-O,4'-C-alkylenethymidine, nucleosides produced according to the method described in International Publication No. WO 00/47599 can be bound thereto, according to the literature (Oligonucleotide Synthesis, Edited by M. J. Gait, Oxford University Press, 1984). An oligonucleotide in which a 2-hydroxyethyl phosphate group is bound to the 3' end can be synthesized by using a modified CPG (disclosed in Example 12b of Japanese Patent Laid-Open No. 7-87982). Further, an oligonucleotide in which a hydroxyalkyl phosphate group or an aminoalkyl phosphate group is bound to the 3' end can be synthesized by using 3'-amino-Modifier C3 CPG, 3'-amino-Modifier C7 CPG, Glyceryl CPG (Glen Research), 3'-specer C3 SynBase CPG 1000, or 3'-specer C9 SynBase CPG 1000 (link technologies).

### <2. GalNAc unit>

In the conjugate of the present invention, the oligonucleotide may have GalNAc bound via a linker and a phosphate group.

The GalNAc unit in the present invention is a phosphate group to which the linker with the GalNAc bound thereto is bound and may have one further bond. The phosphate group of the GalNAc unit can bind to the 5' end and/or the 3' end of the oligonucleotide, preferably, to the 5' end of the oligonucleotide. In the case where the GalNAc unit has a bond, the bond can bind to a hydroxyl group, GalNAc, a linker with the GalNAc bound thereto, a phosphate group of another GalNAc unit, or a phosphate group at the 3' end of the oligonucleotide. One GalNAc unit contains two GalNAcs.

In the conjugate, one GalNAc unit may bind to the oligonucleotide, or a plurality of GalNAc units may bind to the oligonucleotide in series. The number of GalNAc units to be bound to one oligonucleotide is preferably 1 to 7, more preferably 1 to 5, particularly preferably 1 to 3, optimally 1.

In the case where two GalNAc units are introduced into the 5' end of the oligonucleotide in the present invention, Symmetric Doubler Phosphoramidite (1,3-bis-[5-(4,4'-dimethoxytrityloxy)pentylamido]propyl-2-[(2-cyanoethyl)-(N,N-diisopropyl)]-phosphoramidite, Glen Research) or the like can be used when synthesizing the oligonucleotide, and in the case where three GalNAc units are introduced into the 5' end of the oligonucleotide, Trebler Phosphoramidite (Tris-2,2,2-[3-(4,4'-dimethoxytrityloxy)propyloxymethyl]ethyl-[(2-cyanoethyl)-(N,N-diisopropyl)]-phosphoramidite, Glen Research), Long Trebler Phosphoramidite (Tris-2,2,2-[3-(4,4'-dimethoxytrityloxy)propyloxymethyl]methyleneoxypropyl-[(2-cyanoethyl)-(N,N-diisopropyl)]-phosphoramidite, Glen Research) or the like can be used when synthesizing the oligonucleotide. In the case where four GalNAc units are introduced into the 5' end of the oligonucleotide, Symmetric Doubler Phosphoramidite or the like coupled twice can be used when synthesizing the oligonucleotide. In the case where five GalNAc units are introduced into the 5' end of the oligonucleotide, Symmetric Doubler Phosphoramidite and Trebler Phosphoramidite or Long Trebler Phosphoramidite each coupled once can be used when synthesizing the oligonucleotide. In the case where five or more GalNAc units are introduced into the 5' end of the oligonucleotide, Symmetric Doubler Phosphoramidite, Trebler Phosphoramidite, or Long Trebler Phosphoramidite can be appropriately combined for synthesis.

In the case where such a GalNAc unit is introduced into the 3' end of the oligonucleotide in the present invention, Asymmetric Doubler (Lev) Phosphoramidite(1-[5-(4,4'-dimethoxytrityloxy)pentylamido]-3-[5-levulinyloxypentylamido]-propyl-2-[(2-cyanoethyl)-(N,N-diisopropyl)]-phosphoramidite, Glen Research) or the like can be used when synthesizing the oligonucleotide. Specifically, after coupling a commercially available Unylinker Support (e.g., Glen UnySuppor 1000, Glen Research) with Asymmetric Doubler (Lev) Phosphoramidite to remove DMTr groups, the chain of the oligonucleotide of interest is extended. After the intended chain length has been achieved, it can be introduced into the 3' end of the oligonucleotide by deprotecting levulinyl groups using a reagent such as hydrazine and coupling the GalNAc unit according to the protocol attached to an Asymmetric Doubler (Lev) Phosphoramidite reagent.

In the present invention, an additional oligonucleotide sequence that is a different nucleotide sequence from that of the oligonucleotide exerting a medicinal effect, is composed of a phosphodiester bond, and can be cleaved in vivo may be contained at the 5' end and/or the 3' end of the oligonucleotide binding to the GalNAc unit. The chain length of the oligonucleotide that can be cleaved is preferably 1 to 6 nucleotides, further preferably 1 to 3 nucleotides. The oligonucleotide that can be cleaved is not particularly limited, as long as it can be cleaved, but examples thereof can include natural oligodeoxynucleotides entirely consisting of DNA and natural oligonucleotides entirely consisting of RNA. The nucleotide sequence is not particularly limited, as long as it is a sequence that can be cleaved, but examples thereof can include 5'-TCATCA-3', 5'-CATCA-3', 5'-ATCA-3'5'-TCA-3', 5'-CA-3', and 5'-A-3'.

In one aspect, the biantennary GalNAc unit in the present invention is a group represented by the following formula: 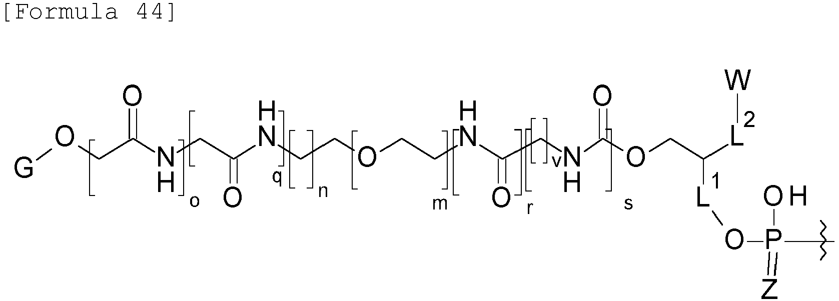 wherein W is a group represented by the following formula: 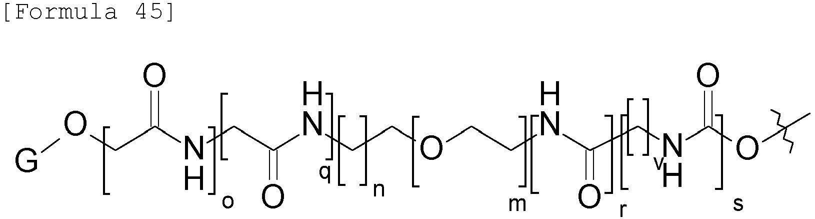 G represents a 5-acetoamido-2-hydroxymethyl-3,4-dihydroxytetrahydropyran-6-yl group (GalNAc); Z represents an oxygen atom or a sulfur atom; one of L¹ and L² represents a methylene group (CH₂), and the other represents no atoms interposed; o, q, r, and s each independently represent 0 or 1; n represents an integer of 1 to 15; m represents an integer of 0 to 5; and v represents 1 to 7, provided that when n is 1, then m is an integer of 0 to 5, and when n is an integer of 2 to 15, then m is 0, preferably o and q are each 0.

In the aforementioned formula, it is preferable that L² represents a methylene group (CH₂), and L¹ represents that no atoms are interposed, in L¹ and L². Preferably, n is an integer of 1 to 10, more preferably an integer of 2 to 7, further preferably an integer of 2 to 4. Preferably, m is an integer of 0 to 3. Preferably, v is an integer of 1 to 5. More preferably, s is 1, n is an integer of 2 to 7, m is 0, 1, or 2, and v is an integer of 1 to 5, further preferably, it is a group in which s is 1, n is 2 or 3, m is 0, and v is an integer of 1 to 3, in the group represented by the aforementioned formula.

The biantennary GalNAc unit of the present invention preferably has a biantennary structure each having a glycerol structure within three atoms from the phosphate group and has a GalNAc at each of the ends of the branched chain, wherein each linker structure from the branched point to the GalNAc is almost the same, the linker skeleton connecting the GalNAc moiety and the phosphate group moiety has a length of 15 atoms or less, the heteroatom content is high, and a continuous alkyl chain length is C5 or less, thereby exhibiting good solubility in water.

Specific examples of the biantennary GalNAc unit of the present invention can include the groups represented by the following formulas: 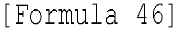 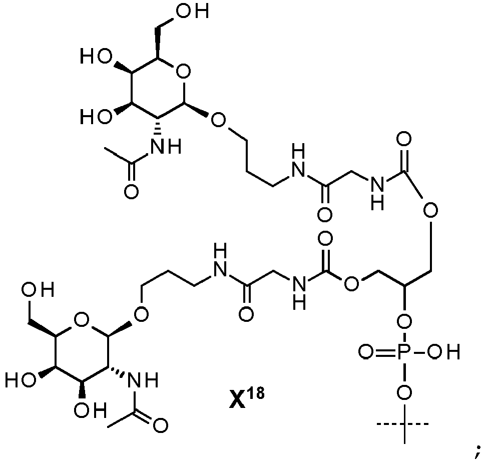 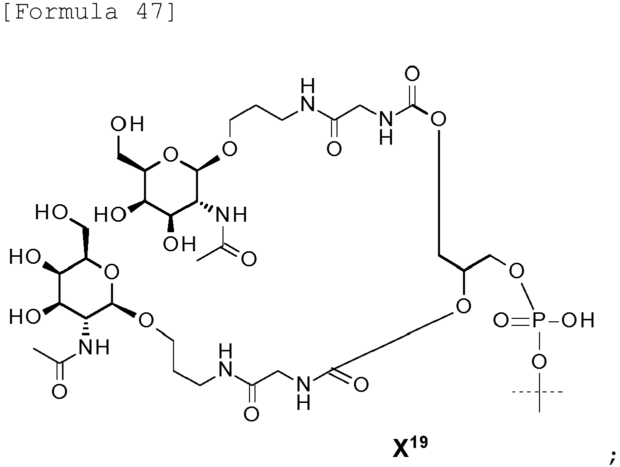 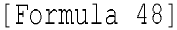 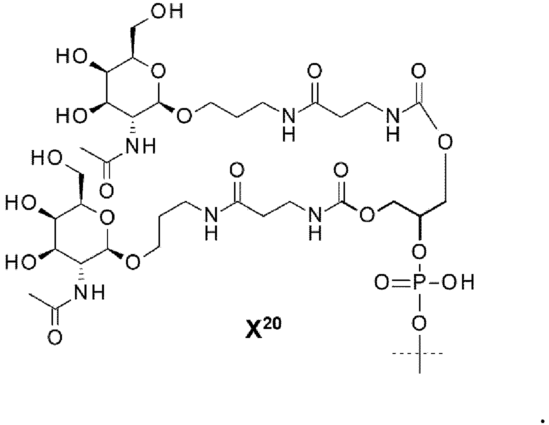 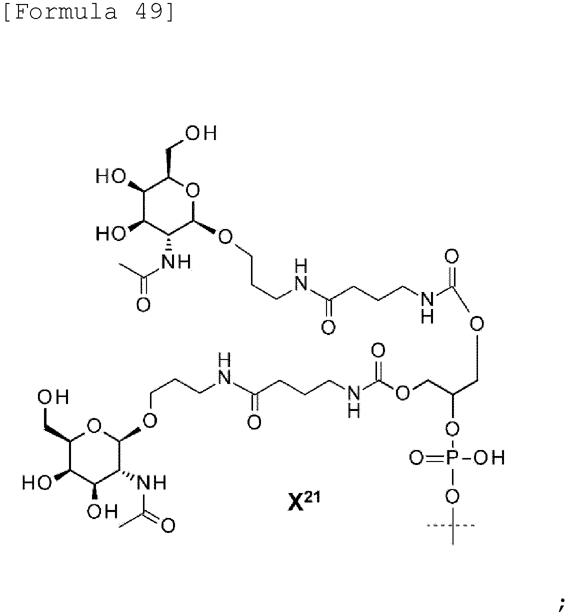 and

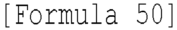

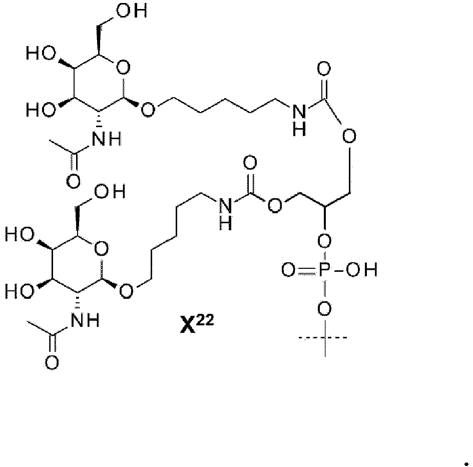

### <3. Method for producing conjugate>

The oligonucleotide to which the GalNAc unit of the present invention is bound can be synthesized according to the method described in the literature (Nucleic Acids Research, 12, 4539 (1984)) using a commercially available synthesizer (e.g., model 392, available from PerkinElmer, Inc., by the phosphoramidite method) or the like and by using a phosphoramidite contained in the GalNAc unit in the same manner as the nucleoside phosphoramidite reagent.

### Method for synthesizing GalNAc unit-containing phosphoramidite

A phosphoramidite or H-phosphonate containing the GalNAc unit of the present invention can be produced according to method A or method B, which will be described below. In method A or method B, the target compound of each reaction is collected from the reaction mixture after completion of the reaction according to conventional methods. For example, the target compound is obtained by appropriately neutralizing the reaction mixture, filtering out insoluble matter, if present, then adding water and an immiscible organic solvent such as ethyl acetate thereto, separating an organic layer containing the target substance, washing it with water or the like, drying it with anhydrous sodium sulfate, and distilling off the solvent. The compound obtained can be separated/purified by a conventional method, e.g., silica-gel column chromatography, if necessary. Alternatively, it can be purified by reprecipitation and recrystallization. Provided that, if r = 0, method C is used instead of method A or method B.

### [Method A]

Method A is a method for producing branched compound (7) and compound (7') using compound (3). The configuration of compound (1) is not limited. By using compound (3), the protecting group for the hydroxyl group at position 6 of the GalNAc moiety can be appropriately selected. If r = 0, method C is used. 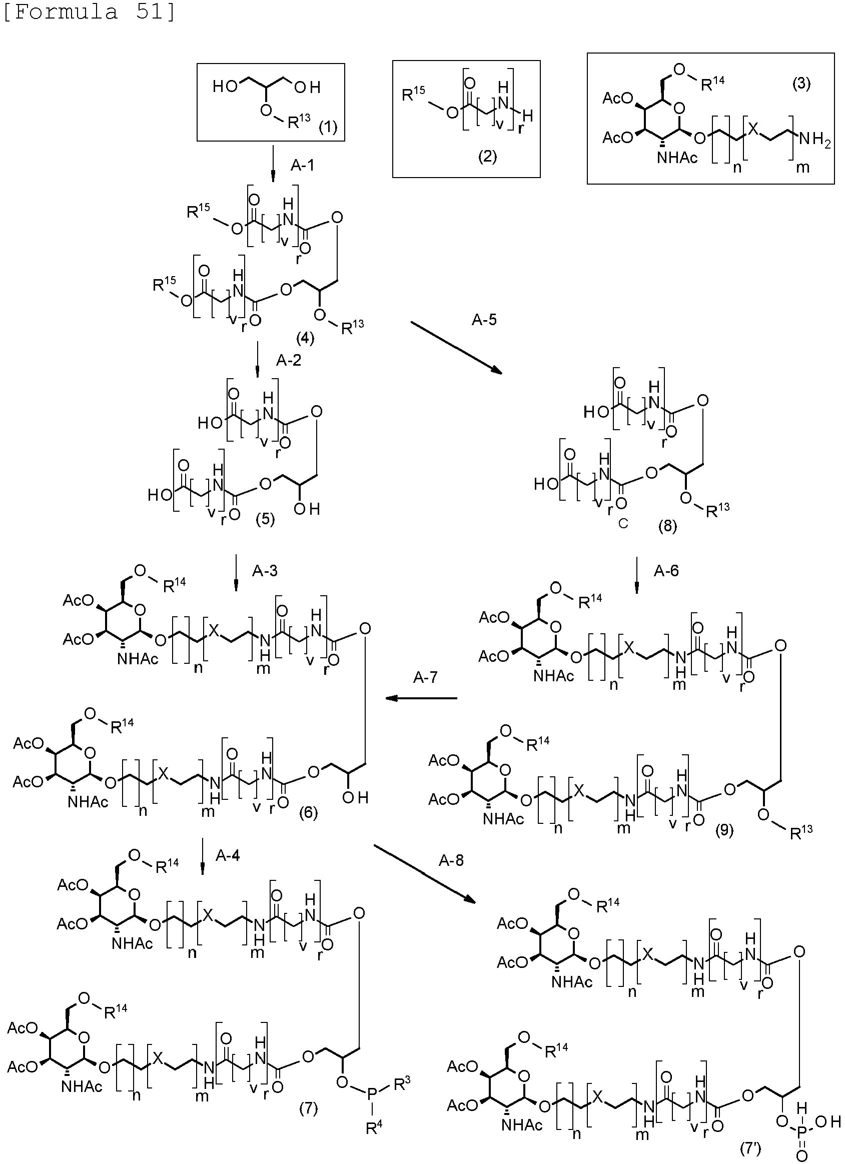 wherein R¹³ is a conventional protecting group for hydroxyl groups but is desirably a benzyl group, R¹⁴ is a conventional protecting group for hydroxyl groups but is desirably an acetyl group or a hydrophobic protecting group (more desirably, a 4,4'-dimethoxytrityl group), R¹⁵ is a conventional protecting group for carbonyl groups but is desirably a benzyl group, in -P(R³)R⁴, R³ and R⁴ may be the same or different and each represent a hydroxyl group, a protected hydroxyl group, a mercapto group, a protected mercapto group, an amino group, an alkoxy group having 1 to 4 carbon atoms, an alkylthio group having 1 to 4 carbon atoms, a cyanoalkoxy group having 1 to 5 carbon atoms, or an amino group substituted with an alkyl group having 1 to 4 carbon atoms, X is carbon or oxygen, n is an integer of 1 to 15, m is an integer of 0 to 5, provided that m is an integer of 0 to 5 when n is 1, and m is 0 when n is an integer of 2 to 15, r is independently 0 or 1, and v is 1 to 7.

Step A-1 is a carbamation step, in which compound (1) is converted into an active ester in an inert solvent in the presence of an activator and a base and is then reacted with compound (2) for carbamation.

Examples of the inert solvent to be used in the reaction include hydrocarbons, aromatic hydrocarbons, hydrocarbon halides, and ethers, preferably dichloromethane.

The activator to be used in the reaction is not particularly limited, as long as it forms an activated ester, but examples thereof include bis(pentafluorophenyl) carbonate, bis(4-nitrophenyl) carbonate, and 4-nitrophenyl chloroformate. Preferably, the activator is 4-nitrophenyl chloroformate.

Examples of the base to be used in the reaction include triethylamine, N,N-diisopropylethylamine, pyridine, and 4-dimethylaminopyridine, and it is preferable that pyridine be used when preparing an active ester, and N,N-diisopropylethylamine be added when subsequently reacting with amine.

The reaction temperature differs depending on the activator, the base, the inert solvent, and the like, but is generally from -20°C to reflux temperature. Preferably, it is from 10°C to 30°C.

The reaction time differs depending on the activator, the base, the inert solvent, the reaction temperature, and the like, but is generally from 15 minutes to 24 hours, preferably 1 hour to 4 hours.

Step A-2 is a step of removing R¹³ and R¹⁵, which are protecting groups. The removal of the protecting groups differs depending on the type thereof but can be generally performed according to well-known methods in synthetic organic chemistry technology such as the methods disclosed in T. H. Greene, P. G. Wuts, Protective Groups in organic Synthesis. Third Edition, 1999, John Wiley & Sons, Inc.

Step A-3 is a step of amide condensation with compound (3) in an inert solvent in the presence of a condensing agent and a base.

The inert solvent to be used in the reaction can be appropriately selected depending on the type of the condensing agent to be used. Examples thereof include water, alcohols, and polar aprotic solvents, preferably dichloromethane or N,N-dimethylformamide.

Examples of the base to be used in the reaction include triethylamine, N,N-diisopropylethylamine, pyridine, and 4-dimethylaminopyridine, preferably N,N-diisopropylethylamine.

Examples of the condensing agent to be used in the reaction include WSC (1-[3-(dimethylamino)propyl]-3-ethylcarbodiimide) and DIC (N,N'-diisopropylcarbodiimide) that are carbodiimide condensing agents, CDI (N,N'-carbonyldiimidazole) that is an imidazole condensing agent, DMT-MM (4-(4,6-dimethoxy-1,3,5-triazin-2-yl)-4-methylmorpholinium = chloride n hydrate) that is a triazine condensing agent, HATU (O-(7-azabenzotriazole-1-yl)-N,N,N',N'-tetramethyluronium hexafluorophosphate) and TSTU (O-(N-succinimidyl)-N,N,N',N'-tetramethyluronium tetrafluoroborate) that are uronium condensing agents, and PyBOP (1H-benzotriazol-1-yloxytripyrrolidinophosphonium hexafluorophosphate) that is a phosphonium condensing agent, preferably WSC or HATU. Further, HOAt (1-hydroxy-7-azabenzotriazole), HOBt (1-hydroxybenzotriazole), and Oxyma (ethyl (hydroxyimino)cyanoacetate) that are additives may be added, as required.

The reaction temperature differs depending on the condensing agent, the base, the solvent, and the like, but is generally from -20°C to reflux temperature. Preferably, it is from 10°C to 30°C.

The reaction time differs depending on the condensing agent, the base, the solvent, the reaction temperature, and the like, but is generally from 15 minutes to 72 hours, preferably from 1 hour to 24 hours.

Step A-4 is a step of producing compound (7), in which -P(R³)R⁴ is introduced into a secondary hydroxyl group of compound (6) in an inert solvent in the presence of 2-cyanoethyldiisopropylchlorophosphoroamidite and a base.

Examples of the inert solvent to be used in the reaction include hydrocarbons, aromatic hydrocarbons, hydrocarbon halides, ethers, and acetonitrile, preferably dichloromethane.

Examples of the base to be used in the reaction include triethylamine, N,N-diisopropylethylamine, pyridine, and 4-dimethylaminopyridine, preferably N,N-diisopropylethylamine.

The reaction temperature differs depending on the base, the inert solvent, and the like, but is generally from -20°C to reflux temperature. Preferably, it is from 10°C to 30°C.

The reaction time differs depending on the base, the inert solvent, the reaction temperature, and the like, but is generally from 15 minutes to 24 hours, preferably from 1 hour to 4 hours.

Step A-5 is a step of synthesizing compound (8) from compound (7), in which only R¹⁵ that is a protecting group is removed. The removal of the protecting group differs depending on the type thereof but can be generally performed according to well-known methods in synthetic organic chemistry technology such as the methods disclosed in T.H.Greene, P.G.Wuts, Protective Groups in organic Synthesis. Third Edition, 1999, John Wiley & Sons, Inc.

Step A-6 is a step of amidating compound (8). It can be performed in the same manner as in step A-3 above.

Step A-7 is a step of removing R¹³ that is a protecting group. The removal of the protecting group differs depending on the type thereof but can be generally performed according to well-known methods in synthetic organic chemistry technology such as the methods disclosed in T.H.Greene, P.G.Wuts, Protective Groups in organic Synthesis. Third Edition, 1999, John Wiley & Sons, Inc.

Step A-8 is a step of synthesizing compound (7') that is an H-phosphonate from compound (6). An H-phosphonate can be obtained, for example, by reacting tris-(1,2,4-triazolyl) phosphite prepared in advance from phosphorus trichloride and 1,2,4-triazole according to the literature (B. C. Freohler, P. G. Ng and MD. Matteucci, Nucleic Acids Res., 14 5399 (1986)), instead of the phosphoramidite reagent, with compound (6) in an inert solvent, then adding water thereto to terminate the reaction, and performing post-treatment. The solvent to be used is not particularly limited, as long as it does not inhibit the reaction, but is preferably a halogenated hydrocarbon such as methylene chloride. The reaction temperature is not particularly limited within a range from -20° to 100°C but is generally room temperature. The reaction time differs depending on the solvent to be used and the reaction time but is 30 minutes in the case of a reaction in methylene chloride at room temperature.

### [Method B]

Method B is a method for producing branched compound (14) and compound (14') using compound (3). The configuration of compound (10) is not limited. By using compound (3), the protecting group for the hydroxyl group at position 6 of the GalNAc moiety can be appropriately selected. If r = 0, method C is used.

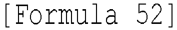

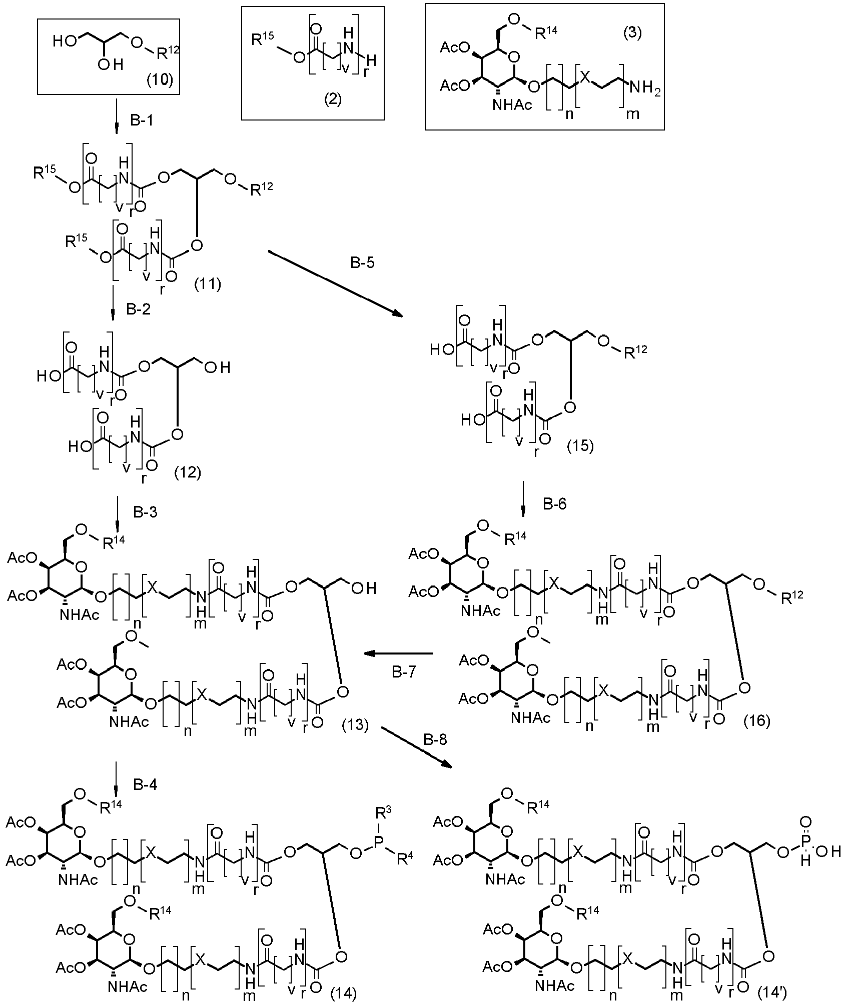

R¹² is a conventional protecting group for hydroxyl groups but is desirably a benzyl group. In the formula, R³, R⁴, X, n, m, r, v, R¹⁴, and R¹⁵ each have the same meaning as described above.

Step B-1 is a step of carbamating compound (10) and can be performed in the same manner as in step A-1 above.

Step B-2 is a step of removing R¹² and R¹⁵ that are protecting groups. Step B-2 can be performed in the same manner as in step A-2 above.

Step B-3 is a step of amidating compound (12) and can be performed in the same manner as in step A-3 above.

Step B-4 is a step of introducing -P(R³)R⁴ into a hydroxyl group of compound (13) and can be performed in the same manner as in step A-3 above.

Step B-5 is a step of synthesizing compound (15) from compound (11), in which only R¹⁵ that is a protecting group is removed. Step B-5 can be performed in the same manner as in step A-5 above.

Step B-6 is a step of amidating compound (15). Step B-6 can be performed in the same manner as in step A-6 above.

Step B-7 is a step of removing R¹² that is a protecting group. Step B-7 can be performed in the same manner as in step A-7 above.

Step B-8 is a step of synthesizing compound (14') from compound (13). Step B-8 can be performed in the same manner as in step A-8 above.

### [Method C]

Method C is a method for producing branched compound (19) or compound (22), and compound (19') or compound (22') using compound (3). The configurations of compound (1) and compound (10) are not limited. By using compound (3), the protecting group for the hydroxyl group at position 6 of the GalNAc moiety can be appropriately selected.

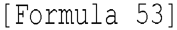

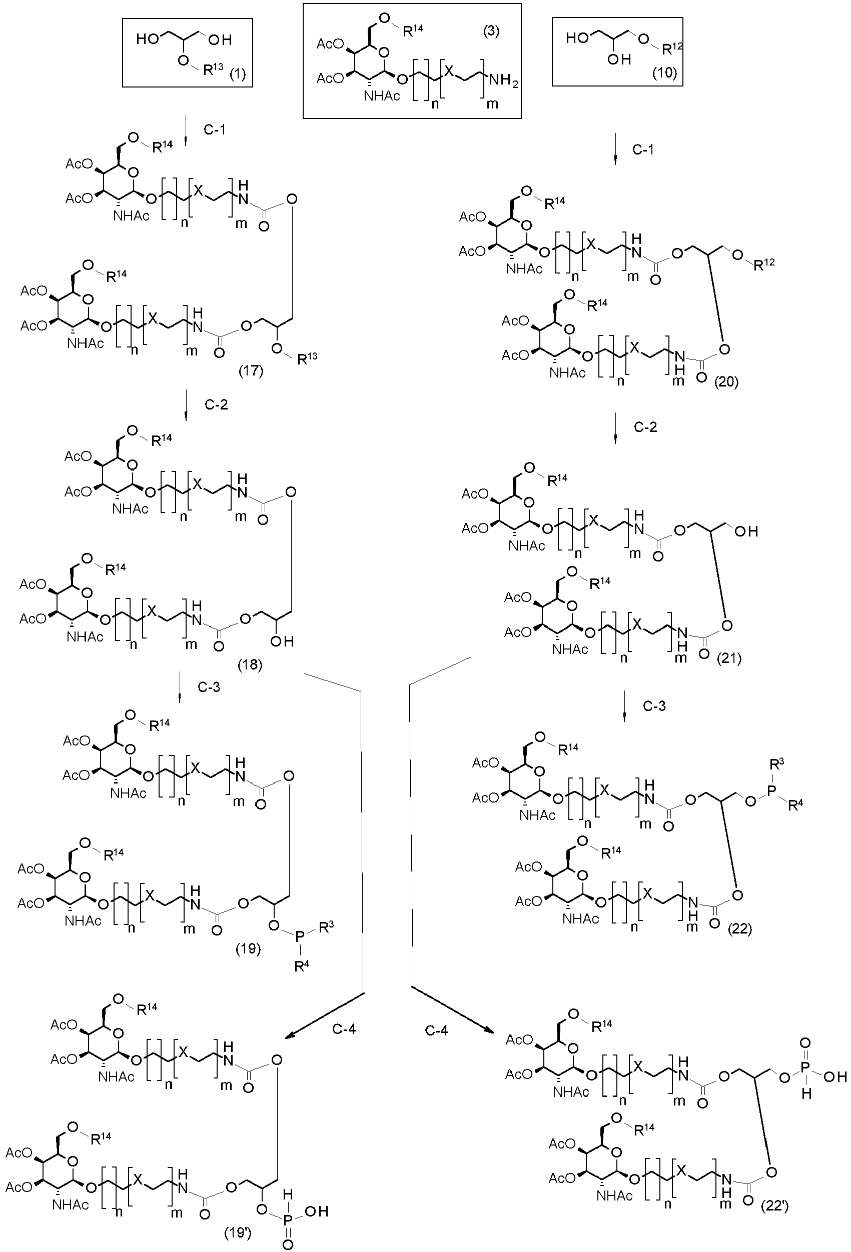

wherein R³, R⁴, X, n, m, r, R¹², R¹³, and R¹⁴ each have the same meaning as described above.

Step C-1 is a step of carbamating compound (1) or compound (10) and can be performed in the same manner as in step A-1 above using compound (3) instead of compound (2) .

Step C-2 is a step of removing R¹² or R¹³ that is a protecting group. The removal of the protecting group differs depending on the type thereof but can be generally performed according to well-known methods in synthetic organic chemistry technology such as the methods disclosed in T.H.Greene, P.G.Wuts, Protective Groups in organic Synthesis. Third Edition, 1999, John Wiley & Sons, Inc.

Step C-3 is a step of introducing -P(R³)R⁴ into a hydroxyl group of compound (19) or compound (22) and can be performed in the same manner as in step A-3 above.

Step C-4 is a step of synthesizing compound (19') from compound (18) or synthesizing compound (22') from compound (21). Step C-4 can be performed in the same manner as in step A-8 above.

### <4. Production method using hydrophobic protecting group>

The present invention provides: a method for producing a conjugate of a GalNAc unit with a synthetic oligonucleotide by binding a GalNAc unit, into which a highly hydrophobic protecting group is introduced on one or more hydroxyl groups at any of position 3, position 4, and position 6 of the GalNAc moiety, to the 5' end or 3' end of the oligonucleotide, and separating the GalNAc unit from impurities by adsorption by a resin having adsorptivity due to hydrophobic interaction; a binding compound (such as phosphoramidite and H-phosphonate) comprising a hydrophobic protecting group-containing GalNAc to be used as an intermediate in the production method; a conjugate; and the like.

The production method of the present invention includes the following steps of:
a: synthesizing a conjugate of a structure comprising a hydrophobic protecting group-containing GalNAc unit-phosphate group bound to the 5' end or the 3' end of an oligonucleotide;
b: passing a solution containing the product obtained in step a through a column filled with a resin having adsorptivity due to hydrophobic interaction and passing an aqueous washing solution through the column; and
c: eluting the oligonucleotide from the column after step b.

In this production method, various hydrophobic protecting groups can be adopted, as long as they meet the aforementioned definition. In normal nucleic acid synthesis, an oligonucleotide is released from a support by a basic treatment using ammonia water or the like, and therefore protecting groups that are removed by treatment under acidic conditions, such as optionally substituted trityl groups and optionally substituted silyl groups can be used. Specific examples of the optionally substituted trityl groups include a trityl group, a monomethoxytrityl group, and a dimethoxytrityl group. Examples of the optionally substituted silyl groups include a triphenyl silyl group, a tert-butyldimethylsilyl group, and a tert-butyldiphenylsilyl group, preferably a 4,4'-dimethoxytrityl group (DMTr group).

Various ester groups can be adopted as the hydrophobic protecting group to be removed under basic conditions, but preferable examples thereof include an acetyl group and a benzoyl group.

In the present invention, the term "structure comprising a hydrophobic protecting group-containing GalNAc unit-phosphate group" refers to a structure represented by the following formula.

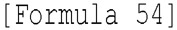

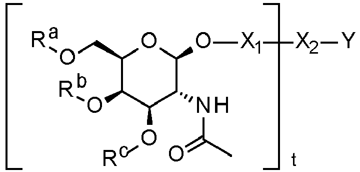

In the formula, at least one of R^{a}, R^{b}, and R^{c}, which are substituents of hydroxyl groups on GalNAc, is a hydrophobic protecting group, and the others are hydrogen atoms or protecting groups to be removed under release conditions. All of them may be hydrophobic protecting groups, any two of them may be hydrophobic protecting groups, or only any one of them may be a hydrophobic protecting group. Preferably, R^{a} is a hydrophobic protecting group (more preferably, a hydrophobic protecting group to be removed under acidic conditions, further preferably an optionally substituted trityl group, most preferably a DMTr group), and R^{b} and R^{c} are hydrogen atoms. In the case where this substituent is not a hydrophobic protecting group, protecting groups to be removed under release conditions may be bound thereto, but these protecting groups are removed in the process of cleaving the oligonucleotide from the support so as to be hydrogen atoms (hydroxyl groups on GalNAc) at the stage when used in step a.

X₁ and X₂ each independently represent a linker structure, which may be branched between X₁ and X₂. As the linker structure to be adopted herein, various skeletons such as an alkyl chain, a PEG chain, and a peptide chain can be adopted. This linker structure may contain a plurality of GalNAcs by branching in the middle. The number of branched chains (t) in the linker structure is about 1 to 10, preferably 1 to 3. In the case of having two or more branched chains, the linker structures between the branched points and the GalNAcs may be the same or different. In the case of containing a plurality of GalNAcs, at least one hydrophobic protecting group is bound to each GalNAc.

In the aforementioned formula, Y represents a bond which is bound to the oligonucleotide via a phosphate group or a thiophosphate group. In the case of binding to the 5' end of the oligonucleotide, a phosphodiester bond or a phosphorothioate bond is formed together with the 5'-hydroxyl group of the nucleoside at the end. Such binding can be synthesized by solid-phase synthesizing the oligonucleotide and then using a compound (hydrophobic protecting group-containing GlcNAc unit amidite-like compound) in which Y having a structure comprising a hydrophobic protecting group-containing GalNAc unit-phosphate group forms a phosphoramidite structure or an H-phosphonate structure, in the same manner as the nucleoside phosphoramidite reagent. Specific examples of the hydrophobic protecting group-containing GalNAc unit amidite-like compound corresponding to the bisecting GalNAc unit of the present invention are described in (B13) above.

The production method of the present invention is not limited in application to the biantennary GalNAc unit of the present invention mentioned above and can be applied also to the production of a conjugate of a known GalNAc unit (e.g., those described in Patent Literature 1 to 9 and Non Patent Literature 1 and 2) with an oligonucleotide. In this case, a compound with a structure corresponding to the known GlcNAc unit is adopted as the hydrophobic protecting group-containing GlcNAc unit phosphoramidite or the H-phosphonate. Examples of the amidite-like compound corresponding to the known GalNAc unit can include the following compounds.

The following compounds can be produced with reference to J. Med. Chem. 2016, 59, 2718-2733, Cited References thereof, and the like.

### For Tris based GalNAc unit

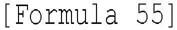

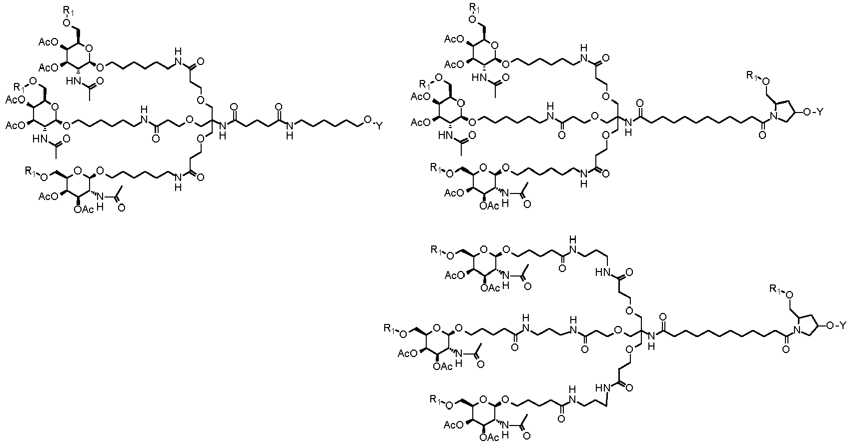

### For Triacid based GalNAc unit

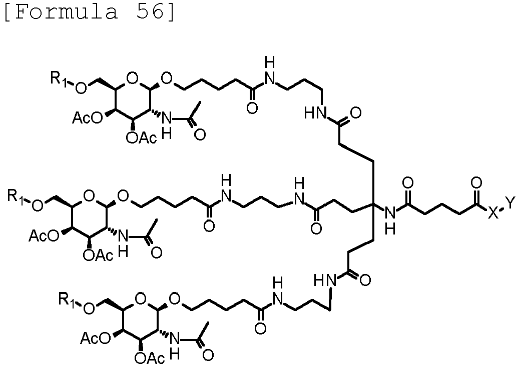

### For Pentaerythritol based GalNAc unit

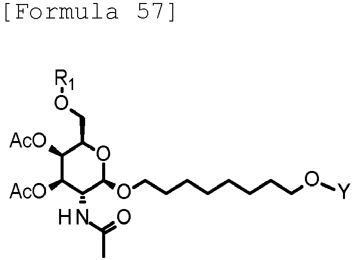

### For Lys based GalNAc unit

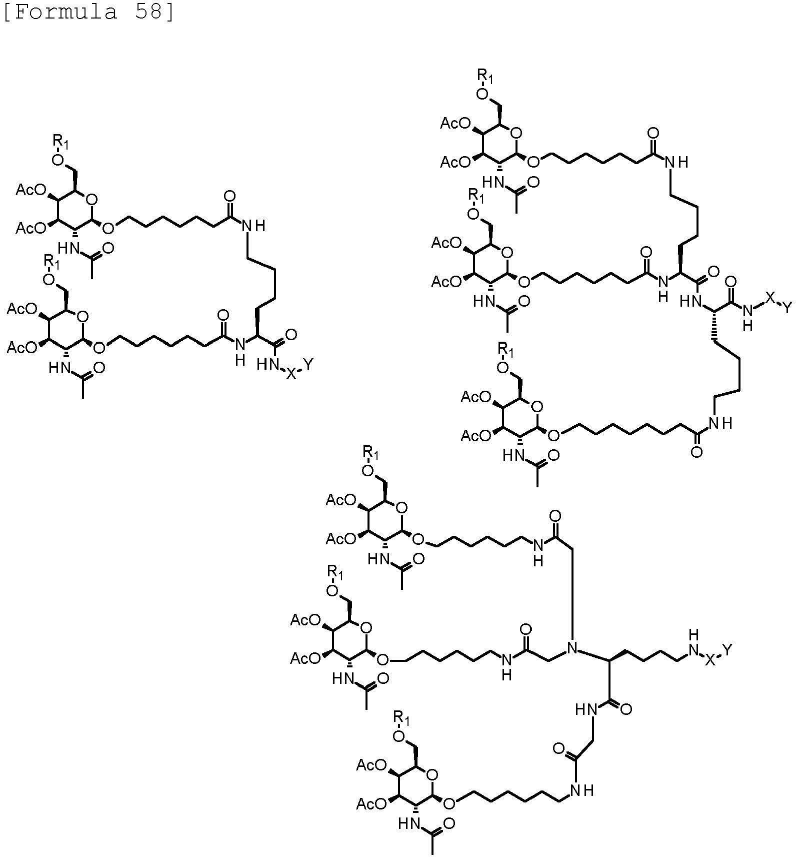

The hydrophobic protecting group-containing GalNAc unit phosphoramidite represented by the following formula can be produced with reference to International Publication No. WO 19027009.

### For Aryl based GalNAc unit

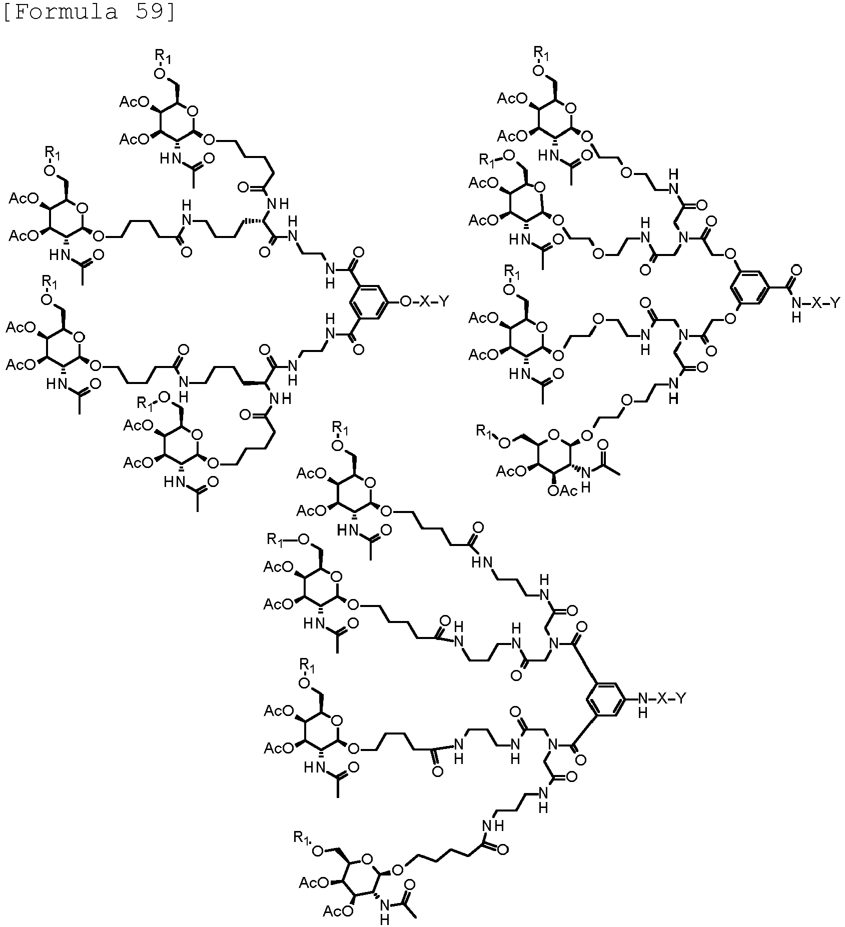

The following compound can be produced with reference to J. Med. Chem. 2016, 59, 2718-2733, Cited References, and the like.

### For Hydroxyprolinol based GalNAc unit/Piperidine based GalNAc unit

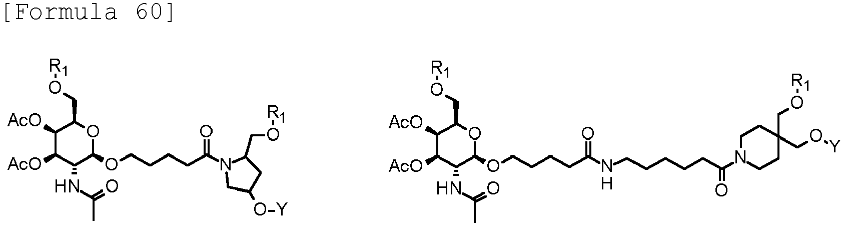

In the aforementioned formula of the amidite-like compound corresponding to the known GlcNAc unit, R₁ represents a hydrophobic protecting group. X represents a linker structure, preferably C3 to C6 straight chain alkyl. Y represents a phosphoramidite group or an H-phosphonate group.

The hydrophobic protecting group-containing GalNAc unit-phosphoramidite or the hydrophobic protecting group-containing GalNAc unit-H-phosphonate corresponding to the known GlcNAc unit can be synthesized by appropriately using GalNAc derivatives falling under compound (3) described above in method A to method C in the process of synthesizing the GalNAc unit. Various GalNAc derivatives with at least one of the hydroxyl groups at position 3, position 4, and position 6 protected by a hydrophobic protecting group can be synthesized, for example, by referring to the method of Example 1.

A conjugate having a structure comprising a hydrophobic protecting group-containing GalNAc-phosphate group (in the aforementioned formula, Y represents a bond which is bound to the oligonucleotide via a phosphate group or a thiophosphate group) can be synthesized by introducing the hydrophobic protecting group-containing GalNAc unit amidite-like compound as mentioned above into the 5' end or the 3' end of the oligonucleotide synthesized in the same manner as the nucleoside amidite reagent according to the aforementioned method. Examples of the thus synthesized conjugate can include the conjugates described in (B17) and (B18).

The solvent to be used for the reaction to introduce the hydrophobic protecting group-containing GalNAc unit-H-phosphonate compound into the 5' end or the 3' end of the oligonucleotide synthesized is not particularly limited, as long as it does not inhibit the reaction. Preferably, anhydrous acetonitrile is used. Examples of the reagent to be used as a condensing agent include acid chlorides of carboxylic acid and phosphoric acid, preferably pivaloyl chloride. The oxidant to oxidize the H-phosphonate bond into a phosphodiester bond is not particularly limited, as long as it is generally used for oxidation reactions. Examples thereof can include inorganic metal oxidants including manganese oxides such as potassium permanganate and manganese dioxide; ruthenium oxides such as ruthenium tetroxide; selenium compounds such as selenium dioxide; iron compounds such as iron chloride; osmium compounds such as osmium tetroxide; silver compounds such as silver oxide; mercury compounds such as mercury acetate; lead oxide compounds such as lead oxide and lead tetroxide; chromate compounds such as potassium chromate, chromium acid-sulfate complex, and chromium acid-pyridine complex; and cerium compounds such as cerium ammonium nitrate (CAN), inorganic oxidants including halogen molecules such as chlorine molecules, bromine molecules, and iodine molecules; periodic acids such as sodium periodate; ozone; hydrogen peroxide solution; nitrous acid compounds such as nitrous acid; chlorous acid compounds such as potassium chlorite, sodium chlorite; and persulfate compounds such as potassium persulfate and sodium persulfate, and organic oxidants including reagents to be used for DMSO oxidation (complex of dimethylsulfoxide and dicyclohexylcarbodiimide, oxalyl chloride, acetic anhydride, or phosphorus pentoxide, or pyridine-acetic anhydride complex); peroxides such as t-butyl hydroperoxide; stable cations such as triphenyl methyl cation; succinimides such as N-bromo succinimide; hypochlorite compounds such as t-butyl hypochlorite; azodicarboxylate compounds such as methyl azodicarboxylate; disulfides such as dimethyl disulfide, diphenyl disulfide, and dipyridyldisulfide and triphenylphosphine; nitrite esters such as methyl nitrite; tetrahalocarbons such as methane tetrabromide; and quinone compounds such as 2,3-dichloro-5,6-dicyano-p-benzo quinone (DDQ), preferably iodine molecules. Examples of the deoxidant to be used include heterocyclic amines such as pyridine and dimethylaminopyridine, and aliphatic amines such as trimethylamine, triethylamine, and diisopropylethylamine, preferably heterocyclic amines (particularly, pyridine). In the case of forming a phosphorothioate bond, there is no limitation, as long as it is a reagent capable of forming a phosphorothioate bond by reacting the H-phosphonate bond. Examples thereof include sulfur, tetraethylthiuram disulfide, Beaucage reagent, and phenyl acetyl disulfide, preferably sulfur. The conjugate synthesized can be released from the support while retaining the hydrophobic protecting group in the same manner as in general DMTr-on purification of oligonucleotides.

Such a conjugate having a structure comprising a hydrophobic protecting group-containing GalNAc unit-phosphate group can be released from the support while retaining the hydrophobic protecting group in the same manner as in general DMTr-on purification of oligonucleotides. In general, the bond between the 3' end of an oligonucleotide and a support is cleaved with ammonia water, and the oligonucleotide is released under basic conditions. Therefore, a protecting group that is not removed by basicity, for example, a group that is removed under acidic conditions is selected as the hydrophobic protecting group. Meanwhile, in the case of adopting a binding mode in which the bond between the 3' end of the oligonucleotide and the support is cleaved under acidic conditions as the binding mode, the release conditions of the oligonucleotide are acidic, and therefore a protecting group that is not removed under acidic conditions, for example, a protecting group that is removed under basic conditions is selected as the hydrophobic protecting group.

The hydrophobic protecting group-containing GalNAc unit-oligonucleotide conjugate thus obtained is adsorbed to a column filled with a resin having adsorptivity due to hydrophobic interaction, and then the column is washed with an aqueous washing solution, so that impurities having no hydrophobic protecting groups can be separated.

The "resin having adsorptivity due to hydrophobic interaction" is not particularly limited, as long as it is a resin in which highly hydrophobic components are used for the substrate. Various resins are used therefor, and examples thereof include styrenedivinylbenzene adsorption resins, modified styrenedivinylbenzene adsorption resins, methacrylic adsorption resins, or phenolic adsorption resins.

As the filling solution to be used when the hydrophobic protecting group-containing GalNAc unit-oligonucleotide conjugate is adsorbed to the column, a basic aqueous solution that does not contain a large amount of organic solvent is desirable. For example, concentrated ammonia water used for cutting out the oligomer from the support after the solid phase synthesis or a basic aqueous solution obtained by diluting the ammonia water with an appropriate amount of solvent (such as water and buffer) can be used.

Various solutions can be adopted as the washing solution to be used for removing impurities from the column, as long as they are solutions having properties such that the hydrophobic protecting group does not undergo deprotection, and the hydrophobic interaction with the resin is maintained. The efficiency of removing impurities is improved by using an aqueous solution containing an organic solvent having a sufficiently low concentration to maintain the hydrophobic interaction with the resin as the washing solution. Specifically, an aqueous solution containing an appropriate amount of salt, an aqueous solution containing acetonitrile or the like can be used as the washing solution in the case where the hydrophobic protecting group is a DMTr group.

The hydrophobic protecting group bound to GalNAc can undergo deprotection by treating the conjugate under deprotection conditions corresponding to the protecting group adopted. In the case of applying this production method following general oligonucleotide synthesis, a hydrophobic protecting group that undergoes deprotection under acidic conditions such as a DMTr group is adopted, and therefore the deprotection is performed by treatment with an acidic solution.

The deprotection treatment is appropriately performed on the column adsorbing the conjugate, thereby enabling a deprotected GalNAc-oligonucleotide conjugate to be obtained while impurities derived from the protecting group are adsorbed onto the column.

Further, the impurities derived from the protecting group can be separated from the deprotected conjugate by performing deprotection treatment the hydrophobic protecting group-containing GalNAc-oligonucleotide conjugate that has not been deprotected and eluted from the column and passing the solution after the deprotection treatment again through the same resin column for adsorption due to hydrophobic interaction.

The GalNAc-oligonucleotide conjugate can be fractionated with a high degree of purification by subjecting the column retaining the conjugate to an appropriate gradient elution method corresponding to the resin adopted. In the case of elution from the column without deprotecting the hydrophobic protecting group, the conjugate can be eluted by gradient elution, which increases the content of organic solvent, to inhibit the hydrophobic interaction between the hydrophobic protecting group and the adsorption resin.

In the case where the deprotection treatment is performed in the adsorption state on the column, appropriate elution conditions can be appropriately selected according to the mode of retaining the deprotected conjugate on the column.

In the case of using an ion exchange column having adsorptivity due to hydrophobic interaction, deprotection treatment is performed while the conjugate is adsorbed onto the column, and the GalNAc-oligonucleotide conjugate can be eluted/fractionated from the column according to a conventional method to be adopted for elution from an ion exchange column using the salt concentration in the eluent. Specifically, in the case of using RESOURCE Q (available from GE Healthcare Japan Corporation) as the column, a gradient eluent which gradually increases the content of sodium chloride in an aqueous sodium hydroxide/sodium chloride mixed solution from 0 to 2 M (2 mol/L) can be used.

In the case of using a reverse phase column, the GalNAc-oligonucleotide conjugate can be eluted/fractionated from the column according to a conventional method to be adopted for elution from a reverse phase column using a suitable organic solvent. Specifically, in the case of using Clarity QSP (available from Phenomenex Inc.) as the column, a trishydroxymethylaminomethane (Tris)-hydrochloric acid buffer containing an appropriate amount of acetonitrile can be used.

For detecting the target substance, absorbance at 260 nm, which is the detection wavelength of nucleic acids, is used. The target substance can be obtained by measuring the absorbance at 260 nm of the passing fluid of the column over time and collecting the fraction to be detected in the elution step.

### <5. Medicament>

The conjugate of the present invention can be used as a medicament for treating a disease targeted by the oligonucleotide contained therein. The treatment includes both prophylactic and post treatment.

The conjugate of the present invention may be used in the form of a pharmaceutically acceptable salt. The "pharmaceutically acceptable salt" refers to a salt of the oligonucleotide, and examples of such a salt can include metal salts including alkali metal salts such as sodium salt, potassium salt, and lithium salt, alkaline earth metal salts such as calcium salt and magnesium salt, aluminum salt, iron salt, zinc salt, copper salt, nickel salt, and cobalt salt; amine salts including inorganic salts such as ammonium salt, and organic salts such as t-octylamine salt, dibenzyl amine salt, morpholine salt, glucosamine salt, phenylglycine alkyl ester salt, ethylenediamine salt, N-methylglucamine salt, guanidine salt, diethylamine salt, triethylamine salt, dicyclohexylamine salt, N,N'-dibenzylethylenediamine salt, chloroprocaine salt, procaine salt, diethanolamine salt, N-benzyl-phenethyl amine salt, piperazine salt, tetramethyl ammonium salt, and tris(hydroxymethyl) aminomethane salt; inorganic acid salts including hydrohalides such as hydrofluorate, hydrochloride, hydrobromide, and hydroiodide, nitrates, perchlorates, sulfates, and phosphates; organic acid salts including lower alkane sulfonates such as methanesulfonate, trifluoromethanesulfonate, and ethanesulfonate, aryl sulfonates such as benzenesulfonate and p-toluenesulfonate, acetates, malates, fumarates, succinates, citrates, tartrates, oxalates, and maleates; and amino acid salts such as glycine salt, lysine salt, arginine salt, ornithine salt, glutamate, and aspartate, preferably alkali metal salts, more preferably sodium salt. These salts can be produced by known methods.

Further, the conjugate and the pharmaceutically acceptable salt thereof may be present also as solvates (e.g., hydrates) and such solvates may be used. In the present invention, such a solvate is treated as being within the scope of the salt of the oligonucleotide or the conjugate.

In the case of using the conjugate or the pharmaceutically acceptable salt thereof of the present invention as a pharmaceutical product, the pharmaceutical product itself, or mixed with an appropriate pharmaceutically acceptable excipient or a diluent, can be orally administered in the form of a tablet, a capsule, granules, powder, or syrup or parenterally administered in the form of an injection, a suppository, a patch, or an external medicine.

These preparations are produced by well-known methods using additives such as excipients (e.g., organic excipients including sugar derivatives such as lactose, white sugar, dextrose, mannitol, and sorbitol; starch derivatives such as corn starch, potato starch, α starch, and dextrin; cellulose derivatives such as crystalline cellulose; gum arabic; dextran; and pullulan, and inorganic excipients including silicate derivatives such as light anhydrous silicate, synthetic aluminum silicate, calcium silicate, and magnesium aluminate metasilicate; phosphates such as calcium hydrogen phosphate; carbonates such as calcium carbonate; and sulfates such as calcium sulfate), lubricants (e.g., stearic acid; metal stearates such as calcium stearate and magnesium stearate; talc; colloid silica; waxes such as beeswax and gay wax; boric acid; adipic acid; sulfates such as sodium sulfate; glycol; fumaric acid; sodium benzoate; DL leucine; lauryl sulfates such as sodium lauryl sulfate and magnesium lauryl sulfate; silicic acids such as anhydrous silicate and silicate hydrate; and the starch derivatives mentioned above), binders (e.g., hydroxypropyl cellulose, hydroxypropylmethylcellulose, polyvinylpyrrolidone, macrogol, and the same compounds as described for the excipients above), disintegrants (e.g., cellulose derivatives such as hydroxypropyl cellulose with low degree of substitution, carboxymethylcellulose, carboxymethylcellulose calcium, and internally crosslinked sodium carboxymethyl cellulose; and chemically modified starch/celluloses such as carboxymethyl starch, sodium carboxymethyl starch, and crosslinked polyvinylpyrrolidone), emulsifiers (e.g., colloidal clays such as bentonite and veegum; metal hydroxides such as magnesium hydroxide and aluminum hydroxide; anionic surfactants such as sodium lauryl sulfate and calcium stearate; cationic surfactants such as benzalkonium chloride; and non-ionic surfactants such as polyoxyethylene alkyl ether, polyoxyethylene sorbitan fatty acid ester, and sucrose fatty acid ester), stabilizers (paraoxybenzoic acid esters such as methylparaben and propylparaben; alcohols such as chlorobutanol, benzyl alcohol, and phenylethyl alcohol; benzalkonium chloride; phenols such as phenol and cresol; thimerosal; dehydroacetic acid; and sorbic acid), flavoring agents (e.g., sweeteners, acidulants, and flavors, which are commonly used), and diluents.

The medicament of the present invention may contain 0.1 to 250 µmoles/mL of an oligonucleotide (antisense oligonucleotide), preferably 1 to 50 µmoles/mL of an oligonucleotide or a pharmaceutically acceptable salt thereof, 0.02 to 10%w/v of a carbohydrate or a polyhydric alcohol, and 0.01 to 0.4%w/v of a pharmaceutically acceptable surfactant.

As the carbohydrate, monosaccharides or disaccharides are particularly preferable. Examples of the carbohydrate and the polyhydric alcohol include glucose, galactose, mannose, lactose, maltose, mannitol, and sorbitol. These may be used individually or in combination.

Further, preferable examples of the surfactant in the present invention include polyoxyethylene sorbitan mono- to tri-esters, alkylphenyl polyoxyethylene, sodium taurocholate, sodium cholate, and polyhydric alcohol esters. Among them, polyoxyethylene sorbitan mono- to tri-esters are particularly preferable, wherein oleates, laurates, stearates, and palmitates are particularly preferable as the esters. These may be used individually or in combination.

Further, the medicament of the present invention further preferably contains 0.03 to 0.09 M pharmaceutically acceptable neutral salts, such as sodium chloride, potassium chloride, and/or calcium chloride.

Further, the medicament of the present invention can further preferably contain a 0.002 to 0.05 M pharmaceutically acceptable buffer. Preferable examples of the buffer include sodium citrate, sodium glycinate, sodium phosphate, and tris(hydroxymethyl) aminomethane. These buffers may be used individually or in combination.

Further, the medicament may be supplied in the form of a solution. However, in the case where storage for a certain period is needed, freeze drying is usually preferable in order to stabilize the oligonucleotide to prevent a decrease in its therapeutic effect. In such a case, the medicament may be reconstituted with a lysate (such as distilled water for injection) (reconstitution), that is, into the form of a liquid to be administered in use. Accordingly, the medicament of the present invention also includes those in the freeze-dried state to be used after reconstitution with a lysate such that each component falls within a predetermined concentration range. Amino acids such as albumin and glycine may be further contained for the purpose of promoting the solubility of the freeze-dried product.

The conjugate of the present invention may be encapsulated using the lipid described in International Publication No. WO 2015/005253 or the like and may be administered as nucleic acid lipid nanoparticles or a liposome as described in International Publication No. WO 2015/005253 or the like.

In the case of administering the conjugate or the pharmaceutically acceptable salt thereof of the present invention to a human, an adult may have a dose of about 0.01 to 100 mg/kg (body weight), preferably 0.1 to 20 mg/kg (body weight) for one day, once or several times by subcutaneous injection, intravenous drip intravenous injection, or intravenous injection, for example. The dose and frequency of administration can be appropriately changed depending on the type of disease, symptoms, age, administration method, or the like.

### Examples

Hereinafter, the present invention will be described specifically by way of examples. These examples are shown for illustrating the present invention and do not limit the scope of the present invention. Any of "ABI 392 DNA/RNA Synthesizer" (available from Applied Biosystems), "ABI 394 DNA/RNA Synthesizer" (available from Applied Biosystems), AKTA Oligopilot (available from GE Healthcare), and nS-8II Synthesizer (Gene Design Inc.) were used as an automatic nucleic acid synthesizer, and the phosphoramidite method (Nucleic Acids Research, 12, 4539 (1984)) and a synthetic program corresponding to each synthetic scale were used.

Activator solution-3 (0.25 mol/L 5-benzyl thio-1H-tetrazole/acetonitrile solution, product No. 013-20011, available from Wako Pure Chemical Industries, Ltd.) was used as a condensation activator.

CAP A for AKTA (1-methylimidazole/acetonitrile solution, product No. L040050, available from Sigma-Aldrich Co. LLC), Cap B1 for AKTA (acetic anhydride/acetonitrile solution, product No. L050050, available from Sigma-Aldrich Co. LLC), or Cap B2 for AKTA (pyridine/acetonitrile solution, product No. L050150, available from Sigma-Aldrich Co. LLC) was used as a capping reagent.

DCA Deblock (dichloroacetate/toluene solution, product No. L023050, available from Sigma-Aldrich Co. LLC) or deblocking solution-1 (3w/v% trichloroacetic acid/dichloromethane solution, product No. 042-28921, available from FUJIFILM Wako Pure Chemical Corporation) was used as a deblock.

A phenyl acetyl disulfide (product No. FP07495, available from Carbosynth Holdings Limited) dissolved to 0.2 M using a 1:1 (v/v) solution of acetonitrile (dehydrated, product No. 01837-05, KANTO CHEMICAL CO., INC.) and pyridine (dehydrated, product No. 11339-05, KANTO CHEMICAL CO., INC.) was used as a thionation reagent for forming a phosphorothioate bond.

OXDIZER 0.05 M (product No. L560250-04, available from Sigma-Aldrich Co. LLC) or iodine (product No. 20035-00, available from KANTO CHEMICAL CO., INC.) dissolved to 0.02 M using a 78:20:2 (v/v/v) solution of tetrahydrofuran (dehydrated, product No. 40993-05, available from KANTO CHEMICAL CO., INC.), pyridine (dehydrated, product No. 11339-05, available from KANTO CHEMICAL CO., INC.), and distilled water was used as an oxidant.

A phosphoramidite of 2'-O-Me nucleoside (adenosine, product No. ANP-5751, cytidine, product No. ANP-5752, guanosine, product No. ANP-5753, or uridine, product No. ANP-5754) available from ChemGenes Corporation was used as a nucleoside phosphoramidite reagent.

As a phosphoramidite of a non-natural nucleoside, a compound according to Example 14 (5'-O-dimethoxytrityl-2'-O,4'-C-ethylene-6-N-benzoyladenosine-3'-O-(2-cyanoethyl N,N-diisopropyl) phosphoramidite), Example 27 (5'-O-dimethoxytrityl-2'-O,4'-C-ethylene-2-N-isobutyrylguanosine-3'-O-(2-cyanoethyl N,N-diisopropyl) phosphoramidite), Example 22 (5'-O-dimethoxytrityl-2'-O,4'-C-ethylene-4-N-benzoyl-5-methylcytidine-3'-O-(2-cyanoethyl N,N-diisopropyl) phosphoramidite), or Example 9 (5'-O-dimethoxytrityl-2'-O,4'-C-ethylene-5-methyluridine-3'-O-(2-cyanoethyl N,N-diisopropyl) phosphoramidite) of Japanese Patent Laid-Open No. 2000-297097 was used.

NittoPhase UnyLinker 100, 10 µmol (available from NITTO DENKO CORPORATION), Primer Support 5G UnyLinker 350, 10 µmol (available from GE Healthcare), Primer Support 5G Unylinker 350, 100 µmol (available from GE Healthcare), or Glen UnySupport CPG 500, 1 µmol (available from Glen Research) was appropriately selected according to the synthetic scale as a solid phase carrier.

### (Example 1) Synthesis of hydrophobic protecting group-containing GalNAc

### (1A) Synthesis of [(2R,3R,4R,5R)-5-acetoamido-3,4-diacetoxy-6-[3-(benzyloxycarbonylamino)propoxy]tetrahydropyran-2-yl]methyl acetate (compound 1A)

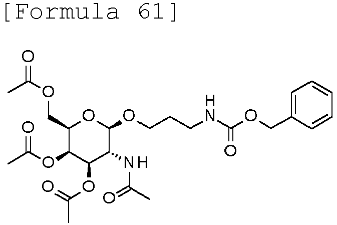

To a suspension of benzyl N-(3-hydroxypropyl) carbamate (6.4 g, 30.8 mmol) and [(2R,3R,4R,5R)-5-acetoamido-3,4,6-triacetoxy-tetrahydropyran-2-yl]methyl acetate as a compound known in the literature (International Publication No. WO 2011053614) (10 g, 25.7 mmol) in dichloromethane (200 ml), was added trifluoromethanesulfone acid (450 µl, 5.1 mmol), followed by stirring at 45 °C for 4 hours. After completion of the reaction, about half of the solvent was distilled off under reduced pressure for concentration. The organic layer was washed with a saturated aqueous sodium bicarbonate solution and saturated saline, followed by drying over anhydrous sodium sulfate, filtration, and distilling off the solvent under reduced pressure, to obtain a crude product. Isopropyl ether (100 m) was added thereto, followed by stirring for 3 hours. The solid product obtained was filtered, to obtain a target substance 1A (11.9 g). It was used for the next reaction without further purification.

¹H-NMR (CDCl₃) δ: 7.43-7.29 (5H, m), 6.26 (1H, d, J = 8.5 Hz), 5.36-5.29 (1H, m), 5.12 (1H, d, J = 12.1 Hz), 5.08 (1H, d, J = 12.1 Hz), 5.03-4.95 (2H, m), 4.33 (1H, d, J = 9.1 Hz), 4.21-4.06 (3H, m), 4.01-3.93 (1H, m), 3.80-3.73 (1H, m), 3.62-3.49 (1H, m), 3.44-3.35 (1H, m), 3.14-3.04 (1H, m), 2.15 (3H, s), 2.05 (3H, s), 2.01 (3H, s), 1.95 (3H, s), 1.88-1.75 (1H, m), 1.69-1.56 (1H, m). Calcd for C₂₅H₃₄N₂O₁₁: [M+H]+ 539, Found 539.

### (1B) Synthesis of benzyl N-[3-[(2R,3R,4R,5R,6R)-3-acetoamido-4,5-dihydroxy-6-(hydroxymethyl)tetrahydropyran-2-yl]oxypropyl] carbamate (compound 1B)

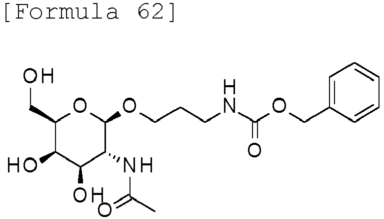

To a suspension of the compound (12.4 g, 23 mmol) synthesized in step (1A) in ethyl alcohol (200 ml), was added a 28% sodium methoxide methanol solution (0.85 ml), followed by stirring at room temperature. As the reaction progressed, the solution became a clear solution. A precipitate was obtained by standing for 60 minutes. The precipitate was filtered and washed with ethyl alcohol and diisopropyl ether, followed by drying under reduced pressure. A crude product (10 g) containing a target substance 1B was obtained as a solid. It was used for the next reaction without further purification.

¹H-NMR (DMSO-D₆) δ: 7.60 (1H, d, J = 9.1 Hz), 7.40-7.17 (5H, m), 5.01 (2H, s), 4.62-4.55 (2H, m), 4.49 (1H, d, J = 4.2 Hz), 4.20 (1H, d, J = 8.5 Hz), 3.76-3.27 (6H, m), 3.08-2.99 (2H, m), 1.81 (3H, s), 1.66-1.56 (2H, m). Calcd for C₁₉H₂₈N₂O₈: [M+Na]+ 435, Found 435.

### (1C) Synthesis of N-[(2R,3R,4R,5R,6R)-2-(3-aminopropoxy)-4,5-dihydroxy-6-(hydroxymethyl)tetrahydropyran-3-yl]acetamide hydrochloride (compound 1C)

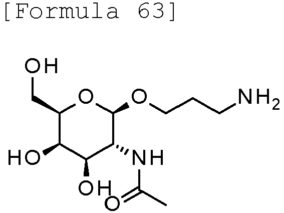

The compound (22.8 g, 55.3 mmol) synthesized in step (1B) was dissolved in tetrahydrofuran (280 ml)/1 N hydrochloric acid (61 ml). 10% palladium/carbon (wet) (5.56 g) was added thereto, followed by vigorous stirring at room temperature under a hydrogen atmosphere for 3.5 hours. After completion of the reaction, the mixture was filtered, and the passing fluid obtained was distilled off under reduced pressure. A solid product generated by adding an appropriate amount of ethyl alcohol was filtered and washed with ethyl alcohol and diethyl ether, followed by drying under reduced pressure. A crude product (17 g) containing a target substance 1C was obtained. It was used for the next reaction without further purification.

¹H-NMR (D₂O) δ: 4.27 (1H, d, J = 8.5 Hz), 3.90-3.50 (8H, m), 2.93 (2H, t, J = 7.0 Hz), 1.89 (3H, s), 1.82-1.75 (2H, m).

### (1D) Synthesis of 9H-fluorene-9-ylmethyl N-[3-[(2R,3R,4R,5R,6R)-3-acetoamido-4,5-dihydroxy-6-(hydroxymethyl)tetrahydropyran-2-yl]oxypropyl] carbamate (compound 1D)

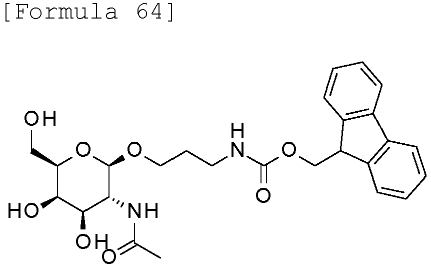

The compound (16.4 g, 52.1 mmol) synthesized in step (1C) was dissolved in tetrahydrofuran (200 ml)/pure water (50 ml), and N,N-diisopropylethylamine (12.7 mL, 72.9 mmol) was added thereto. Under ice cooling, N-[(9H-fluorene-9-ylmethoxy)carbonyloxy]succinimide (21.1 g, 62.5 mmol) was added thereto, followed by stirring at room temperature for 1.5 hours. After completion of the reaction, the solvent was distilled off under reduced pressure. Diethyl ether (100 ml) was added thereto, followed by stirring for 1 hour. The solid product was washed sequentially with ethyl alcohol, ethyl acetate, and diethyl ether, followed by drying under reduced pressure, to obtain a crude product (22.9 g) containing a target substance 1D.

¹H-NMR (DMSO-D₆) δ: 7.89 (2H, d, J = 7.3 Hz), 7.69 (2H, d, J = 7.3 Hz), 7.61 (1H, d, J = 9.1 Hz), 7.42 (2H, t, J = 7.3 Hz), 7.33 (2H, t, J = 7.3 Hz), 7.24 (1H, t, J = 5.7 Hz), 4.60-4.57 (2H, m), 4.49 (1H, d, J = 4.2 Hz), 4.31-4.18 (4H, m), 3.76-3.28 (8H, m), 3.05-2.97 (2H, m), 1.81 (3H, s), 1.62-1.57 (2H, m).

Calcd for C26H32N2O8: [M+Na]+ 523, Found 523.

### (1E) Synthesis of [(2R,3R,4R,5R,6R)-5-acetoamido-4-acetoxy-2-[[bis(4-methoxyphenyl)-phenyl-methoxy]methyl]-6-[3-(9H-fluorene-9-ylmethoxycarbonylamino)propoxy]tetrahydropyran-3-yl] acetate (compound 1E)

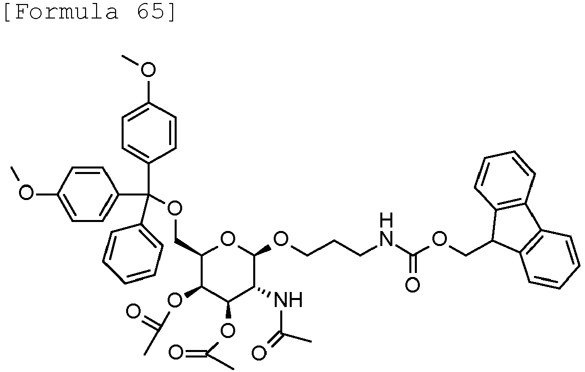

To a suspension of the compound (1.56 g, 3.1 mmol) synthesized in step (1D) in pyridine (20 ml), was added 4,4'-dimethoxytrityl chloride (1.27 g, 3.7 mmol), followed by stirring at room temperature for 30 minutes. Subsequently, acetic anhydride (1.18 ml, 12.5 mmol) was added thereto, followed by stirring at room temperature for 20 hours. After completion of the reaction, N,N-diisopropylethylamine (1.1 mL, 6.2 mmol) and ethyl alcohol (2 ml) were added thereto, and the solvent was distilled off under reduced pressure, to obtain a crude product. This was purified by silica-gel column chromatography (hexane: ethyl acetate = 75:25-0:100, v/v, containing 1% triethylamine), to obtain an amorphous target substance 1E (2.8 g, quant.).

¹H-NMR (CDCl₃) δ: 7.77 (2H, d, J = 7.3 Hz), 7.62 (2H, d, J = 7.3 Hz), 7.43-7.18 (13H, m), 6.83-6.79 (4H, m), 6.03 (1H, d, J = 9.1 Hz), 5.55 (1H, d, J = 3.0 Hz), 5.14-5.03 (2H, m), 4.46-4.43 (3H, m), 4.24-4.04 (2H, m), 3.97-3.91 (1H, m), 3.85-3.82 (1H, m), 3.77 (6H, s), 3.72-3.71 (1H, m),3.54-3.33 (3H, m), 3.09-3.04 (2H, m), 2.03 (3H, s), 1.89 (6H, s), 1.65-1.56 (2H, m).

Calcd for C₅₂H₅₅NO₁₂: [M+Na]+ 908, Found 909.

### (1F) Synthesis of [(2R,3R,4R,5R,6R)-5-acetoamido-4-acetoxy-6-(3-aminopropoxy)-2-[[bis(4-methoxyphenyl)-phenyl-methoxy]methyl]tetrahydropyran-3-yl] acetate (compound 1F)

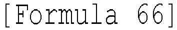

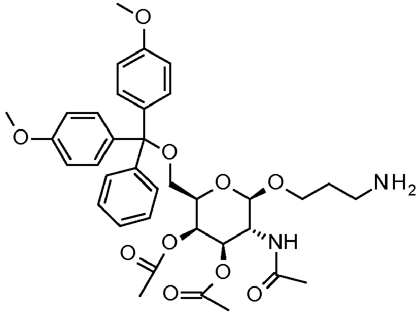

To a solution of the compound (2.49 g, 2.8 mmol) synthesized in step (1E) in dichloromethane (8 ml), was added piperidine (0.45 ml, 4.5 mmol), followed by stirring at room temperature. If the reaction was not complete, piperidine was added as appropriate. After completion of the reaction, the solvent was distilled off under reduced pressure, to obtain a crude product. This was purified by NH-silica-gel column chromatography (dichloromethane: methanol = 100:0-85:15, v/v), to obtain an amorphous target substance 1F (1.48 g, yield 79%). ¹H-NMR (CDCl₃) δ: 7.39-7.18 (9H, m), 6.83-6.80 (4H, m), 5.55 (1H, d, J = 3.0 Hz), 5.51 (1H, d, J = 9.1 Hz), 5.27 (1H, dd, J = 11.2, 3.3 Hz), 4.64 (1H, d, J = 8.5 Hz), 3.98-3.90 (2H, m), 3.84-3.82 (1H, m), 3.79 (6H, s), 3.75-3.74 (1H, m), 3.58-3.56 (1H, m), 3.35 (1H, dd, J = 9.1, 5.4 Hz), 3.07 (1H, t, J = 8.5 Hz), 2.82-2.74 (4H, m), 2.01 (3H, s), 1.96 (3H, s), 1.89 (3H, s), 1.74-1.69 (2H, m) .

Calcd for C₃₇H₄₅NO₁₀: [M+Na]+ 687, Found 687.

### (Example 2) Synthesis of amidite reagent corresponding to GalNAc unit X¹⁸ (compound having hydrophobic protecting group-containing GalNAc-phosphate group) (2A) Synthesis of benzyl 2-[[2-benzyloxy-3-[(2-benzyloxy-2-oxo-ethyl)carbamoyloxy]propoxy] carbonylamino] acetate (compound 2A)

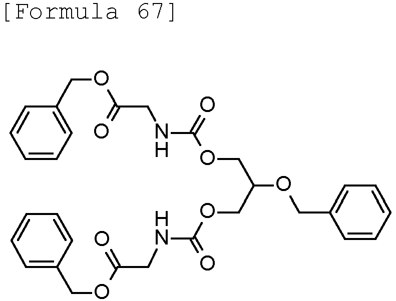

2-Benzyloxy-1,3-propanediol (2.5 g, 14 mmol) and 4-nitrophenyl chloroformate (5.8 g, 29 mmol) were dissolved in tetrahydrofuran (100 ml), and pyridine (5.0 ml, 61.8 mmol) was added thereto, followed by stirring at room temperature for 20 minutes. The precipitated solid was filtered, and then tetrahydrofuran (70 ml) was added thereto. Subsequently, glycine benzyl ester TFA salt (9.2 g, 33 mmol) and N,N-diisopropylethylamine (9.6 mL, 55 mmol) were added thereto, followed by stirring at room temperature overnight. After completion of the reaction, the solvent was distilled off under reduced pressure, to obtain a crude product. This was purified by silica-gel column chromatography (hexane: ethyl acetate = 100:0-40:60, v/v), to obtain a gum-like target substance 2A (6.5 g, yield 84%).

¹H-NMR (CDCl₃) δ: 7.37-7.33 (15H, m), 5.48 (2H, t, J = 5.4 Hz), 5.18 (4H, s), 4.64 (2H, s), 4.23 (4H, d, J = 5.4 Hz), 3.99 (4H, d, J = 5.4 Hz), 3.84 (1H, t, J = 5.4 Hz). Calcd for C₃₀H₃₂N₂O₉: [M+H]+ 565, Found 565, [M+Na]+ 587, Found 587

### (2B) Synthesis of 2-[[3-(carboxymethylcarbamoyloxy)-2-hydroxy-propoxy]carbonylamino]acetic acid (compound 2B)

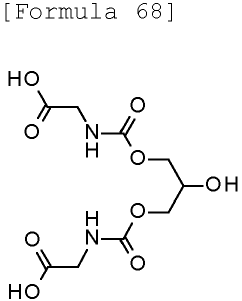

The compound 2A (7.3 g, 2.8 mmol) synthesized in step (2A) was dissolved in tetrahydrofuran (200 ml)/pure water (50 ml). 20% palladium hydroxide-activated carbon (about 50% aqueous) (3 g) was added thereto, followed by vigorous stirring at room temperature under a hydrogen atmosphere for 5 hours. After completion of the reaction, the mixture was filtered, and the passing fluid obtained was distilled off under reduced pressure, to obtain a crude product (3.2 g) containing a target substance 2B. It was used for the next reaction without further purification.

¹H-NMR (D₂O) δ: 4.21-4.14 (5H, m), 3.86 (4H, s).

### (2C) Synthesis of [(2R,3R,4R,5R,6R)-5-acetoamido-6-[3-[[2-[[3-[[2-[3-[(2R,3R,4R,5R,6R)-3-acetoamido-4,5-diacetoxy-6-[[bis(4-methoxyphenyl)-phenyl 1-methoxy]methyl]tetrahydropyran-2-yl]oxypropylamino]-2-oxo-ethyl]carbamoyloxy]-2-hydroxy-propoxy]carbonylamino]acetyl]amino]propoxy]-4-acetoxy-2-[[bis(4-methoxyphenyl)-phenyl-methoxy]methyl]tetrahydropyran-3-yl] acetate (compound 2C)

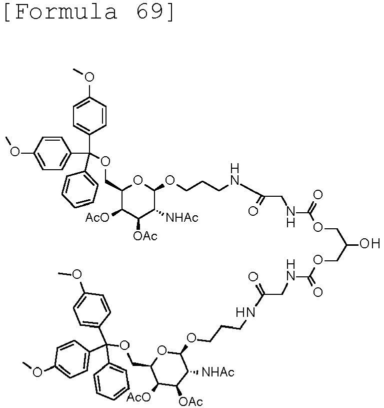

To a solution of the compound 2B (0.31 g, 1.05 mmol) synthesized in step (2B), the compound 1F (1.47 g, 2.21 mmol) synthesized in step (1E), and N,N-diisopropylethylamine (0.91 mL, 5.27 mmol) in N,N-dimethylformamide (9 ml), was added 1-[bis(dimethylamino)methylene]-1H-1,2,3-triazolo[4,5-b]pyridinium 3-oxide hexafluorophosphate (11.3 g, 29.6 mmol), followed by stirring at room temperature for 3 hours. After completion of the reaction, the reaction solution was added dropwise to diethyl ether (185 ml in total). A jelly-like crude product obtained after removing the solvent in the supernatant was purified by silica-gel column chromatography (dichloromethane: methanol = 100:0-15:85, v/v, containing 1% triethylamine), to obtain a target substance 2C (1.05 g, yield 63%) as a solid.

¹H-NMR (CDCl₃) δ: 7.39-7.17 (18H, m), 6.83-6.80 (8H, m), 6.70-6.27 (2H, m), 5.56-5.54 (2H, m), 5.10 (2H, d, J = 10.9 Hz), 4.49-3.02 (44H, m), 2.01 (6H, s), 1.96 (6H, s), 1.90 (6H, s), 1.80-1.63 (4H, m).

Calcd for C₈₁H₉₈N₆O₂₇: [M+Na]+ 1611, Found 1611

### (2D) Synthesis of [(2R,3R,4R,5R,6R)-5-acetoamido-6-[3-[[2-[[3-[[2-[3-[(2R,3R,4R,5R,6R)-3-acetoamido-4,5-diacetoxy-6-[[bis(4-methoxyphenyl)-phenyl-methoxy]methyl]tetrahydropyran-2-yl]oxypropylamino]-2-oxo-ethyl]carbamoyloxy]-2-[2-cyanoethoxy-(diisopropylamino)phosphanyl]oxy-propoxy]carbonylamino]acetyl]amino]propoxy]-4-acetoxy-2-[[bis(4-methoxyphenyl)-phenylmethoxy]methyl]tetrahydropyran-3-yl] acetate (compound 2D)

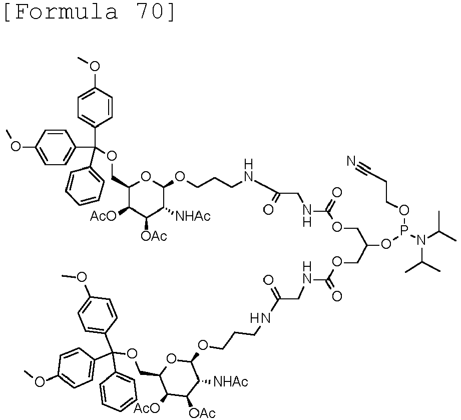

The compound 2C (0.47 g, 0.30 mmol) synthesized in step (2C) was azeotroped with an appropriate amount of ethyl acetate/toluene (1/1) and then dissolved in dichloromethane (3 ml). N,N-Diisopropylethylamine (210 µl, 1.18 mmol) and 2-cyanoethyldiisopropylchlorophosphoroamidite (79 µl, 0.36 mmol) were added thereto, followed by stirring at room temperature for 1 hour. Further, 2-cyanoethyldiisopropylchlorophosphoroamidite (20 µl, 0.09 mmol) was added thereto, followed by stirring at room temperature for 15 minutes. After completion of the reaction, the solvent was distilled off under reduced pressure, to obtain a crude product. This was purified by silica-gel column chromatography (ethyl acetate: methanol = 100:0-85:15, v/v, containing 1% triethylamine), to obtain an amorphous target substance 2D (0.34 g, yield 64%).

¹H-NMR (CDCl₃) δ: 7.39-7.17 (18H, m), 6.87-6.70 (8H, m), 6.29-6.06 (2H, m), 5.55 (2H, d, J = 3.0 Hz), 5.11 (2H, d, J = 10.9 Hz), 4.47-3.02 (47H, m), 2.63 (2H, t, J = 6.3 Hz), 2.02 (6H, s), 1.96 (6H, s), 1.90 (6H, s), 1.80-1.64 (4H, m), 1.17 (12H, d, J = 6.7 Hz).

### (Example 3) Synthesis of amidite reagent (compound having hydrophobic protecting group-containing GalNAc-phosphate group) corresponding to GalNAc unit X¹⁹ (3A) Synthesis of benzyl 2-[[3-benzyloxy-2-[(2-benzyloxy-2-oxo-ethyl) carbamoyloxy]propoxy] carbonylamino] acetate (compound 3A)

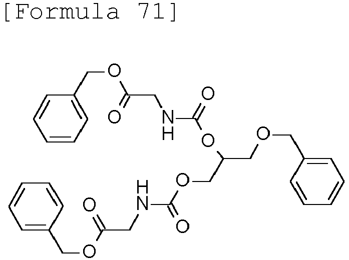

A target substance 3A (2.8 g, yield 90%) was obtained in the same manner as in step (2A) using (+/-)-3-benzyloxy-1,2-propanediol (1.0 g, 5.5 mmol) instead of 2-benzyloxy-1,3-propanediol.

¹H-NMR (CDCl₃) δ: 7.39-7.30 (15H, m), 5.37-5.35 (2H, m), 5.17-5.12 (5H, m), 4.56-4.51 (2H, m), 4.33-4.30 (2H, m), 4.03-3.94 (4H, m), 3.61 (2H, d, J = 5.4 Hz).

Calcd for C₃₀H₃₂N₂O₉: [M+Na]+ 587, Found 587

### (3B) Synthesis of 2-[[2-(carboxymethylcarbamoyloxy)-3-hydroxy-propoxy]carbonylamino]acetic acid (compound 3B)

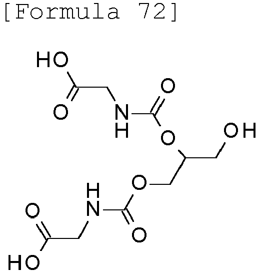

A crude product containing a target substance 3B (1.2 g) was obtained in the same manner as in step (2B) using the compound 3A (2.8 g, 5.0 mmol) instead of the compound 2A.

¹H-NMR (D₂O) δ: 4.32-4.19 (3H, m), 3.94-3.87 (4H, m), 3.81-3.71 (2H, m).

### (3C) Synthesis of [(2R,3R,4R,5R,6R)-5-acetoamido-6-[3-[[2-[[2-[[2-[3-[(2R,3R,4R,5R,6R)-3-acetoamido-4,5-diacetoxy-6-[[bis(4-methoxyphenyl)-phenyl-methoxy]methyl]tetrahydropyran-2-yl]oxypropylamino]-2-oxo-ethyl]carbamoyloxy]-3-hydroxy-propoxy]carbonylamino]acetyl]amino]propoxy]-4-acetoxy-2-[[bis(4-methoxyphenyl)-phenyl 1-methoxy]methyl]tetrahydropyran-3-yl] acetate (compound 3C)

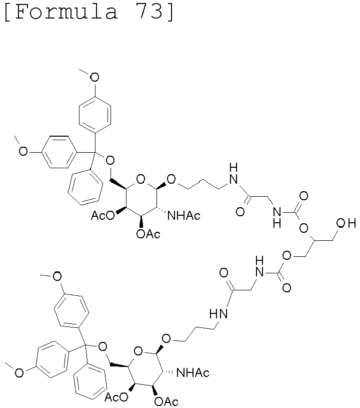

A target substance 3C (0.74 g, yield 44%) was obtained in the same manner as in step (2C) using the compound 3B (0.31 g, 1.05 mmol) instead of the compound 2B.

¹H-NMR (CDCl₃) δ: 7.40-7.18 (18H, m), 6.81 (8H, dd, J = 9.1, 3.0 Hz), 6.70-6.50 (1H, m), 6.16-6.02 (1H, m), 5.56-5.54 (2H, br), 5.11-5.00 (2H, m), 4.53-2.99 (44H, m), 2.01 (6H, s), 1.95 (6H, s), 1.88 (6H, s), 1.77-1.68 (4H, m) .

Calcd for C₈₁H₉₈N₆O₂₇: [M+Na]+ 1611, Found 1611

### (3D) Synthesis of [(2R,3R,4R,5R,6R)-5-acetoamido-6-[3-[[2-[[2-[[2-[3-[(2R,3R,4R,5R,6R)-3-acetoamido-4,5-diacetoxy-6-[[bis(4-methoxyphenyl)-phenyl-methoxy]methyl]tetrahydropyran-2-yl]oxypropylamino]-2-oxo-ethyl]carbamoyloxy]-3-[2-cyanoethoxy-(diisopropylamino)phosphanyl]oxy-propoxy]carbonylamino]acetyl]amino]propoxy]-4-acetoxy-2-[[bis(4-methoxyphenyl)-phenyl-methoxy]methyl]tetrahydropyran-3-yl] acetate (compound 3D)

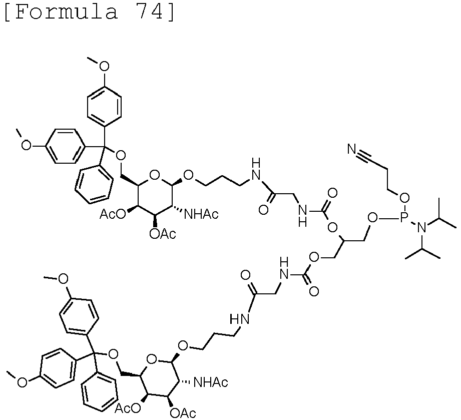

A target substance 3D (0.60 g, yield 72%) was obtained in the same manner as in step (2D) using the compound 3C (0.31 g, 1.05 mmol) instead of the compound 2C.

¹H-NMR (CDCl₃) δ: 7.38-7.18 (18H, m), 7.13-6.97 (1H, m), 6.83-6.81 (8H, m), 6.58-6.36 (1H, m), 5.56-5.52 (2H, m), 5.10-5.04 (2H, m), 4.46-2.97 (47H, m), 2.65-2.63 (2H, m), 2.02 (6H, s), 1.95 (3H, s), 1.94 (3H, s), 1.90 (3H, s), 1.89 (3H, s), 1.77-1.67 (4H, m), 1.20-1.16 (12H, m).

### (Example 4) Synthesis of amidite reagent (compound having hydrophobic protecting group-containing GalNAc-phosphate group) corresponding to GalNAc unit X²⁰

### (4A) Synthesis of 3-[[2-benzyloxy-3-[(3-benzyloxy-3-oxopropyl)carbamoyloxy]propoxy]carbonylamino]propanoic acid benzyl ester (compound 4A)

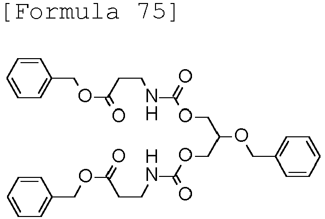

A target substance 4A (1.62 g, yield 80%) was obtained in the same manner as in step (2A) using 3-aminopropanoic acid benzyl ester TFA salt (2.4 g, 8.17 mmol) instead of the glycine benzyl ester TFA salt used in step (2A).

¹H-NMR (CDCl₃) δ: 7.39-7.27 (15H, m), 5.21 (2H, br s), 5.14 (4H, s), 4.63 (2H, s), 4.24-4.10 (4H, m), 3.79-3.74 (1H, m), 3.48-3.44 (4H, m), 2.59 (4H, t, J = 6.0 Hz). Calcd for C₃₂H₃₆N₂O₉: [M+H]+ 593, Found 593, [M+Na]+ 615, Found 615

### (4B) Synthesis of 3-[[3-(2-carboxyethylcarbamoyloxy)-2-hydroxy-propoxy]carbonylamino]propanoic acid (compound 4B)

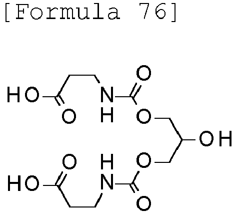

A target substance 4B (0.89 g, quant) was obtained in the same manner as in step (2B) using the compound 4A (1.62 g, 2.73 mmol) instead of the compound 2A used in step (2B).

¹H-NMR (D₂O) δ: 4.22-4.04 (5H, m), 3.46-3.32 (4H, m), 2.57 (4H, t, J = 6.7 Hz).

### (4C) Synthesis of [(2R,3R,4R,5R,6R)-5-acetoamido-6-[3-[3-[[3-[[3-[3-[(2R,3R,4R,5R,6R)-3-acetoamido-4,5-diacetoxy-6-[[bis(4-methoxyphenyl)-phenyl-methoxy]methyl]tetrahydropyran-2-yl]oxypropylamino]-3-oxo-propyl]carbamoyloxy]-2-hydroxy-propoxy]carbonylamino]propanoylamino]propoxy]-4-acetoxy-2-[[bis(4-methoxyphenyl)-phenyl-methoxy]methyl]tetrahydropyran-3-yl] acetate (compound 4C)

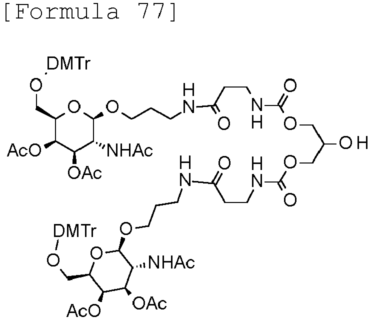

A target substance 4C (950 mg, yield 63%) was obtained in the same manner as in step (2C) using the compound 4B (300 mg, 0.93 mmol) instead of the compound 2B used in step (2C).

¹H-NMR (CDCl₃) δ: 7.32-7.24 (18H, m), 6.82-6.80 (8H, m), 6.64-6.61 (1H, br), 6.27-6.24 (1H, br), 5.91-5.88 (1H, br), 5.56 (2H, d, J = 3.0 Hz), 5.10 (2H, d, J = 11.5 Hz), 4.46-3.05 (43H, m), 2.47-2.37 (4H, m), 2.02 (6H, s), 1.98 (6H, s), 1.90 (6H, s), 1.78-1.67 (4H, m).

Calcd for C₈₃H₁₀₂N₆O₂₇: [M+Na]+ 1638, Found 1638

### (4D) Synthesis of [(2R,3R,4R,5R,6R)-5-acetoamido-6-[3-[3-[[3-[[3-[3-[(2R,3R,4R,5R,6R)-3-acetoamido-4,5-diacetoxy-6-[[bis(4-methoxyphenyl)-phenyl-methoxy]methyl]tetrahydropyran-2-yl]oxypropylamino]-3-oxo-propyl]carbamoyloxy]-2-[2-cyanoethoxy-(diisopropylamino)phosphanyl l] oxy-propoxy]carbonylamino]propanoylamino]propoxy]-4-acetoxy-2-[[bis(4-methoxyphenyl)-phenyl-methoxy]methyl]tetrahydropyran-3-yl] acetate (compound 4D)

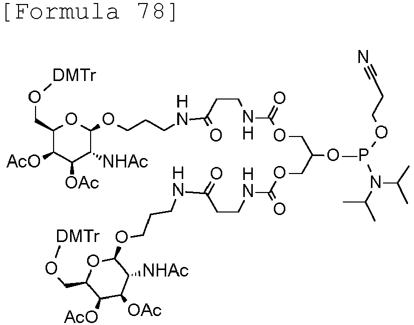

A target substance 4D (638 mg, yield 60%) was obtained in the same manner as in step (2D) using the compound 4C (945 mg, 0.58 mmol) instead of the compound 2C used in step (2D).

¹H-NMR (CDCl₃) δ: 7.39-7.18 (18H, m), 6.85-6.78 (8H, m), 6.73-6.64 (1H, m), 6.40-6.23 (1H, m), 5.83-5.72 (1H, m), 5.56 (2H, d, J = 3.0 Hz), 5.13-5.04 (2H, m), 4.45-4.35 (2H, m), 4.22-3.30 (40H, m), 3.22-3.03 (4H, m), 2.64 (2H, t, J = 6.0 Hz), 2.54-2.32 (4H, m), 2.02 (6H, s), 2.00-1.95 (6H, m), 1.90 (6H, s), 1.83-1.59 (4H, m), 1.20-1.10 (12H, m).

### (Example 5) Synthesis of amidite reagent (compound having hydrophobic protecting group-containing GalNAc-phosphate group) corresponding to GalNAc unit X²¹

### (5A) Synthesis of 4-[[2-benzyloxy-3-[(4-benzyloxy-4-oxobutyl)carbamoyloxy]propoxy]carbonylamino]butanoic acid benzyl ester (compound 5A)

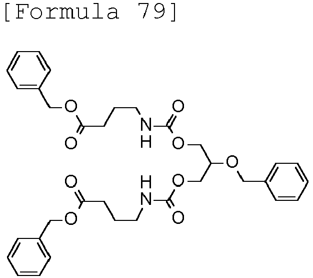

A target substance 5A (0.85 g, yield 78%) was obtained in the same manner as in step (2A) using 4-amino butanoic acid benzyl ester TFA salt (1.30 g, 4.21 mmol) instead of the glycine benzyl ester TFA salt used in step (2A) .

¹H-NMR (CDCl₃) δ: 7.38-7.27 (15H, m), 5.11 (4H, s), 4.80 (2H, br s), 4.63 (2H, s), 4.19-4.15 (4H, m), 3.77-3.75 (1H, m), 3.23-3.18 (4H, m), 2.40 (4H, t, J = 7.3 Hz), 1.88-1.81 (4H, m).

Calcd for C₃₄H₄₀N₂O₉: [M+H]+ 621, Found 622, [M+Na]+ 643, Found 644

### (5B) Synthesis of 4-[[3-(3-carboxypropylcarbamoyloxy)-2-hydroxy-propoxy]carbonylamino]butanoic acid (compound 5B)

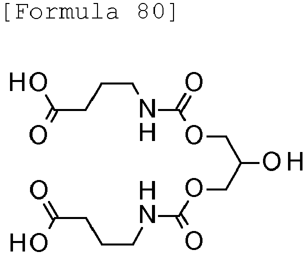

A target substance 5B (0.49 g, quant) was obtained in the same manner as in step (2B) using the compound 5A (0.85 g, 1.4 mmol) instead of the compound 2A used in step (2B).

¹H-NMR (D2O) δ: 4.19-4.05 (5H, m), 3.19-3.12 (4H, m), 2.36-2.27 (4H, m), 1.83-1.73 (4H, m).

### (5C) Synthesis of [(2R,3R,4R,5R,6R)-5-acetoamido-6-[3-[4-[[3-[[4-[3-[(2R,3R,4R,5R,6R)-3-acetoamido-4,5-diacetoxy-6-[[bis(4-methoxyphenyl)-phenyl-methoxy]methyl]tetrahydropyran-2-yl]oxypropylamino]-4-oxo-butyl]carbamoyloxy]-2-hydroxy-propoxy]carbonylamino]butanoylamino]propoxy]-4-acetoxy-2-[[bis(4-methoxyphenyl)-phenyl-methoxy]methyl]tetrahydropyran-3-yl] acetate (compound 5C)

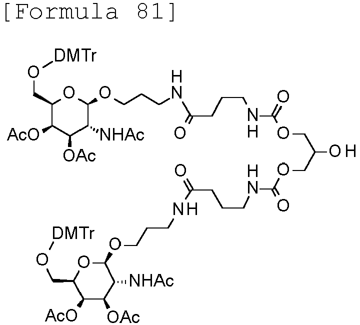

A target substance 5C (1.16 g, yield 82%) was obtained in the same manner as in step (2C) using the compound 5B (300 mg, 0.86 mmol) instead of the compound 2B used in step (2C).

¹H-NMR (CDCl3) δ: 7.31-7.24 (18H, m), 6.82-6.80 (8H, m), 6.68-6.65 (3H, m), 5.91-5.84 (1H, m), 5.56 (2H, d, J = 3.0 Hz), 5.10 (2H, d, J = 10.9 Hz), 4.45 (2H, d, J = 8.5 Hz), 4.23-3.12 (40H, m), 2.31-2.22 (4H, m), 2.01 (6H, s), 1.98 (6H, s), 1.90 (6H, s), 1.81-1.68 (8H, m). Calcd for C₈₅H₁₀₆N₆O₂₇: [M+Na] + 1666, Found 1667

### (5D) Synthesis of [(2R,3R,4R,5R,6R)-5-acetoamido-6-[3-[3-[[3-[[3-[3-[ (2R,3R,4R,5R,6R)-3-acetoamido-4,5-diacetoxy-6-[[bis(4-methoxyphenyl)-phenyl-methoxy]methyl]tetrahydropyran-2-yl]oxypropylamino]-4-oxo-butyl]carbamoyloxy]-2-[2-cyanoethoxy-(diisopropylamino)phosphanyl]oxy-propoxy]carbonylamino]butanoylamino]propoxy]-4-acetoxy-2-[[bis(4-methoxyphenyl)-phenyl-methoxy]methyl]tetrahydropyran-3-yl] acetate (compound 5D)

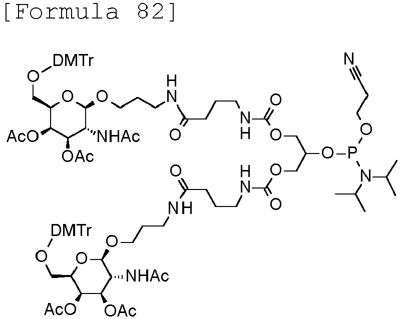

A target substance 5D (795 mg, yield 61%) was obtained in the same manner as in step (2D) using the compound 5C (1.16 g, 0.71 mmol) instead of the compound 2C used in step (2D).

¹H-NMR (CDCl₃) δ: 7.40-7.17 (18H, m), 6.85-6.77 (8H, m), 6.72-5.65 (5H, m), 5.56 (2H,d, J = 3.0 Hz), 5.11-5.03 (2H, m), 4.46-4.35 (2H, m), 4.25-3.02 (42H, m), 2.65 (2H, t, J = 6.0 Hz), 2.39-2.16 (4H, m), 2.02 (6H, s), 1.99-1.94 (6H, m), 1.90 (6H, s), 1.87-1.59 (8H, m), 1.18 (12H, d, J = 6.7 Hz).

### (Example 6) Synthesis of amidite reagent corresponding to GalNAc unit X²²

### (6A) Synthesis of acetic acid [(2R,3R,4R,5R,6R)-5-acetoamido-3,4-diacetoxy-6-[5-(benzyloxycarbonylamino)pentoxyltetrahydropyran-2-yl]methyl (compound 6A)

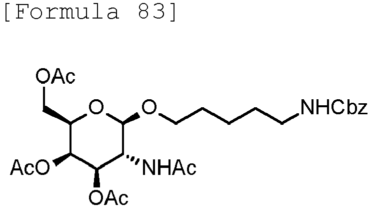

To a suspension of N-benzyloxycarbonyl-1-hydroxypentyl-5-amine as a compound known in the literature (J. Am. Chem. Soc., 2006, 128,4058-4073.) (8.05 g, 33.9 mmol) and acetic acid [(2R,3R,4R,5R)-5-acetoamido-3,4,6-triacetoxy-tetrahydropyran-2-yl]methyl as a compound known in the literature (International Publication No. WO 2011053614) (12.0 g, 30.8 mmol) in dichloromethane (200 mL), was added trifluoromethanesulfone acid (450 µL, 5.23 mmol), followed by stirring at 45°C overnight. After completion of the reaction, about half of the solvent was distilled off under reduced pressure for concentration, and the resultant was added to a mixed solution of ethyl acetate/saturated sodium bicarbonate aqueous solution. The organic layer was washed with a saturated aqueous sodium bicarbonate solution and saturated saline/phosphorus acid buffer (pH 7.0), followed by drying over anhydrous sodium sulfate, filtration, and distilling off the solvent under reduced pressure, to obtain a crude product. This was purified by silica-gel column chromatography (hexane: ethyl acetate = 100:0-0:100, v/v), to obtain an amorphous target substance 6A (17.0 g, yield 94%).

¹H-NMR (CDCl₃) δ: 7.37-7.32 (5H, m), 5.69 (1H, d, J = 9.1 Hz), 5.35 (1H, d, J = 2.4 Hz), 5.29 (1H, d, J = 11.5 Hz), 5.10 (2H, s), 4.94 (1H, br), 4.68 (1H, d, J = 7.9 Hz), 4.20-4.09 (2H, m), 3.97-3.86 (3H, m), 3.49-3.44 (1H, m), 3.20-3.17 (2H, m), 2.14 (3H, s), 2.05 (3H, s), 2.01 (3H, s), 1.93 (3H, s), 1.66-1.34 (6H, m). Calcd for C₂₇H₃₈N₂O₁₁: [M+H]⁺ 567, Found 567.

### (6B) Synthesis of [(2R,3R,4R,5R,6R)-5-acetoamido-3,4-diacetoxy-6-(5-aminopentoxy)tetrahydropyran-2-yl]methyl acetate hydrochloride (compound 6B)

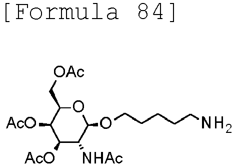

The compound 6A (3.87 g, 6.83 mmol) synthesized in step (6A) was dissolved in tetrahydropyran (32 ml)/1 N hydrochloric acid (8 ml). 10% palladium/carbon (wet) (2 g) was added thereto, followed by vigorous stirring at room temperature under a hydrogen atmosphere. After completion of the reaction, the mixture was filtered, and the passing fluid obtained was distilled off under reduced pressure, to obtain a crude product of a target substance 6B (3.1 g, yield 97%). It was used for the next reaction without further purification.

Calcd for Cₗ₉H₃₂N₂0₉: [M+H]⁺ 433, Found 433.

### (6C) Synthesis of [(2R,3R,4R,5R,6R)-5-acetoamido-6-[5-[[3-[5-[(2R,3R,4R,5R,6R)-3-acetoamido-4,5-diacetoxy-6-(acetoxymethyl)tetrahydropyran-2-yl]oxypentylcarbamoyloxy]-2-benzyloxy-propoxy]carbamoylamino]pentoxy]-3,4-diacetoxy-tetrahydropyran-2-yl]methyl acetate (compound 6C)

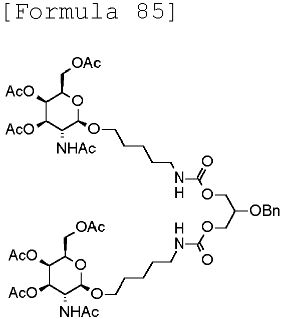

2-Benzyloxy-1,3-propanediol (600 mg, 3.29 mmol) and 4-nitrophenyl chloroformate (1.39 g, 6.91 mmol) were dissolved in tetrahydrofuran (22 ml), and pyridine (1.1 ml, 13.2 mmol) was added thereto, followed by stirring at room temperature for 15 minutes. After filtering a precipitated solid, tetrahydrofuran (16 ml) was added thereto. Subsequently, a solution of the compound (6B) (3.09 g, 6.59 mmol) synthesized in step (6B) in tetrahydrofuran (15 ml)/dichloromethane (5 ml) and N,N-diisopropylethylamine (3.4 mL, 19.8 mmol) were added thereto, followed by stirring at 40°C for 2 hours. After completion of the reaction, the solvent was distilled off under reduced pressure. Dichloromethane was added thereto, and the organic layer was washed with 0.5 N hydrochloric acid, saturated saline, 0.2 N aqueous sodium hydroxide solution, and saturated saline, followed by drying over anhydrous sodium sulfate, filtration, and distilling off the solvent under reduced pressure, to obtain a crude product. This was purified by silica-gel column chromatography (dichloromethane: methanol = 100:0-90:10, v/v), to obtain a mixture (2.1 g) mainly containing a target substance 6C.

Calcd for C₅₀H₇₄N₄O₂₃ : [M+H]⁺1099, Found 1099. [M+Na]⁺1121, Found 1121.

### (6D) Synthesis of [(2R,3R,4R,5R,6R)-5-acetoamido-6-[5-[[3-[5-[(2R,3R,4R,5R,6R)-3-acetoamido-4,5-diacetoxy-6-(acetoxymethyl)tetrahydropyran-2-yl]oxypentylcarbamoyloxy]-2-hydroxy-propoxy]carbonylamino]pentoxy]-3,4-diacetoxy-tetrahydropyran-2-yl]methyl acetate (compound 6D)

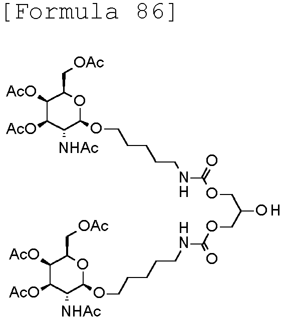

A mixture (2.1 g) containing the compound 6C obtained in step (6C) was dissolved in tetrahydropyran (20 ml)/pure water (1 ml). 1 N Hydrochloric acid (0.38 ml) and 10% palladium/carbon (wet) (1 g) were added thereto, followed by vigorous stirring at room temperature under a hydrogen atmosphere. After completion of the reaction, the mixture was filtered, and the passing fluid obtained was distilled off under reduced pressure, to obtain a crude product. This was purified by silica-gel column chromatography (ethyl acetate: methanol = 100:0-90:15, v/v), to obtain a target substance 6D (1.38 g, yield 72%) as a solid.

¹H-NMR (CDCl₃) δ: 6.10-5.96 (1H, m), 5.38-4.94 (6H, m), 4.70-4.64 (2H, m), 4.23-3.44 (19H, m), 3.20-3.15 (4H, m), 2.15 (6H, s), 2.05 (6H, s), 2.01 (6H, s), 1.97 (6H, s), 1.67-1.38 (12H, m).

Calcd for C₄₃H₆₈N₄O₂₃ : [M+H]⁺1009, Found 1009. [M+Na]⁺1031, Found 1031.

### (6E) Synthesis of [(2R,3R,4R,5R,6R)-5-acetoamido-6-[5-[[3-[5-[(2R,3R,4R,5R,6R)-3-acetoamido-4,5-diacetoxy-6-(acetoxymethyl)tetrahydropyran-2-yl]oxypentylcarbamoyloxy]-2-[2-cyanoethoxy-(diisopropylamino)phosphanyl]oxy-propoxy]carbamoylamino]pentoxy]-3,4-diacetoxy-tetrahydropyran-2-yl]methyl acetate (compound 6E)

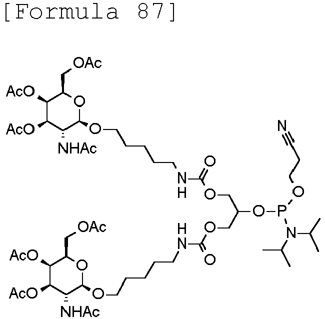

A target substance 6E (850 mg, yield 51%) was obtained in the same manner as in step (2D) using the compound 6D (1.38 g, 1.37 mmol) instead of the compound 2C used in step (2D).

¹H-NMR (CDCl₃) δ: 5.95-5.89 (1H, m), 5.38-5.26 (4H, m), 5.20-5.16 (1H, m), 5.00-4.95 (1H, m), 4.71-4.68 (2H, m), 4.36-3.44 (22H, m), 3.15 (4H, q, J = 6.4 Hz), 2.67 (2H, t, J = 6.3 Hz), 2.15 (6H, s), 2.05 (6H, s), 2.01 (6H, s), 1.96 (6H, s), 1.67-1.33 (12H, m), 1.18 (12H, d, J = 6.7 Hz) .

### (Example 7) Synthesis of amidite reagent corresponding to GalNAc unit X²⁰

### (7A) Synthesis of [(2R,3R,4R,5R)-5-acetoamido-3,4-diacetoxy-6-(3-aminopropoxy)tetrahydropyran-2-yl]methyl acetate hydrochloride (compound 7A)

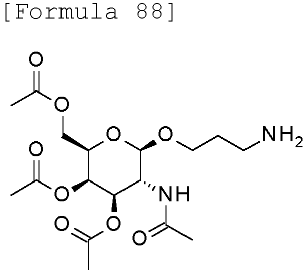

The compound 1A (10 g, 18.6 mmol) synthesized in step (1A) was dissolved in tetrahydrofuran (200 ml)/1 N hydrochloric acid (20 ml). 10% palladium/carbon (wet) (1 g) was added thereto, followed by vigorous stirring at room temperature under a hydrogen atmosphere for 1 hour. After completion of the reaction, the mixture was filtered, and the passing fluid obtained was distilled off under reduced pressure, to obtain a crude product containing a target substance 7A (8.97 g). It was used for the next reaction without further purification. Calcd for C₁₇H₂₈N₂O₉: [M+H]+ 405, Found 405.

### (7B) Synthesis of [(2R,3R,4R,5R,6R)-5-acetoamido-6-[3-[3-[[3-[[3-[3-[(2R,3R,4R,5R,6R)-3-acetoamido-4,5-diacetoxy-6-(acetoxymethyl)tetrahydropyran-2-yl]oxypropylamino]-3-oxo-propyl]carbamoyloxy]-2-hydroxy-propoxy]carbonylamino]propanoylamino]propoxy]-3,4-diacetoxy-tetrahydropyran-2-yl]methyl acetate (compound 7B)

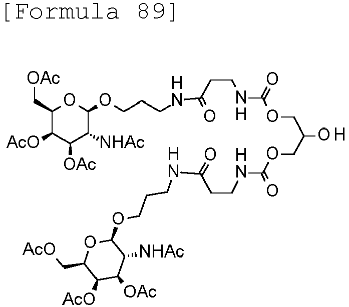

To a solution of the compound 4B (200 mg, 0.62 mmol) synthesized in step (4B) in N,N-dimethylformamide (2 ml), were added 0-(N-succinimidyl)-N,N,N',N'-tetramethyluronium tetrafluoroborate (411 mg, 1.37 mmol) and N,N-diisopropylethylamine (0.32 ml, 1.86 mmol), followed by stirring at room temperature for 20 minutes. The reaction solution was added to a solution of the compound 37B (657 mg, 1.49 mmol) synthesized in step (37B) in N,N-dimethylformamide (2 ml), and N,N-diisopropylethylamine (0.32 ml, 1.86 mmol) was further added thereto, followed by stirring at room temperature for 60 minutes. After completion of the reaction, the reaction solution was added dropwise to diethyl ether (80 ml in total). A jelly-like crude product obtained after removing the solvent in the supernatant was purified by silica-gel column chromatography (dichloromethane: methanol = 100:0-75:25, v/v), to obtain an amorphous target substance 7B (408 mg, yield 60%).

¹H-NMR (CDCl₃) δ: 6.76 (2H, br), 6.69-6.63 (2H, m), 5.96 (1H, br), 5.36 (2H, d, J = 3.0 Hz), 5.13-5.08 (2H, m), 4.53 (2H, d, J= 8.5 Hz), 4.25-3.07 (27H, m), 2.51-2.44 (4H, m), 2.17 (6H, s), 2.06 (6H, s), 2.02 (6H, s), 2.00 (6H, s), 1.86-1.73 (4H, m).

Calcd for C₄₅H₇₀N₆O₂₅: [M+H]⁺ 1095, Found 1096. [M+Na]⁺ 1117, Found 1117.

### (7C) Synthesis of [(2R,3R,4R,5R,6R)-5-acetoamido-6-[3-[3-[[3-[[3-[3-[(2R,3R,4R,5R,6R)-3-acetoamido-4,5-diacetoxy-6-(acetoxymethyl)tetrahydropyran-2-yl]oxypropylamino]-3-oxo-propyl]carbamoyloxy]-2-[2-cyanoethoxy-(diisopropylamino)phosphanyl]oxy-propoxy]carbonylamino]propanoylamino]propoxy]-3,4-diacetoxy-tetrahydropyran-2-yl]methyl acetate (compound 7C)

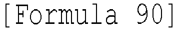

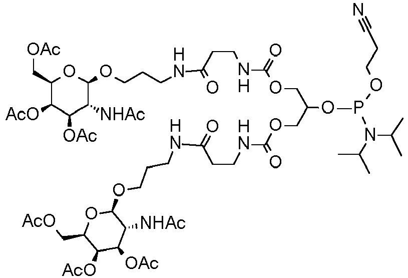

A target substance 7C (510 mg, yield 28%) was obtained in the same manner as in step (2D) using the compound 7B (1.53 g, 1.40 mmol) instead of the compound 2C used in step (2D).

¹H-NMR (CDCl₃) δ: 6.60-6.44 (4H, m), 5.76-5.68 (1H, m) , 5.36 (2H, d, J = 3.0 Hz), 5.09-5.05 (2H, m), 4.50-4.45 (2H, m), 4.26-3.37 (28H, m), 3.17 (2H, br), 2.67 (2H, t, J = 6.3 Hz), 2.52-2.42 (4H, m), 2.17 (6H, s), 2.05 (6H, s), 2.02 (6H, s), 1.99 (6H, s), 1.81-1.70 (4H, m), 1.18 (12H, d, J = 6.7 Hz).

### (Example 8)

X^18-A^m1s-A^e2s-U^m1s-C^m1s-C^e2s-G^m1s-A^m1s-T^e2s-G^m1s-G^m1s-C^e2s-G^m1s-A^m1s-

A^e2s-G^m1t-H (SEQ ID No: 40)

Using ABI 392 DNA/RNA Synthesizer (available from Applied Biosystems) as a nucleic acid automatic synthesizer and NittoPhase UnyLinker 100, 10 µmol (available from NITTO DENKO CORPORATION) as a solid phase carrier, an oligonucleotide sequence 15e_001 was synthesized according to the phosphoramidite method (Nucleic Acids Research, 12, 4539 (1984)), and then it was condensed once using the compound 2D synthesized in Example 2 as a GlcNAc unit amidite. Oligomers were excised from the support by treating protected oligonucleotide analogs with 6 mL of concentrated ammonia water, and a cyanoethyl group as a protecting group on the phosphorus atom and a protecting group on a nucleobase were removed. Using Clarity QSP (available from Phenomenex Inc.), purification was performed according to the protocol attached.

The nucleotide sequence of this compound was a sequence complementary to the sequence from the 92nd to the 106th from the 5' end of exon 5 of c.648G>T mutated G6PC gene with a mutation at nucleotide 728 in Homo sapiens glucose-6-phosphatase catalytic subunit (G6PC), transcript variant 1, mRNA (NCBI-GenBank accession No. NM_000151.3) from G to T. The compound was identified by negative ion ESI mass spectrometry (measured value: 6247.96) .

### (Example 9)

X^18-A^m1s-A^e2s-U^m1s-C^m1s-C^e2s-G^m1s-A^e2s-U^m1s-G^m1s-G^m1s-C^e2s-G^m1s-A^m1s-

A^e2s-G^m1t-H (SEQ ID No: 40)

Using the aforementioned sequence 15e_001.5 instead of the sequence used in Example 8, synthesis was performed in the same manner as in Example 8.

The nucleotide sequence of this compound was a sequence complementary to the sequence from the 92nd to the 106th from the 5' end of exon 5 of c.648G>T mutated G6PC gene with a mutation at nucleotide 728 in Homo sapiens glucose-6-phosphatase catalytic subunit (G6PC), transcript variant 1, mRNA (NCBI-GenBank accession No. NM_000151.3) from G to T. The compound was identified by negative ion ESI mass spectrometry (measured value: 6233.97) .

### (Example 10)

X^18-A^e2s-U^m1s-C^m1s-C^e2s-G^m1s-A^e2s-U^m1s-G^m1s-G^m1s-C^e2s-G^m1s-A^m1s-A^e2s-

G^m1s-C^e2t-H (SEQ ID No: 44)

Using the aforementioned sequence 15e_005.5 instead of the sequence used in Example 8, synthesis was performed in the same manner as in Example 8.

The nucleotide sequence of this compound was a sequence complementary to the sequence from the 91st to the 105th from the 5' end of exon 5 of c.648G>T mutated G6PC gene with a mutation at nucleotide 728 in Homo sapiens glucose-6-phosphatase catalytic subunit (G6PC), transcript variant 1, mRNA (NCBI-GenBank accession No. NM_000151.3) from G to T. The compound was identified by negative ion ESI mass spectrometry (measured value: 6235.97).

### (Example 11)

X^18-U^m1s-C^m1s-C^e2s-G^m1s-A^e2s-U^m1s-G^m1s-G^m1s-C^e2s-G^m1s-A^m1s-A^e2s-G^m1s-

C^e2s-U^m1t-H (SEQ ID No: 45)

Using the aforementioned sequence 15e_006.5 instead of the sequence used in Example 8, synthesis was performed in the same manner as in Example 8.

The nucleotide sequence of this compound was a sequence complementary to the sequence from the 90th to the 104th from the 5' end of exon 5 of c.648G>T mutated G6PC gene with a mutation at nucleotide 728 in Homo sapiens glucose-6-phosphatase catalytic subunit (G6PC), transcript variant 1, mRNA (NCBI-GenBank accession No. NM_000151.3) from G to T. The compound was identified by negative ion ESI mass spectrometry (measured value: 6200.94) .

### (Example 12)

X^18-A^m1s-A^m1s-A^e2s-U^m1s-C^m1s-C^e2s-G^m1s-A^e2s-U^m1s-G^m1s-G^m1s-C^e2s-G^m1s-

A^m1s-A^e2s-G^m1t-H (SEQ ID No: 42)

Using the aforementioned sequence 16e_001.5 instead of the sequence used in Example 8, synthesis was performed in the same manner as in Example 8.

The nucleotide sequence of this compound was a sequence complementary to the sequence from the 92nd to the 107th from the 5' end of exon 5 of c.648G>T mutated G6PC gene with a mutation at nucleotide 728 in Homo sapiens glucose-6-phosphatase catalytic subunit (G6PC), transcript variant 1, mRNA (NCBI-GenBank accession No. NM_000151.3) from G to T. The compound was identified by negative ion ESI mass spectrometry (measured value: 6593.02) .

### (Example 13)

X^18-A^m1s-A^e2s-U^m1s-C^m1s-C^e2s-G^m1s-A^e2s-U^m1s-G^m1s-G^m1s-C^e2s-G^m1s-A^m1s-

A^e2s-G^m12-C^e2t-H (SEQ ID No: 47)

Using the aforementioned sequence 16e_002.5 instead of the sequence used in Example 8, synthesis was performed in the same manner as in Example 8.

The nucleotide sequence of this compound was a sequence complementary to the sequence from the 91st to the 106th from the 5' end of exon 5 of c.648G>T mutated G6PC gene with a mutation at nucleotide 728 in Homo sapiens glucose-6-phosphatase catalytic subunit (G6PC), transcript variant 1, mRNA (NCBI-GenBank accession No. NM_000151.3) from G to T. The compound was identified by negative ion ESI mass spectrometry (measured value: 6595.01).

### (Example 14)

X^18-A^e2s-U^m1s-C^m1s-C^e2s-G^m1s-A^e2s-U^m1s-G^m1s-G^m1s-C^e2s-G^m1s-A^m1s-A^e2s-

G^m1s-C^e2s-U^m1t-H (SEQ ID No: 48)

Using the aforementioned sequence 16e_003.5 instead of the sequence used in Example 8, synthesis was performed in the same manner as in Example 8.

The nucleotide sequence of this compound was a sequence complementary to the sequence from the 90th to the 105th from the 5' end of exon 5 of c.648G>T mutated G6PC gene with a mutation at nucleotide 728 in Homo sapiens glucose-6-phosphatase catalytic subunit (G6PC), transcript variant 1, mRNA (NCBI-GenBank accession No. NM_000151.3) from G to T. The compound was identified by negative ion ESI mass spectrometry (measured value: 6571.98).

### (Example 15)

X^20-A^m1s-A^e2s-U^m1s-C^m1s-C^e2s-G^m1s-A^e2s-U^m1s-G^m1s-G^m1s-C^e2s-G^m1s-A^m1s-

A^e2s-G^m1t-H (SEQ ID No: 40)

X¹⁸ was replaced with X²⁰ using the compound 4D produced in Example 4 as a GalNAc unit amidite instead of the compound 2D, to perform synthesis in the same manner as in Example 8. The sequence used was the aforementioned sequence 15e_001.5.

The nucleotide sequence of this compound was a sequence complementary to the sequence from the 92nd to the 106th from the 5' end of exon 5 of c.648G>T mutated G6PC gene with a mutation at nucleotide 728 in Homo sapiens glucose-6-phosphatase catalytic subunit (G6PC), transcript variant 1, mRNA (NCBI-GenBank accession No. NM_000151.3) from G to T. The compound was identified by negative ion ESI mass spectrometry (measured value: 6261.98).

### (Example 16)

X^20-A^e2s-U^m1s-C^m1s-C^e2s-G^m1s-A^e2s-U^m1s-G^m1s-G^m1s-C^e2s-G^m1s-A^m1s-A^e2s-

G^m1s-C^e2t-H (SEQ ID No: 44)

Using the aforementioned sequence 15e_005.5, synthesis was performed in the same manner as in Example 15.

The nucleotide sequence of this compound was a sequence complementary to the sequence from the 91st to the 105th from the 5' end of exon 5 of c.648G>T mutated G6PC gene with a mutation at nucleotide 728 in Homo sapiens glucose-6-phosphatase catalytic subunit (G6PC), transcript variant 1, mRNA (NCBI-GenBank accession No. NM_000151.3) from G to T. The compound was identified by negative ion ESI mass spectrometry (measured value: 6264.00).

### (Example 17)

X^20-A^m1s-A^m1s-A^e2s-U^m1s-C^m1s-C^e2s-G^m1s-A^e2s-U^m1s-G^m1s-G^m1s-C^e2s-G^m1s-

A^m1s-A^e2s-G^m1t-H (SEQ ID No: 42)

Using the aforementioned sequence 16e_001.5, synthesis was performed in the same manner as in Example 15.

The nucleotide sequence of this compound was a sequence complementary to the sequence from the 92nd to the 107th from the 5' end of exon 5 of c.648G>T mutated G6PC gene with a mutation at nucleotide 728 in Homo sapiens glucose-6-phosphatase catalytic subunit (G6PC), transcript variant 1, mRNA (NCBI-GenBank accession No. NM_000151.3) from G to T. The compound was identified by negative ion ESI mass spectrometry (measured value: 6621.04).

### (Example 18)

X^20-A^m1s-A^e2s-U^m1s-C^m1s-C^e2s-G^m1s-A^e2s-U^m1s-G^m1s-G^m1s-C^e2s-G^m1s-A^m1s-

A^e2s-G^m1s-C^e2t-H (SEQ ID No: 47)

Using the aforementioned sequence 16e_002.5, synthesis was performed in the same manner as in Example 15.

The nucleotide sequence of this compound was a sequence complementary to the sequence from the 91st to the 106th from the 5' end of exon 5 of c.648G>T mutated G6PC gene with a mutation at nucleotide 728 in Homo sapiens glucose-6-phosphatase catalytic subunit (G6PC), transcript variant 1, mRNA (NCBI-GenBank accession No. NM_000151.3) from G to T. The compound was identified by negative ion ESI mass spectrometry (measured value: 6623.05).

### (Example 19)

X^21-A^m1s-A^e2s-U^m1s-C^m1s-C^e2s-G^m1s-A^e2s-U^m1s-G^m1s-G^m1s-C^e2s-G^m1s-A^m1s-

A^e2s-G^m1t-H (SEQ ID No: 40)

X¹⁸ was replaced with X²¹ using the compound 5D synthesized in Example 5 as a GalNAc unit amidite, to perform synthesis in the same manner as in Example 8. The sequence used was the aforementioned sequence 15e_001.5.

The nucleotide sequence of this compound was a sequence complementary to the sequence from the 92nd to the 106th from the 5' end of exon 5 of c.648G>T mutated G6PC gene with a mutation at nucleotide 728 in Homo sapiens glucose-6-phosphatase catalytic subunit (G6PC), transcript variant 1, mRNA (NCBI-GenBank accession No. NM_000151.3) from G to T. The compound was identified by negative ion ESI mass spectrometry (measured value: 6290.02).

### (Example 20)

X^22-A^m1s-A^e2s-U^m1s-C^m1s-C^e2s-G^m1s-A^e2s-U^m1s-G^m1s-G^m1s-C^e2s-G^m1s-A^m1s-

A^e2s-G^m1t-H (SEQ ID No: 40)

X¹⁸ was replaced with X²² using the compound 6D synthesized in Example 6 as a GalNAc unit amidite, to perform synthesis in the same manner as in Example 8. The sequence used was the aforementioned sequence 15e_001.5.

The nucleotide sequence of this compound was a sequence complementary to the sequence from the 92nd to the 106th from the 5' end of exon 5 of c.648G>T mutated G6PC gene with a mutation at nucleotide 728 in Homo sapiens glucose-6-phosphatase catalytic subunit (G6PC), transcript variant 1, mRNA (NCBI-GenBank accession No. NM_000151.3) from G to T. The compound was identified by negative ion ESI mass spectrometry (measured value: 6175.96).

### (Example 21)

X^22-A^e2s-U^m1s-C^m1s-C^e2s-G^m1s-A^e2s-U^m1s-G^m1s-G^m1s-C^e2s-G^m1s-A^m1s-A^e2s-

G^m1s-C^e2t-H (SEQ ID No: 44)

Using the aforementioned sequence 15e_005.5, synthesis was performed in the same manner as in Example 20.

The nucleotide sequence of this compound was a sequence complementary to the sequence from the 91st to the 105th from the 5' end of exon 5 of c.648G>T mutated G6PC gene with a mutation at nucleotide 728 in Homo sapiens glucose-6-phosphatase catalytic subunit (G6PC), transcript variant 1, mRNA (NCBI-GenBank accession No. NM_000151.3) from G to T. The compound was identified by negative ion ESI mass spectrometry (measured value: 6177.98).

### (Example 22)

X^22-A^m1s-A^m1s-A^e2s-U^m1s-C^m1s-C^e2s-G^m1s-A^e2s-U^m1s-G^m1s-G^m1s-C^e2s-G^m1s-

A^m1s-A^e2s-G^m1t-H (SEQ ID No: 42)

Using the aforementioned sequence 16e_001.5, synthesis was performed in the same manner as in Example 20.

The nucleotide sequence of this compound was a sequence complementary to the sequence from the 92nd to the 107th from the 5' end of exon 5 of c.648G>T mutated G6PC gene with a mutation at nucleotide 728 in Homo sapiens glucose-6-phosphatase catalytic subunit (G6PC), transcript variant 1, mRNA (NCBI-GenBank accession No. NM_000151.3) from G to T. The compound was identified by negative ion ESI mass spectrometry (measured value: 6535.03).

### (Example 23)

X^22-A^m1s-A^e2s-U^m1s-C^m1s-C^e2s-G^m1s-A^e2s-U^m1s-G^m1s-G^m1s-C^e2s-G^m1s-A^m1s-

A^e2s-G^m1s-C^e2t-H (SEQ ID No: 47)

Replacing the sequence with the aforementioned sequence 16e_002.5, synthesis was performed in the same manner as in Example 20.

The nucleotide sequence of this compound was a sequence complementary to the sequence from the 91st to the 106th from the 5' end of exon 5 of c.648G>T mutated G6PC gene with a mutation at nucleotide 728 in Homo sapiens glucose-6-phosphatase catalytic subunit (G6PC), transcript variant 1, mRNA (NCBI-GenBank accession No. NM_000151.3) from G to T. The compound was identified by negative ion ESI mass spectrometry (measured value: 6537.05).

### (Example 24)

X²⁰-G^{e2s}-C^{e2s}-A^{s}-T^{s}-T^{s}-G^{s}-G^{s}-T^{s}-A^{s}-T^{s}-T^{e2s}-C^{e2s}-A^{e2t}-H (SEQ ID No: 96)

Using the aforementioned sequence complementary to the nucleic acid sequence 10177-10189 of human apolipoprotein B (APOB) GenBank accession No. NM_000384.2 instead of the sequence used in Example 15, a conjugate of a GalNAc unit with an oligonucleotide (ApoB-ASO conjugate) was synthesized in the same manner as in Example 15. In the aforementioned method, the synthesis was terminated upon the oligonucleotide having the aforementioned sequence was synthesized, followed by excision/collection, to synthesize an oligonucleotide (ApoB-ASO) with no GalNAc units bound. The compound was identified by negative ion ESI mass spectrometry (measured values: 5296.83 (ApoB-ASO conjugate) and 4392.50 (ApoB-ASO)).

### (Experimental Example 1) Evaluation of repair of abnormal splicing by Example compound using cultured cells (1-1) Culture of 293A cells (human fetal kidney cells)

293A cells (R705-07 invitrogen) were cultured as follows.

293A cells were maintained and cultured in a maintenance medium (DMEM, 10% FBS). In the experiment, cells were seeded on a 6-well plate at a density of 2 × 10⁵ cells/well and on a 12-well plate at a density of 1 × 10⁵ cells/well, and the compound of each example and a plasmid vector were simultaneously introduced on the next day, as will be described later, to be used for evaluation.

### (1-2) Production of human G6PC full-length plasmid vector

A human G6PC full-length plasmid vector (pcDNA hG6PC, pcDNA hG6PC (c.648G>T) + Int4) was produced as follows.

Using Human Multiple Tissue cDNA Panel as a template, G6PC cDNA was amplified using the following G6PC cDNA Amplified primers and then further amplified using the G6PC cDNA IF primers. The fragments amplified were inserted into the BamHI site of pcDNA3.1 using the InFusion System (pcDNA hG6PC).

G6PC cDNA Amplified primers:
   Forward primer 5'-ATAGCAGAGCAATCACCACCAAGCC-3' (SEQ ID No: 49)
   Reverse primer 5'-ATTCCACGACGGCAGAATGGATGGC-3' (SEQ ID No: 50)
G6PC IF primers:
   Forward primer 5'-TACCGAGCTCGGATCCACCACCAAGCCTGGAATAACTGC-3' (SEQ ID No: 51)
   Reverse primer 5'-CTGGACTAGTGGATCCTGGCATGGTTGTTGACTTTAAAC-3' (SEQ ID No: 52)

Using Human genome DNA as a template and the following G6PC Int4 Amplified primers, G6PC Intron4 and a region partially containing Exon5 were amplified, and G6PC Intron4 was further amplified with the G6PC Int4 IF primers. Further, the pcDNA hG6PC created in STEP1-1 was amplified using the following hG6PC vector IF primers. The c.648G>T mutation in Exon5 was introduced into InFusion primers. The two fragments were ligated by the InFusion System, and the G6PC Intron4 sequence was inserted into pcDNA hG6PC (pcDNA hG6PC (c.648G>T) + Int4).

G6PC Int4 Amplified primers:
   Forward primer 5'-TCTGGGCTGTGCAGCTGAATGTCTG-3' (SEQ ID No: 53)
   Reverse primer 5'-GTAGGGGATGACACTGACGGATGCC-3' (SEQ ID No: 54)
G6PC Int4 IF primers:
   Forward primer 5'-CTGGAGTCCTGTCAGGTATGGGC-3' (SEQ ID No: 55)
   Reverse primer 5'-AGCTGAAAAGGAAGAAGGTAATGAG-3' (SEQ ID No: 56)
hG6PC vector IF primers:
   Forward primer 5'-TCTTCCTTTTCAGCTTCGCCATCGG-3' (SEQ ID No: 57)
   Reverse primer 5'-CTGACAGGACTCCAGCAACAAC-3' (SEQ ID No: 58)

### (1-3) Cotransfection of Example compound and plasmid vector

The compound of each example and a plasmid vector were cotransfected as follows.

Solution A and solution B below were produced and then mixed.

In the case of using a 6-well plate, 250 µL of Opti-MEM Medium (Gibco), 0.50 µL of plasmid vector (1 mg/mL), and 4.0 µL (final: 20 nM) of the compound produced in each example (12.5 µM) as solution A and 250 µL of Opti-MEM Medium (Gibco) and 6.0 µL of Lipofectamine2000 (Invitrogen) as solution B were prepared per well, and solution A and solution B were mixed. In the case of using a 12-well plate, 125 µL of Opti-MEM Medium (Gibco), 0.25 µL of plasmid vector (1 mg/mL), and 2.0 µL (final: 20 nM) of the compound produced in each example (12.5 µM) as solution A and 125 µL of Opti-MEM Medium (Gibco) and 3.0 µL of Lipofectamine 2000 (Invitrogen) as solution B were prepared per well, and solution A and solution B were mixed.

The mixed solution was incubated at room temperature for 20 minutes and then added to the cells the day after passage (cotransfection). The medium was replaced with a fresh maintenance medium 6 hours after the addition, and the mixture was incubated in a CO₂ incubator for 24 hours from the addition of the mixed solution.

### (1-4) Evaluation of mRNA by RT-PCR

### [1] RNA extraction (in vitro)

RNA was extracted as follows.

The cells incubated for 24 hours after the cotransfection were washed once with cold PBS. A cell lysate of the RNeasy mini kit or RNeasy 96 kit (QIAGEN K.K.) was added thereto in an amount of 300 µL per well and collected after incubation at room temperature for 5 minutes. The solution collected was subjected to RNA purification according to the protocol of the kit including DNase treatment. RNase-Free DNase set (QIAGEN K.K. 89254) was used for the DNase treatment. The purified/eluted RNA was subjected to the later-described reverse transcription reaction.

### [2] Reverse transcription reaction

The reverse transcription reaction was performed as follows.

After adjusting the extracted RNA to 25 to 100 mg/mL, 10 µL of Buffer mix, 1 µL of Enzyme mix, 9 µL of purified water, and the extracted RNA were mixed per sample using a High Capacity RNA-to-cDNA kit (Applied biosystems) for the reverse transcription reaction (37°C 60 min, 95°C 5 min, 4°C Hold) .

20 µL of the reverse transcription reaction product was diluted 5 times with 80 µL of purified water and stored at -30°C.

### [3] qRT-PCR (SYBR Green)

The qRT PCR Primers (SYBR Green) were designed as follows.
Repaired hG6PC primers (SYBR):
   Forward primer 5'-TTGTGGTTGGGATTCTGGGC-3' (SEQ ID No: 59)
   Reverse primer 5'-ATGCTGTGGATGTGGCTGAA-3' (SEQ ID No: 60)
hActin primers (SYBR):
   Forward primer 5'-TGGCACCCAGCACAATGAA-3' (SEQ ID No: 61)
   Reverse primer 5'-CTAAGTCATAGTCCGCCTAGAAGCA-3' (SEQ ID No: 62)

5 µL of 2x FAST SYBR Green Master Mix (Applied Biosystems), 2 µL of purified water, 1 µL of Primer mix (10 µM), and 2 µL of cDNA (diluted 5 times) were suspended per well as a PCR reaction solution to perform a PCR reaction using viia7 (Applied Biosystems) (program: SYBR Green Regents, FAST, including Melt curve).

### [4] qRT-PCR (Taqman assay)

A Repaired hG6PC primer set and a Total hG6PC primer set were designed as follows, and 20x primer probe mix (primer concentration: 1000 nM probe concentration: 250 nM) was adjusted. For the hActin primer set and the mActin primer set, the stock solution was used as it was.
Repaired hG6PC primer set (Taqman):
   Forward primer 5'-GCTGCTCATTTTCCTCATCAAGTT-3' (SEQ ID No: 63)
   Reverse primer 5'-TGGATGTGGCTGAAAGTTTCTGTA-3' (SEQ ID No: 64)
   Probe 5'-TCCTGTCAGGCATTGC-3' FAM (SEQ ID No: 65)
hActin primer set (Taqman): ABI Hs01060665_g1 FAM
mActin primer set (Taqman): ABI Mm02619580_g1 FAM
18s primer set (Taqman): ABI Hs99999901_s1 FAM

5 µL of 2x Taqman Fast Advanced Master Mix (Applied Biosystems), 2.5 µL of purified water, 0.5 µL of 20x Primer probe mix (10 µM), and 2 µL of cDNA (diluted 5 times) were suspended per well to adjust the PCR reaction solution (per tube) and to perform a PCR reaction (program: Taqman regents, FA) using viia7 or Quantstadio7 (Applied Biosystems).

### (1-5) Evaluation of repair of abnormal splicing of G6PC mRNA by Example oligonucleotide using human G6PC full-length plasmid vector (pcDNA hG6PC (c.648G>T) + Int4)

A human G6PC full-length plasmid vector (pcDNA hG6PC (c.648G>T) + Int4) and each oligonucleotide (15e_001, 15e_002, and the compound of Example 93 of International Publication No. WO 2004/048570 as a control) were cotransfected and evaluated by qRT-PCR (SYBR Green) for whether abnormal splicing due to G6PC (c.648G>T) was repaired. As a result, abnormal splicing of G6PC mRNA was normalized in the compounds of 15e_001 and 15e_002.

### (Experimental Example 2) Evaluation of repair of abnormal splicing by Example compound using model mice

### (2-1) Creation of mice

### Creation of vector

According to the following procedure, a G6PC KI vector was produced.

Using Mouse genomic DNA as a template and the following mG6PC 5' arm Amplified primers, a G6PC 5' arm region was amplified. It was further amplified with the mG6PC 5' arm IF primers and then inserted into the XhoI site of pBluescriptII(+/-) (G6PC 5' arm vector).
mG6PC 5' arm Amplified primers:
   Forward primer 5'-GGGAAACATGCATGAAGCCCTGGGC-3' (SEQ ID No: 67)
   Reverse primer 5'-TCCCTTGGTACCTCAGGAAGCTGCC-3' (SEQ ID No: 68)
mG6PC 5' arm IF primers:
   Forward primer 5'-CGGGCCCCCCCTCGAAAACTAGGCCTGAAGAGATGGC-3' (SEQ ID No: 69)
   Reverse primer 5'-TACCGTCGACCTCGAGGGTTGGCCTTGATCCCTCTGCTA-3' (SEQ ID No: 70)

Then, using Mouse genome DNA as a template and the following mG6PC 3' arm Amplified primers, a G6PC 3' arm region was amplified. It was further amplified with the mG6PC 3' arm IF primers and inserted into the NotI site of the G6PC 5' arm vector (G6PC 5'+3' arm vector).
mG6PC 3' arm Amplified primers:
   Forward primer 5'-GGTTGAGTTGATCTTCTACATCTTG-3' (SEQ ID No: 71)
   Reverse primer 5'-GCAAGAGAGCCTTCAGGTAGATCCC-3' (SEQ ID No: 72)
mG6PC 3' arm IF primers:
   Forward primer 5'-AGTTCTAGAGCGGCCGCCCATGCAAAGGACTAGGAACAAC-3' (SEQ ID No: 73)
   Reverse primer 5'-ACCGCGGTGGCGGCCAATGTTGCCTGTCTTCCTCAATC-3' (SEQ ID No: 74)

Using the aforementioned pcDNA hG6PC (c.648G>T) + Int4 as a template and the following hG6PC + Int4 IF primers, hG6PC (c.648G>T)+Intron4 was amplified. It was further amplified using the G6PC 5'+3' arm vector as a template and the following Arm vector IF Primers. The two fragments were ligated using InFusion System to create a G6PC KI vetor.
hG6PC + Int4 IF primers:
   Forward primer 5'-GGCCAACCCTGGAATAACTGCAAGGGCTCTG-3' (SEQ ID No: 75)
   Reverse primer 5'-TTGCATGGTTGTTGACTTTAAACACCGAAGA-3' (SEQ ID No: 76)
Arm vector IF Primers:
   Forward primer 5'-TCAACAACCATGCAAAGGACTAGGAACAAC-3' (SEQ ID No: 77)
   Reverse primer 5'-ATTCCAGGGTTGGCCTTGATCCCTCTGCTA-3' (SEQ ID No: 78)

The following KI 5' gRNA and KI 3' gRNA sequences were introduced into the gRNA sequence introduced region of pSPgRNA (addgene) to produce pSPgRNA (KI 5') and pSPgRNA (KI 3').
KI 5' gRNA: 5'-GGGATCAAGGCCAACCGGCTGG-3' (SEQ ID No: 79)
KI 3' gRNA: 5'-TAAAGTCAACCGCCATGCAAAGG-3' (SEQ ID No: 80)

### (2-2) Microinjection

Various vectors adjusted using sterile distilled water to a final concentration of G6PC KI vector 10 ng/µL, pSPgRNA (KI 5') 5 ng/µL, pSPgRNA (KI 3') 5 ng/µL, and pSPCas9 (addgene) 5 ng/µL and passed through a MILLEX-GV syringe filter (Millipore) were each injected to the extent that the pronucleus of a C57BL/6J mouse fertilized egg sufficiently swelled (about 2 pL). The fertilized egg was transplanted into an oviduct of a recipient C57BL/6J mouse, to obtain a F0 mouse.

### (2-3) Genotyping, F1 systemization

Genotyping was performed by the following procedure for F1 systemization.

Genomic DNA was extracted from the tail tissue of the F0 mouse using an automatic nucleic acid extractor (PI-200, available from KURABO INDUSTRIES LTD.) and a special kit. The extracted DNA was amplified (95°C 10 min, 35 cycles (95°C 30 sec, 60°C 30 sec, and 72°C 30 sec), 72 °C 2 min, 4°C Hold) using Amplitaq Gold Master mix (Thermo Fisher Scientific) and the following KI screening primers.
KI screening primers:
Forward primer 5'-TACGTCCTCTTCCCCATCTG-3' (SEQ ID No: 81),
Reverse primer 5'-CTGACAGGACTCCAGCAACA-3' (SEQ ID No: 82)

The aforementioned PCR product was subjected to gel electrophoresis and amplified using the genomic DNA of an individual with a band recognized around 433 bp as a template, PrimeSTAR GXL (Takara Bio Inc.), and the following KI genotyping primers (98°C 2 min, 38 cycles (95°C 15 sec, 68°C 5 min), 68 °C 7 min, 15°C Hold).
KI genotyping primers (5'):
   Forward primer 5'-TTCCTTCCAAAGCAGGGACTCTCTATGT-3' (SEQ ID No: 83 the same (1))
   Reverse primer 5'-CTTGCAGAAGGACAAGACGTAGAAGACC-3' (SEQ ID No: 84 the same (2))
KI genotyping primers (3'):
   Forward primer 5'-GAGTCTATATTGAGGGCAGGCTGGAGTC-3' (SEQ ID No: 85),
   Reverse primer 5'-TAGTCTGCCTGCTCACTCAACCTCTCCT-3' (SEQ ID No: 86)

The aforementioned PCR product was subjected to gel electrophoresis. The PCR product using KI genotyping primers (5') of the genomic DNA of individuals with a sequence length amplified using any of KI genotyping primers (5') and KI genotyping primers (3') as expected (4705 bp and 4026 bp) were subjected to direct sequencing using a Gentetic analyzer (Lifetechnology) and the following KI sequence primers. Those with KI having an expected sequence were used as KI positive F0.
KI sequence primer (5'): 5'-GAGTCTATATTGAGGGCAGGCTGGAGTC-3' (SEQ ID No: 87)

The KI positive F0 and C57BL/6J were mated to obtain F1. The genomic DNA was extracted from the auricular tissue of F1 using a DNeasy 96 Blood & Tissue Kit (QIAGEN K.K.) and amplified using PrimeSTAR GXL (Takara Bio Inc.) and the aforementioned KI genotyping primers (5') (98°C 2 min, 38 cycles (95°C 15 sec, 68°C 5 min), 68 °C 7 min, 15°C Hold) .

The aforementioned PCR product was subjected to gel electrophoresis, and the individuals having a sequence length amplified as expected (4705b) were used as KI positive F1. One line selected from the KI positive F1 was proliferated to give hG6PC (c.648G>T) + Int4 KI line.

### (2-4) Genotyping of hG6PC (c.648G>T) + Int4 line

The genomic DNA was extracted from the auricular tissue using a DNeasy 96 Blood & Tissue Kit (QIAGEN K.K.) and multiplex-amplified using KOD FX (TOYOBO CO., LTD.) and the following KI genotyping primers and mG6PC WT primers (98°C 2 min, 32 cycles (95°C 15 sec, 68°C 5.5 min), 68 °C 5 min, 4°C Hold).
KI genotyping primers (5'):
   Forward primer 5'-TTCCTTCCAAAGCAGGGACTCTCTATGT-3' (SEQ ID No: 83 the same (1))
   Reverse primer 5'-CTTGCAGAAGGACAAGACGTAGAAGACC-3' (SEQ ID No: 84 the same (2))
mG6PC WT primers:
   Forward primer 5'-TAAATTTGACCAATGAGCACTGGAGGTC-3' (SEQ ID No: 88)
   Reverse primer 5'-AAAATCATGTGTATGCGTGCCTTTCCTA-3' (SEQ ID No: 89),

The aforementioned PCR product was subjected to gel electrophoresis, and the genotyping (WT, Ht, or Homo) of offsprings was determined, while the amplification in the vicinity of 4705b was designated as the KI allele, and the amplification in the vicinity of 2536 bp was designated as the mG6PC WT allele.

### (2-5) Sampling of mouse

Sampling of a mouse was performed as follows.

In the evening of the day before sampling, fasting was started after a floor net was installed to avoid coprophagia. On the next day, the abdomen was opened under the introduction of anesthesia, and the liver and kidneys were extracted after sufficient blood removal. Each organ was washed with ice cold PBS, then trimmed to an appropriate size, and stored in a tube containing homogenized beads (NIKKATO CORPORATION) in advance. Each organ was instantly cooled with liquid nitrogen and then stored at -80°C.

### (2-6) Evaluation of mRNA by qRTPCR

### [1] RNA extraction (in vivo)

RNA was extracted as follows.

A cell lysate of the RNeasy mini kit or Qiacube system (QIAGEN K.K.) was added in an amount of 600 µL per tissue storage tube for homogenization at 25 kHz for 2 min in tissue lyserII (QIAGEN K.K.). After incubation for 10 minutes under ice cooling, the supernatant was collected by centrifugation at room temperature at 8000 G for 10 minutes. The supernatant was subjected to RNA purification according to the protocol of each kit including DNase treatment. RNase-Free DNase set (QIAGEN K.K.) was used for the DNase treatment. The purified/eluted RNA was subjected to the aforementioned reverse transcription reaction, and repair G6PC mRNA was quantified according to the aforementioned qRT-PCR (Taqman assay).

### (2-7) Evaluation of repair of abnormal splicing by Example compound using hG6PC (c.648G>T) + Int4 Ht KI mouse

The compounds of Examples 8 to 14 were each dissolved in Otsuka saline injection and subcutaneously administered to hG6PC (c.648G>T) + Int4 Ht KI mouse at 30 mg/kg. 7 days after the administration, the liver tissue of the mouse was collected under overnight fasting conditions, and whether abnormal splicing due to G6PC (c.648G>T) was repaired was evaluated by qRT-PCR (Taqman). As a result, abnormal splicing of mRNA in the liver of the hG6PC (c.648G>T) + Int4 Ht KI mouse was normalized by the compounds of Examples 8 to 14, as shown in Figure 1. Further, the compounds of Examples 15 to 23 were evaluated in the same manner. As a result, abnormal splicing of mRNA in the liver of hG6PC (c.648G>T) + Int4 Ht KI mouse was normalized by the compounds of Examples 15 to 23, as shown in Figure 2.

### (Experimental Example 3) Evaluation of oligonucleotide delivery to liver cells by GalNAc unit conjugate using ApoB Knock down test

### (3-1) Culture of human primary hepatocytes

Human primary hepatocytes were cultured as follows.

FBS (Gibco) was added to a Modified Lanford Medium (Nissui Pharmaceutical Co., Ltd.) to 10%, to prepare an adhesion medium. Frozen human primary hepatocytes (Gibco, Hu8105) were taken out from a liquid nitrogen tank, thawed at 37°C, then immediately poured into CHRM (Invitrogen), and gently mixed, followed by centrifugation (100 g, 10 minutes) at room temperature. The supernatant was removed with an aspirator, and then an adhesion medium heated to 37°C was added, to prepare a cell suspension. The number of living cells was counted by an automatic counter (Bio-Rad Laboratories, Inc.), and then the cells were seeded on a collagen-coated 24-well plate (the number of living cells seeded: 1.4 to 2.1 × 10⁵ cells/well). After culture in a CO₂ incubator for 4 to 5 hours, the cells were confirmed to be adhered with a microscope, and the compound addition experiment described below was performed.

### (3-2) Addition of Example compound

The compound of each example was added to the human primary hepatocytes as follows.

To a Modified Lanford Medium containing 10% FBS, was added the compound of Reference Example 45 with no GalNAc derivatives bound or the compound of Example 208 with a GalNAc derivative bound, to prepare a compound-containing medium with a target concentration (which was 100 nM in ApoB mRNA expression evaluation (1) and 10 nM and 100 nM in ApoB mRNA expression evaluation (2), as described below). After the medium of human primary hepatocytes in each well cultured in the 24-well plate had been removed by aspiration, the compound-containing medium was added in an amount of 0.5 mL for each, to start incubation. 72 hours later, the later-described RNA extraction experiment was performed.

### (3-3) Evaluation of mRNA by qRT-PCR

### [1] RNA extraction (in vitro)

RNA was extracted as follows.

Following incubation, the compound-containing medium in each well of human primary hepatocytes was removed by aspiration and the cells were washed with ice-cooled PBS (0.5 mL). A 1/100 amount of 2-Mercaptoethanol was added to the RLT buffer of the RNeasy Mini QIAcube Kit (QIAGEN K.K.) to prepare a cell lysate. The cell lysate was added in an amount of 400 µL per well and collected after incubation at room temperature for 5 minutes. RNA was purified from 350 µL of the collected solution using QIAcube according to the protocol of RNeasy Mini-Animal tissues and cells-DNase digest. RNase-Free DNase set (QIAGEN K.K.) was used for the DNase treatment. The purified/eluted RNA was subjected to the later-described reverse transcription reaction.

### [2] Reverse transcription reaction

The reverse transcription reaction was performed as follows. After adjusting the extracted RNA to 25 to 100 mg/mL, 10 µL of Buffer mix, 1 µL of Enzyme mix, 9 µL of purified water, and the extracted RNA were mixed per sample using a High Capacity RNA-to-cDNA kit (Applied biosystems) for the reverse transcription reaction (37°C 60 min, 95°C 5 min, 4°C Hold). 20 µL of the reverse transcription reaction product was diluted 5 times with 80 µL of purified water and stored at -30°C or evaluated by qRT-PCR as follows.

### [3] qRT-PCR (Taqman assay)

The following ApoB primer probe set (x20) and Actinb primer probe set (x20) were used.
ApoB primer probe set (x20) (Taqman): ABI Hs00181142_m1 FAM
Actinb primer probe set (x20) (Taqman): ABI Hs01060665_g1 FAM

5 µL of 2x Taqman Fast Advanced Master Mix (Applied Biosystems), 2.5 µL of purified water, 0.5 µL of 20x Primer probe mix (10 µM), and 2 µL of cDNA (diluted 5 times) were suspended per well to adjust a PCR reaction solution (per tube) and to perform a PCR reaction using Quantstadio7 (Applied Biosystems) (95°C 20 sec, 40 cycles (95°C 1 sec, 60°C 20 sec), 4°C Hold). Using Actinb as an endogenous control, the relative expression level of ApoB mRNA was evaluated.

### (3-4) Evaluation of inhibition of ApoB mRNA expression by Example compound using human liver cultured cells (2)

The compound synthesized in Example 24 adjusted to 10 nM and 100 nM was added simultaneously with the seeding of human primary hepatocytes, and the ApoB mRNA expression in the cells after culture for 72 hours was evaluated by qRT-PCR (Taqman assay). When the average ApoB mRNA expression in 4 wells with no compounds added was taken as 100%, 10 nM ApoB-ASO inhibited the expression by 4.4%, and the ApoB-ASO conjugate inhibited the expression by 30.3%, as shown in Figure 3. 100 nM ApoB-ASO inhibited the expression by 41.2%, and the ApoB-ASO conjugate inhibited the expression by 68.6%. This indicates that a compound with a GalNAc derivative bound can inhibit the expression more strongly than compounds with no GalNAc derivatives bound.

### (Example 25) DMTr-on ion exchange column purification of oligonucleotide bound to GalNAc unit having 4,4'-dimethoxytrityl group on hydroxyl group at position 6 in GalNAc moiety

### (1) Synthesis of oligomer and preparation of mixed solution

The compound described in Example 16 was synthesized. However, AKTA Oligopilot (available from GE Healthcare) was used as a nucleic acid automatic synthesizer, Primer Support 5G Unylinker 350, 390 µmol (available from GE Healthcare) was used as a solid phase carrier, and a synthetic program suitable for the scale was used. After completion of the synthesis, a mixture of a conjugate and an oligonucleotide analog having a target oligonucleotide sequence with the hydroxyl group at position 6 in the GalNAc moiety protected by a 4,4'-dimethoxytrityl group was obtained. The mixture obtained was divided into two, and each was treated with 25 mL of concentrated ammonia water to excise the oligonucleotide from the support, together with removal of a cyanoethyl group as a protecting group on the phosphorus atom and a protecting group on a nucleobase and filtration using a tube with a filter, to obtain an oligonucleotide mixed solution (25 mL) (two bottles) containing the target conjugate. Using AKTA pure150 (available from GE Healthcare) as a purification device, a column FineLINE Pilot (35 mm x 100 mm) (available from GE Healthcare) was filled with a filler SOURCE 15Q (96 mL) (available from GE Healthcare), the aforementioned oligonucleotide mixture was purified in two separate batches by the following method. The UV detection wavelength was set to 260 nm, 280 nm, or 350 nm, and the following three steps were used as one purification program. Figure 5 shows a chromatogram detecting absorption at the detection wavelength of 260 nm and 350 nm as the column passing fluid was detected in these steps.

### (i) Step of adsorbing DMTr-on oligonucleotide on column

The flow rate was set to 40 ml/min, and 1 CV of buffer A1 (10 mM aqueous sodium hydroxide solution) was poured. The flow rate was changed to 10 ml/min, the oligonucleotide mixture (25 mL) was added to the top of the column, and 170 mL of buffer A1 (10 mM aqueous sodium hydroxide solution) was diverted. Subsequently, the flow rate was set to 40 ml/min, and 1 CV of buffer A1 (10 mM aqueous sodium hydroxide solution) was poured. Subsequently, 6 CV of buffer A1 (10 mM aqueous sodium hydroxide solution):buffer B1 (10 mM aqueous sodium hydroxide/2 M aqueous sodium chloride solution) (35:65) was poured, to wash away oligonucleotides with no X²⁰ bound and impurities. Finally, 2 CV of buffer A1 (10 mM aqueous sodium hydroxide solution) was poured.

### (ii) Step of de-DMTr conversion on column

The flow rate was set to 40 ml/min, and buffer A2 (0.4% trifluoroacetic acid aqueous solution) 1 CV of was poured. Subsequently, the flow rate was changed to 20 ml/min, and 6.25 CV was poured, thereby performing de-DMTr conversion of oligonucleotides with X²⁰ bound that were adsorbed on the column filler.

### (iii) Step of eluting target substance

The flow rate was set to 40 ml/min, and 3 CV of buffer A1 (10 mM aqueous sodium hydroxide solution) was poured for equilibration. After gradient feeding (15 CV) of buffer A1 (10 mM aqueous sodium hydroxide solution):buffer B1 (10 mM aqueous sodium hydroxide/2 M aqueous sodium chloride solution) from (75:25) to (25:75), 2 CV of buffer B1 (10 mM aqueous sodium hydroxide/2 M aqueous sodium chloride solution) was poured, to fractionate the de-DMTr converted oligonucleotides (amount fractionated was 48 mL).

The fractions detected by UV (260 nm) during the elution were checked using a microspectrophotometer Xpose (available from Trinean nv), to obtain fraction solutions (first time: 432 ml and second time: 480 ml) (Figure 5).

After using the column once, it was washed with a 2 M aqueous sodium chloride solution containing 30% isopropyl alcohol before the next purification.

### (2) Concentration, dialysis, and freeze drying

The fraction solutions (first time: 432 ml and second time: 480 ml) were combined into one, and an appropriate amount of acetic acid was added thereto, to prepare a mixture with a pH of 7.5 to 8.0. Thereafter, it was concentrated using a KR2i TFF system (Repligen Corporation) equipped with Xampler ultrafiltration cartridge 3K, 140 cm² (GE Healthcare) and then dialyzed against Otsuka distilled water, followed by freeze drying. A freeze-dried powder of the target substance (1.14 g in total) was obtained.

### (Comparative Example 1) DMTr-on ion exchange column purification of conjugate with DMTr phase introduced near oligonucleotide

For comparison with Example 25, a GalNAc conjugate composed of X¹³ shown below was subjected to DMTr-on ion exchange column purification. The protecting groups of the hydroxyl group at position 3, the hydroxyl group at position 4, and the hydroxyl group at position 6 on GalNAc contained in X¹³ can be unified into an acetyl group. This eliminates the need for distinguishing the protecting groups in the synthesis step and therefore facilitates synthesizing a GalNAc unit moiety. Further, the number of GalNAcs can be easily increased or decreased by adjusting the number of times of coupling to the oligonucleotide on the 5' end side in the solid phase synthesis step. However, only one 4,4'-dimethoxytrityl group can be left after completion of the solid phase synthesis of the oligomer.

### [Comparative Example 1-1] Synthesis of GalNAc unit amidite (compound Ref1F) corresponding to X13 (Ref1A) Synthesis of 9H-fluorene-9-ylmethyl N-[2-[2-[(2R)-3-benzyloxy-2-hydroxy-propoxy]ethoxy]ethyl] carbamate (compound Ref1A)

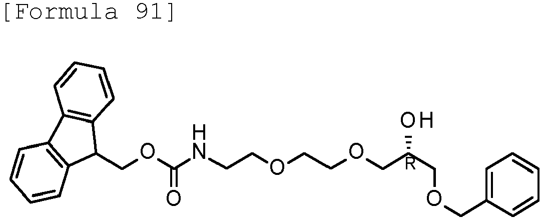

9H-Fluorene-9-ylmethyl N-[2-(2-hydroxyethoxy)ethyl] carbamate as a compound known in the literature (Bioconjugate Chemistry, 2000, 11, 755-761) (3.11 g, 10 mmol) and benzyl (S)-(+)-glycidyl ether (0.8 g, 5 mmol) were dissolved in dichloromethane (30 mL), and a boron trifluoride-diethyl ether complex (0.1 mL, 1 mmol) was added thereto, followed by stirring at room temperature for 16 hours. After completion of the reaction, a saturated sodium bicarbonate aqueous solution was added. The organic layer was washed with a saturated aqueous sodium bicarbonate solution and saturated saline, followed by drying over anhydrous sodium sulfate, filtration, and distilling off the solvent under reduced pressure, to obtain a crude product. It was purified by silica-gel column chromatography (hexane: ethyl acetate = 80:20-0:100, v/v), to obtain an oily target substance Ref1A (0.74 g, yield 30%).

¹H-NMR (CDCl₃) δ: 7.76 (2H, d, J = 7.9 Hz), 7.60 (2H, d, J= 7.3 Hz), 7.42-7.37 (2H, m), 7.35-7.27 (7H, m), 5.40 (1H, s), 4.53 (2H, s), 4.39 (2H, d, J = 6.7 Hz), 4.25-4.19 (1H, m), 4.04-3.97 (1H, m), 3.69-3.35 (12H, m). Calcd for C₂₉H₃₃NO₆: [M+H]⁺492, Found 492.

### (Ref1B) Synthesis of (2R)-1-[2-(2-aminoethoxy)ethoxy]-3-benzyloxy-propane-2-ol, trifluoroacetate (compound Ref1B)

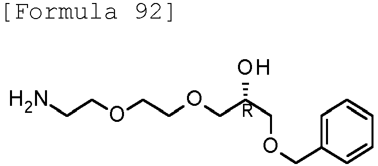

The compound Ref1A (13.8 g, 28.1 mmol) synthesized in step (Ref1A) was dissolved in ethanol/tetrahydrofuran (50 mL/50 mL), and a 1 N aqueous sodium hydroxide solution (100 mL) was added thereto, followed by stirring at room temperature for 2 hours. After completion of the reaction, the organic solvent was distilled off under reduced pressure, and 1 N hydrochloric acid (100 mL) was added. The solid product generated was removed by filtration. The by-products contained in the aqueous layer that was a passing fluid were extracted with ethyl acetate and distilled off under reduced pressure, and acetonitrile was added to the residue obtained, followed by filtration. The passing fluid was distilled off under reduced pressure to obtain a crude product. Separation and purification were performed by reverse-phase HPLC (GL Sciences Inc., Inertsil ODS-3) using a 0.1% trifluoroacetic acid aqueous solution and a 0.1% trifluoroacetic acid acetonitrile solution as eluents, the fractions containing the target substance were combined, and the solvent was distilled off under reduced pressure, to obtain an oily target substance Ref1B (7.1 g, yield 66%).

¹H-NMR (CDCl₃) δ: 7.38-7.27 (5H, m), 4.55 (2H, s), 4.07-3.99 (1H, m), 3.77-3.48 (10H, m), 2.96-2.88 (2H, m).

### (Ref1C) Synthesis of acetic acid [(2R,3R,4R,5R,6R)-5-acetoamido-3,4-diacetoxy-6-[2-[2-[2-[(2R)-3-benzyloxy-2-hydroxy-propoxy]ethoxy]ethylamino]-2-oxo-ethoxy]tetrahydropyran-2-yl]methyl (compound Ref1C)

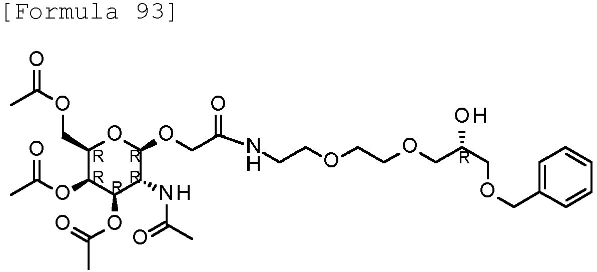

2-[(2R,3R,4R,5R,6R)-3-Acetoamido-4,5-diacetoxy-6-(acetoxymethyl)tetrahydropyran-2-yl]oxyacetic acid as a compound known in the literature (International Publication No. WO 2012037254) (6 g, 14.8 mmol), 1-(-3-dimethylaminopropyl)-3-ethylcarbodiimide hydrochloride (3.4 g, 17.8 mmol), 3H-1,2,3-triazole [4,5-b]pyridine-3-ol (2.0 g, 14.8 mmol), N,N-diisopropylethylamine (9.0 mL, 51.8 mmol), and the compound Ref1B (6.7 g, 17.5 mmol) synthesized in step (Ref1B) were dissolved in dichloromethane (130 mL), followed by stirring at room temperature for 4 hours. After completion of the reaction, the organic layer was washed with 1 N hydrochloric acid, a saturated sodium bicarbonate aqueous solution, and saturated saline, followed by drying over anhydrous sodium sulfate, filtration, and distilling off the solvent under reduced pressure, to obtain a crude product. The solvent was distilled off under reduced pressure, to obtain a crude product. It was purified by silica-gel column chromatography (ethyl acetate: methanol = 100:0-90:10, v/v), to obtain an amorphous target substance Ref1C (7.9 g, yield 81%).

¹H-NMR (CDCl₃) δ: 7.39-7.28 (5H, m), 7.00-6.93 (1H, m), 6.50-6.44 (1H, m), 5.33-5.29 (1H, m), 5.16 (1H, dd, J = 11.5, 3.6 Hz), 4.63-4.53 (3H, m), 4.34 (1H, d, J = 15.1 Hz), 4.22-3.83 (5H, m), 3.73-3.26 (13H, m), 2.15 (3H, s), 2.05 (3H, s), 1.99 (6H, s).

Calcd for C₃₀H₄₄N₂O₁₄: [M+H]⁺ 657, Found 657.

### (Ref1D) Synthesis of acetic acid [(2R,3R,4R,5R,6R)-5-acetoamido-3,4-diacetoxy-6-[2-[2-[2-[(2R)-2,3-dihydroxypropoxy]ethoxy]ethylamino]-2-oxo-ethoxy]tetrahydropyran-2-yl]methyl acetate (compound Ref1D)

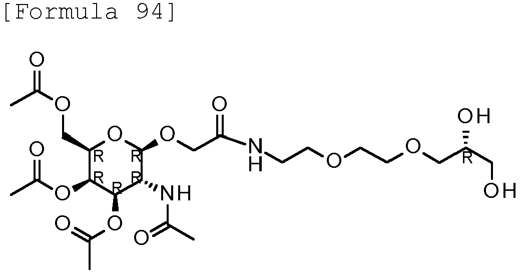

The compound Ref1C (7.9 g, 12 mmol) synthesized in step (Ref1C) was dissolved in ethanol (80 mL). 20% palladium hydroxide/carbon (wet) (2.0 g) was added thereto, followed by vigorous stirring at 50°C under a hydrogen atmosphere for 8 hours. After completion of the reaction, the mixture was filtered, and the passing fluid obtained was distilled off under reduced pressure. An amorphous target substance Ref1D (6.7 g, yield 98%) was obtained.

¹H-NMR (CDCl₃) δ: 7.03-6.98 (1H, m), 6.43 (1H, d, J = 9.1 Hz), 5.35 (1H, d, J = 3.3 Hz), 5.15 (1H, dd, J = 11.2, 3.3 Hz), 4.58 (1H, d, J = 7.9 Hz), 4.35 (1H, d, J = 15.7 Hz), 4.28-3.82 (5H, m), 3.77-3.34 (13H, m), 2.18 (3H, s), 2.06 (3H, s), 2.03 (3H, s), 2.01 (3H, s).

Calcd for C₂₃H₃₈N₂O₁₄: [M+H]⁺ 568, Found 568.

### (Ref1E) Synthesis of acetic acid [(2R,3R,4R,5R,6R)-5-acetoamido-3,4-diacetoxy-6-[2-[2-[2-[(2S)-3-[bis(4-methoxyphenyl)-phenyl-methoxy]-2-hydroxy-propoxy]ethoxy]ethylamino]-2-oxo-ethoxy]tetrahydropyran-2-yl]methyl (compound Ref1E)

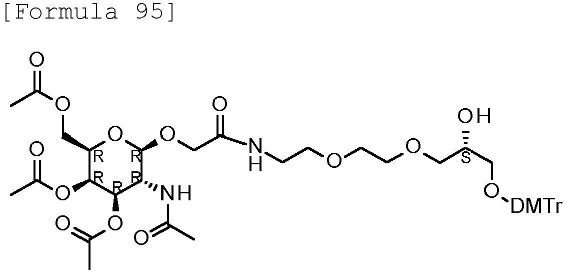

An appropriate amount of pyridine was added to the compound Ref1D (6.8 g, 12 mmol) synthesized in step (Ref1D) and azeotroped under reduced pressure. It was dissolved in pyridine (40 mL), and 4,4'-dimethoxytrityl chloride (4.5 g, 13 mmol) was added thereto, followed by stirring at room temperature for 2 hours. After completion of the reaction, the solvent was distilled off under reduced pressure. After an appropriate amount of toluene was added and azeotroped, a crude product was obtained. This was purified by silica-gel column chromatography (ethyl acetate: acetic acid ethyl/methanol (5/1) = 100:0-50:50, v/v, containing 1% triethylamine), to obtain an amorphous target substance ReflE (6.9 g, yield 66%).

¹H-NMR (CDCl₃) δ: 7.42 (2H, d, J = 7.9 Hz), 7.33-7.19 (7H, m), 7.01-6.94 (1H, m), 6.83 (4H, d, J = 9.1 Hz), 6.37 (1H, d, J = 8.5 Hz), 5.33 (1H, d, J = 3.3 Hz), 5.15 (1H, dd, J = 11.2, 3.3 Hz), 4.58 (1H, d, J = 7.9 Hz), 4.32 (1H, d, J = 15.7 Hz), 4.22-3.86 (5H, m), 3.79 (6H, s), 3.66-3.12 (13H, m), 2.13 (3H, s), 2.03 (3H, s), 1.97 (3H, s), 1.95 (3H, s).

Calcd for C₄₄H₅₆N₂O₁₆: [M+Na]⁺ 893, Found 892.

### (Ref1F) Synthesis of acetic acid [(2R,3R,4R,5R,6R)-5-acetoamido-3,4-diacetoxy-6-[2-[2-[2-[(2S)-3-[bis(4-methoxyphenyl)-phenyl-methoxy]-2-[2-cyanoethoxy-(diisopropylamino)phosphanyl]oxy-propoxy]ethoxy]ethylamino]-2-oxo-ethoxy]tetrahydropyran-2-yl]methyl (compound Ref1F)

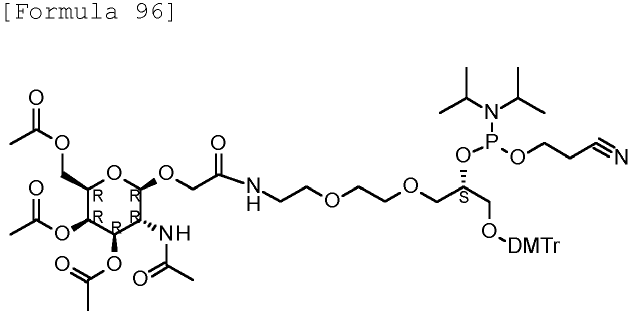

The compound ReflE (6.9 g, 7.9 mmol) synthesized in step (Ref1E) was azeotroped with an appropriate amount of toluene and then dissolved in dichloromethane (80 mL).

N,N-diisopropylethylamine (5.5 mL, 32 mmol) and 2-cyanoethyldiisopropylchlorophosphoroamidite (2.1 mL, 9.5 mmol) were added thereto, followed by stirring at room temperature for 1 hour. After completion of the reaction, the solvent was distilled off under reduced pressure, to obtain a crude product. This was purified by silica-gel column chromatography (ethyl acetate: acetic acid ethyl/methanol (5/1) = 100:0-50:50, v/v, containing 1% triethylamine), to obtain an amorphous target substance ReflF (6.6 g, yield 78%).

¹H-NMR (CDCl₃) δ: 7.47-7.40 (2H, m), 7.35-7.17 (7H, m), 6.98-6.91 (1H, m), 6.85-6.78 (4H, m), 6.05-5.89 (1H, m), 5.37-5.33 (1H, m), 5.17-5.07 (1H, m), 4.59-4.49 (1H, m), 4.37-4.30 (1H, m), 4.23-3.06 (28H, m), 2.69-2.42 (2H, m), 2.15 (3H, s), 2.05 (3H, s), 2.02-1.98 (3H, m), 1.97-1.94 (3H, m), 1.30-1.00 (12H, m).

### [Comparative Example 1-2] Synthesis of conjugate with X13 introduced

HO-X^13-X^13-X^13-A^m1s-A^e2s-U^m1s-C^m1s-C^e2s-G^m1s-A^m1s-T^e2s-G^m1s-G^m1s-C^e2s-

G^m1s-A^m1s-A^e2s-G^m1t-H (SEQ ID No: 40)

X¹⁸ was replaced with X¹³ using the compound ReflF as a GalNAc unit amidite, to perform synthesis in the same manner as in Example 8. However, the addition reaction of the GalNAc unit amidite was repeated three times to bind three X13s.

The nucleotide sequence of this compound was a sequence complementary to the sequence from the 92nd to the 106th from the 5' end of exon 5 of c.648G>T mutated G6PC gene with a mutation at nucleotide 728 in Homo sapiens glucose-6-phosphatase catalytic subunit (G6PC), transcript variant 1, mRNA (NCBI-GenBank accession No. NM_000151.3) from G to T. The compound was identified by negative ion ESI mass spectrometry (measured value: 6878.11) .

### [Comparative Example 1-3] DMTr-on ion exchange column purification of oligonucleotide bound to GalNAc unit having 4,4'-dimethoxytrityl group on 5' end side (1) Synthesis of oligomer and preparation of mixed solution

HO-X13-X13-X13-Am1s-Ae2s-Um1s-Cm1s-Ce2s-Gm1s-Am1s-Te2s-Gm1s-Gm1s-Ce2s-

Gm1s-Am1s-Ae2s-Gm1t-H (SEQ ID No: 40)

AKTA Oligopilot (available from GE Healthcare) was used as a nucleic acid automatic synthesizer, Primer Support 5G Unylinker 350, 390 µmol (available from GE Healthcare) was used as a solid phase carrier, and a synthetic program suitable for the scale was used. Using 10 µmol of the solid phase carrier obtained, the GalNAc units X¹³ were condensed three times with an ABI 392 DNA/RNA Synthesizer (available from Applied Biosystems). After completion of the synthesis, a mixture of oligonucleotide analogs having a target oligonucleotide sequence and bound to a GalNAc unit having a 4,4'-dimethoxytrityl group on the 5' end side was obtained. The oligonucleotides were excised from the support by treatment with 6 mL of concentrated ammonia water, and a cyanoethyl group as a protecting group on a phosphorus atom and a protecting group on a nucleobase were removed.

### Purification method 1: Clarity QSP (available from Phenomenex Inc.) purification

A target substance was obtained by performing purification in the same manner as in Example 8.

The nucleotide sequence of this compound was a sequence complementary to the sequence from the 92nd to the 106th from the 5' end of exon 5 of c.648G>T mutated G6PC gene with a mutation at nucleotide 728 in Homo sapiens glucose-6-phosphatase catalytic subunit (G6PC), transcript variant 1, mRNA (NCBI-GenBank accession No. NM_000151.3) from G to T. The compound was identified by negative ion ESI mass spectrometry (measured value: 6878.11) .

### Purification method 2: DMTr-on ion exchange column purification

The oligonucleotide mixture was substituted with a 10 mM aqueous sodium hydroxide solution before DMTr-on ion exchange column purification.

Using AKTA pure150 (available from GE Healthcare) as a purification device and RESOURCE Q 6 mL (available from GE Healthcare) as a purification column, the aforementioned oligonucleotide mixture was purified by the following method. The UV detection wavelength was set to 260 nm, and the following three steps were used as one purification program. Figure 6 shows a chromatogram at a detection wavelength of 260 nm of the column passing fluid in these steps.
(i) Step of adsorbing DMTr-on oligonucleotide on ion exchange column
   2 CV of buffer A1 (10 mM aqueous sodium hydroxide solution) was poured at a flow rate of 3 ml/min. The oligonucleotide mixture (1.5 mL, 10 mM aqueous sodium hydroxide solution) was added to the top of the column at a flow rate of 2 ml/min, and 2.5 CV of buffer A1 (10 mM aqueous sodium hydroxide solution) was poured. Subsequently, 1 CV of buffer A1 (10 mM aqueous sodium hydroxide solution) was poured at a flow rate of 3 ml/min, and then buffer A1 (10 mM aqueous sodium hydroxide solution):buffer B1 (10 mM aqueous sodium hydroxide/2 M aqueous sodium chloride solution) (2 CV of 40:60 to 25:75 and 4 CV of 25:75) was poured at a flow rate of 2.4 ml/min.
(ii) Step of de-DMTr conversion on ion exchange column 7 CV of buffer A2 (0.4% trifluoroacetic acid aqueous solution) was poured at a flow rate of 1.2 ml/min.
(iii) Step of eluting target substance
   5 CV of buffer A1 (10 mM aqueous sodium hydroxide solution) was poured at a flow rate of 3 ml/min for equilibration. Subsequently, after gradient feeding (10 CV) of buffer A1 (10 mM aqueous sodium hydroxide solution):buffer B1 (10 mM aqueous sodium hydroxide/2 M aqueous sodium chloride solution) from (30:70) to (0:100) at a flow rate of 3 ml/min, 2 CV of buffer B1 (10 mM aqueous sodium hydroxide/2 M aqueous sodium chloride solution) was poured.

It was found from the results of Figure 6 that, since absorption at 260 nm was not detected in the elution step after deprotection, the DMT-on conjugate was not adsorbed on the column in the first adsorption step and passed therethrough. Therefore, no target substances could be yielded by ion exchange column purification. From this, it is considered to be important for DMTr-on column purification of a conjugate of a GalNAc unit with an oligonucleotide that a hydrophobic protecting group be bound onto GalNAc, and/or the number of hydrophobic protecting groups contained in one GalNAc unit be equal to or more than the number of GalNAcs.

In this description, A^{t}, G^{t}, 5meC^{t}, C^{t}, T^{t}, U^{t}, A^{p}, G^{p}, 5meC^{p}, CP, T^{p}, U^{p}, A^{s}, G^{s}, 5meC^{s}, C^{s}, T^{s}, U^{s}, A^{m1t}, G^{m1t}, C^{m1t}, 5meC^{m1t}, U^{m1t}, A^{m1p}, G^{m1p}, C^{m1p}, 5meC^{m1p}, U^{m1p}, A^{m1s}, G^{m1s}, C^{m1s}, 5meC^{m1s}, U^{m1s}, A^{2t}, G^{2t}, C^{2t}, T^{2t}, A^{e2p}, G^{e2p}, C^{e2p}, T^{e2p}, A^{e2s}, G^{e2s}, C^{e2s}, T^{e2s}, A^{1t}, G^{1t}, C^{1t}, T^{1t}, A^{e1p}, G^{e1p}, C^{e1p}, T^{e1p}, A^{e1s}, G^{e1s}, C^{e1s}, T^{e1s}, A^{m2t}, G^{m2t}, 5meC^{m2t}, T^{m2t}, A^{m2p}, G^{m2p}, 5meC^{m2p}, T^{m2p}, A^{m2s}, G^{m2s}, 5meC^{m2s}, T^{m2s}, X, X¹, X², X³, X⁴, X⁵, X⁶, X⁷, X⁸, X⁹, X¹⁰, X¹¹, X¹², X¹³, X¹⁴, X¹⁵, X¹⁶, X¹⁷, X¹⁸, X¹⁹, X²⁰, X²¹, and X²² is a group having the structure shown below.

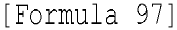

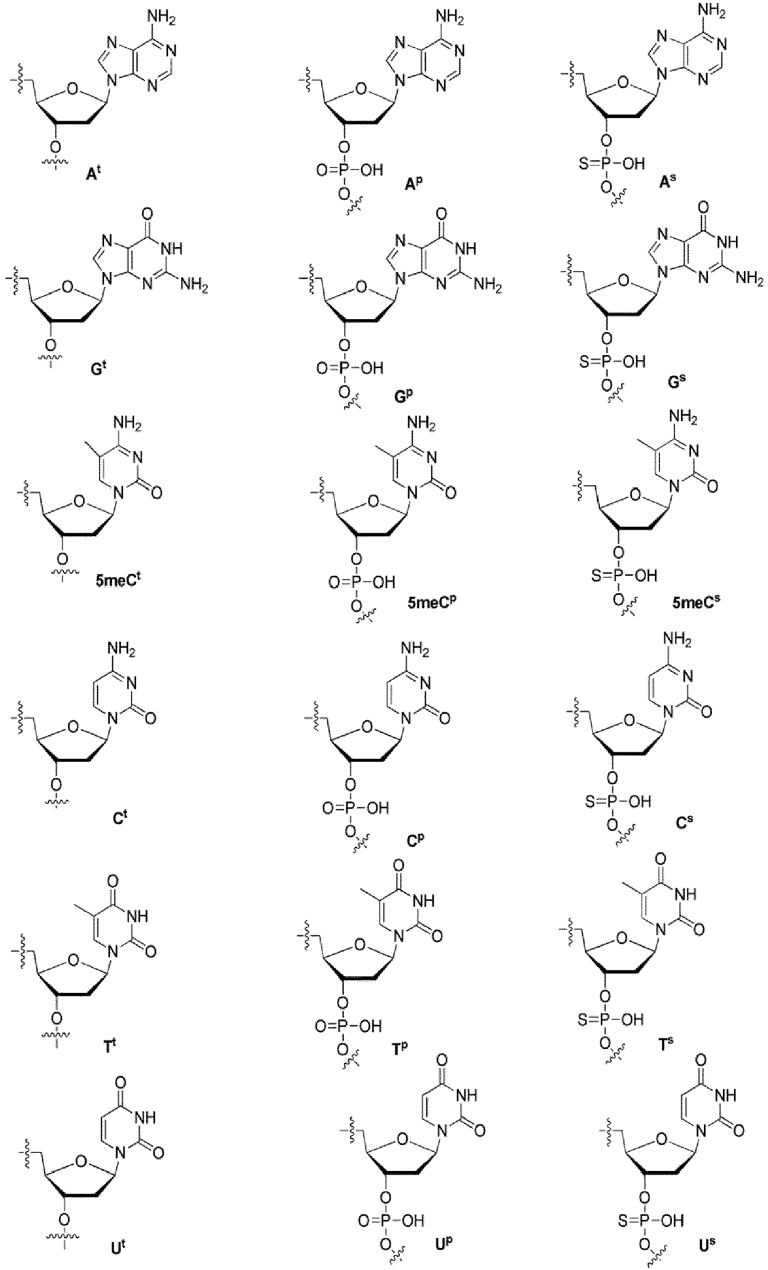

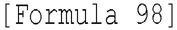

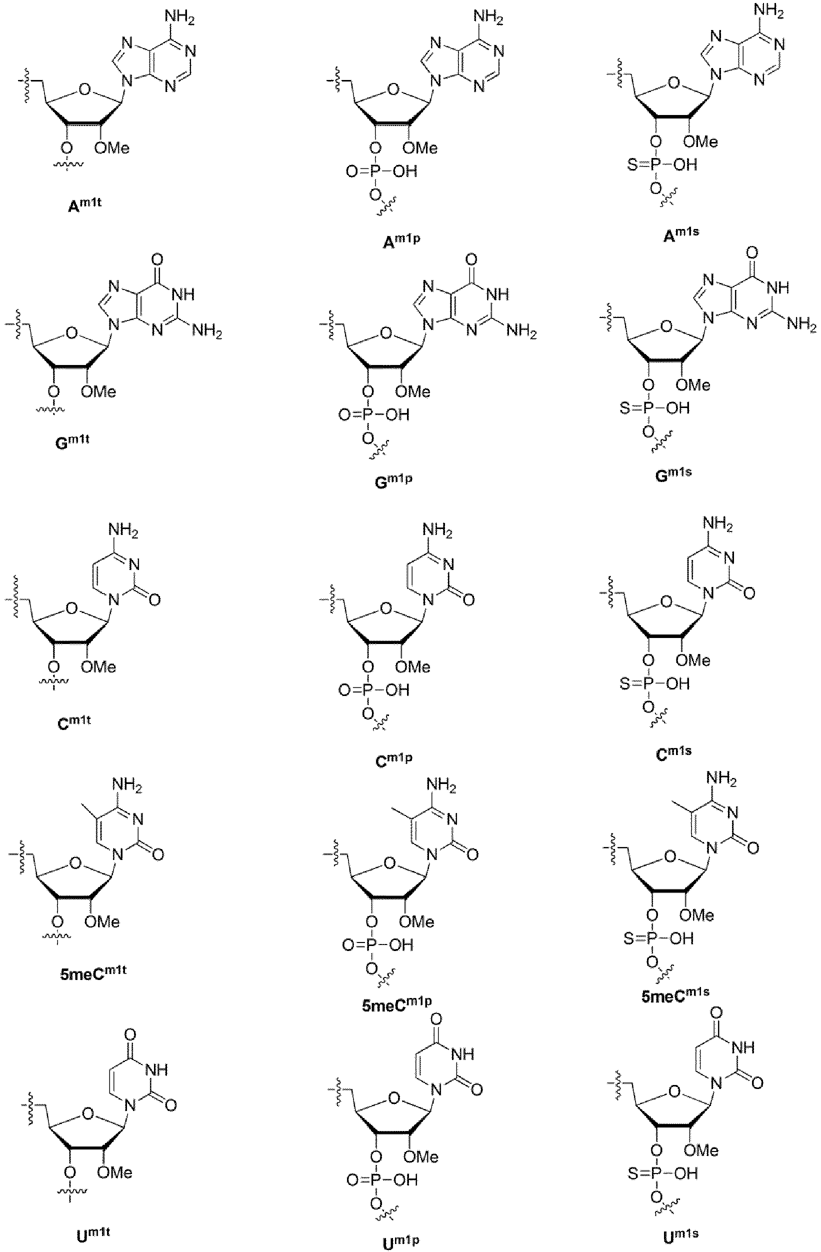

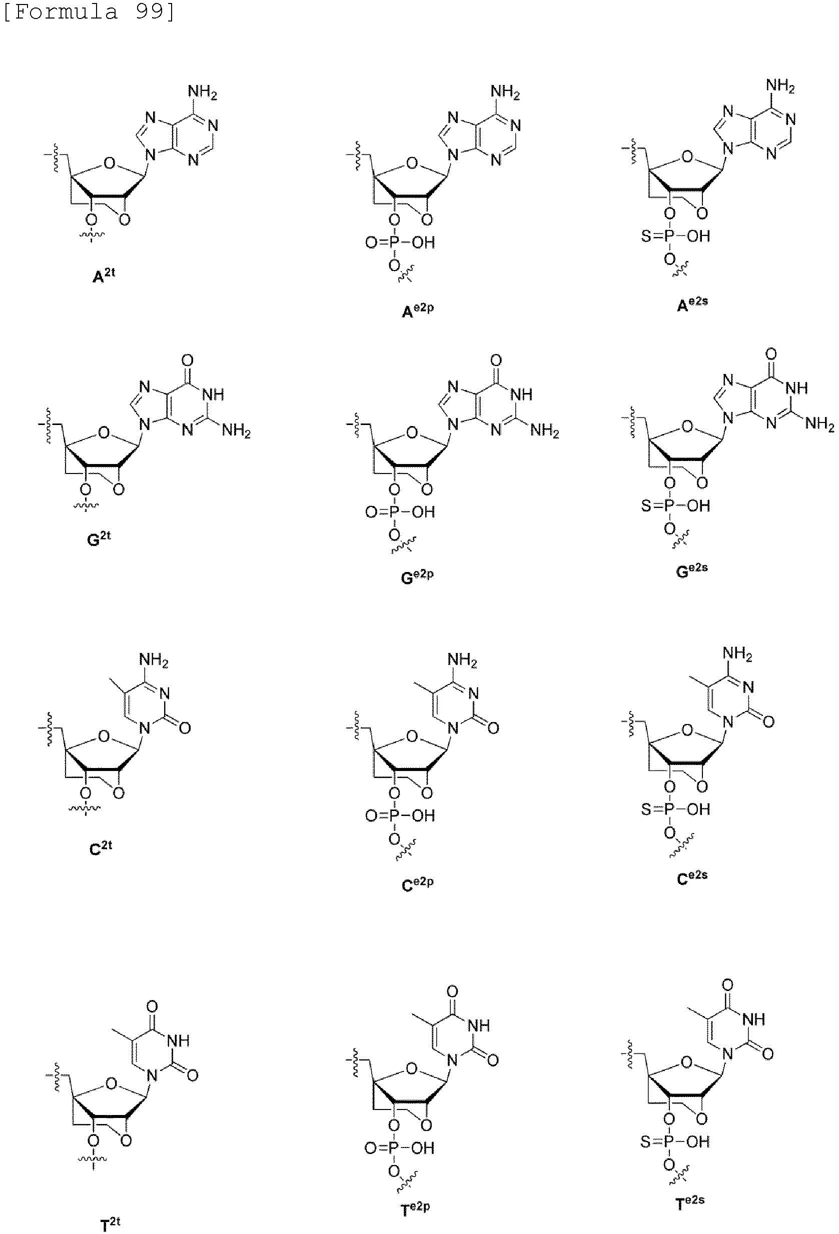

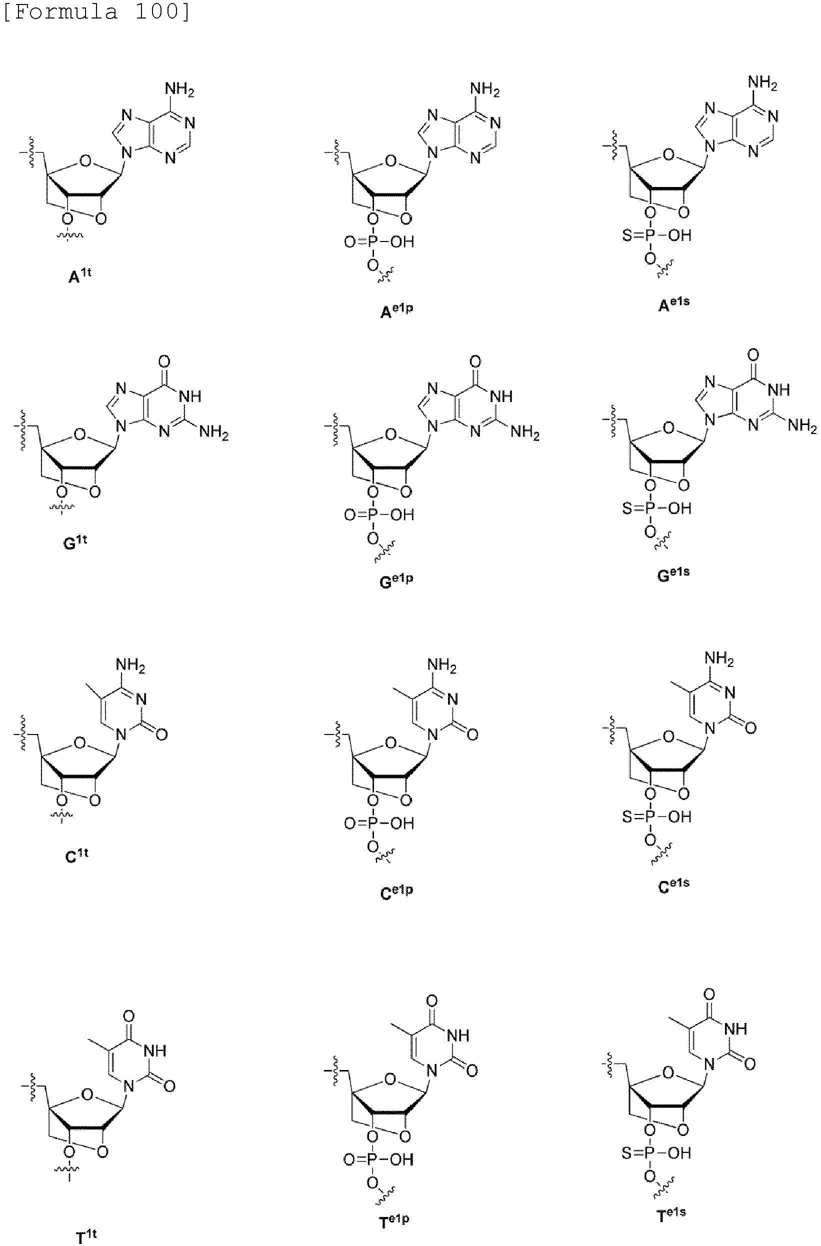

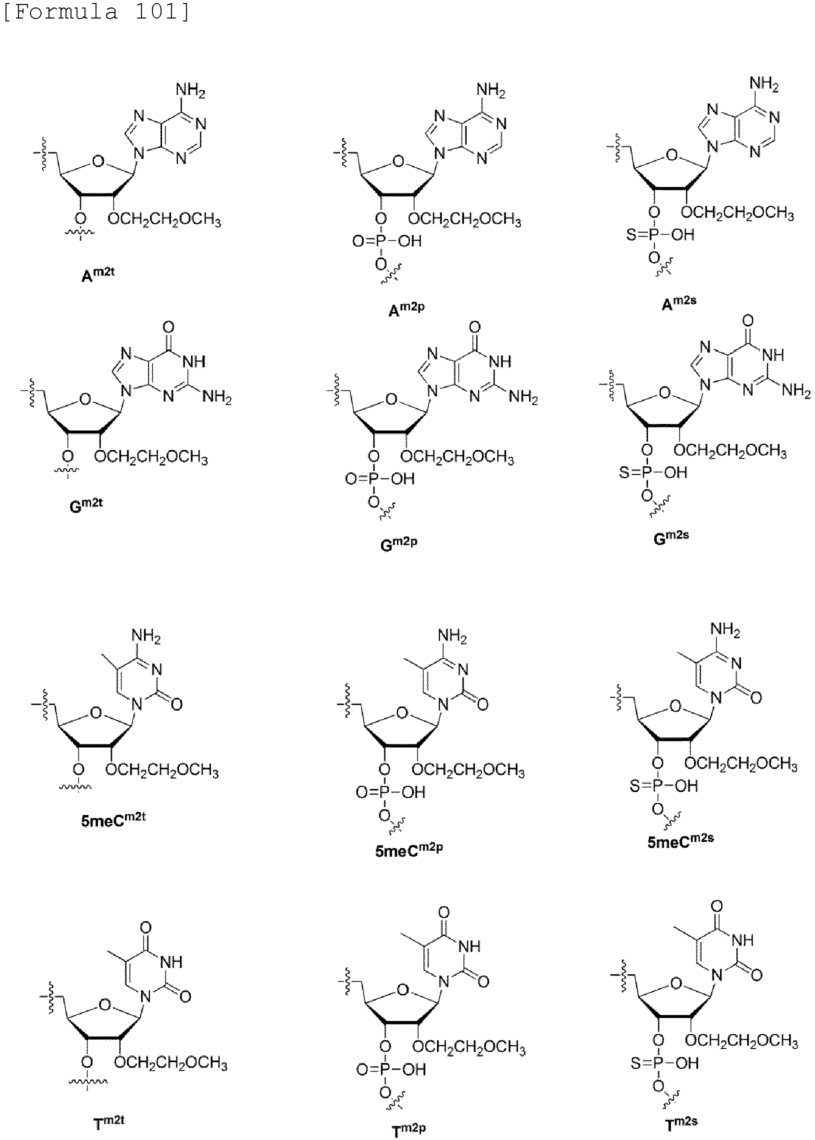

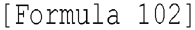

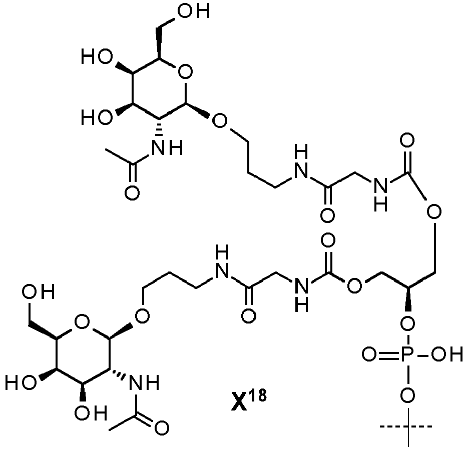

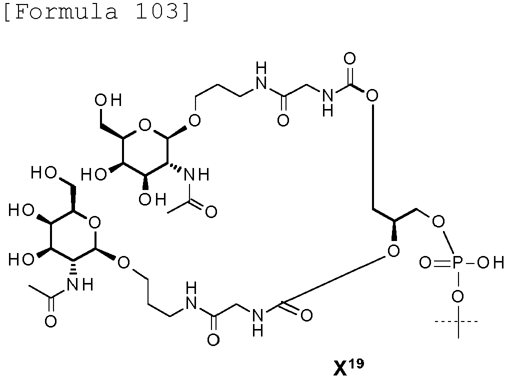

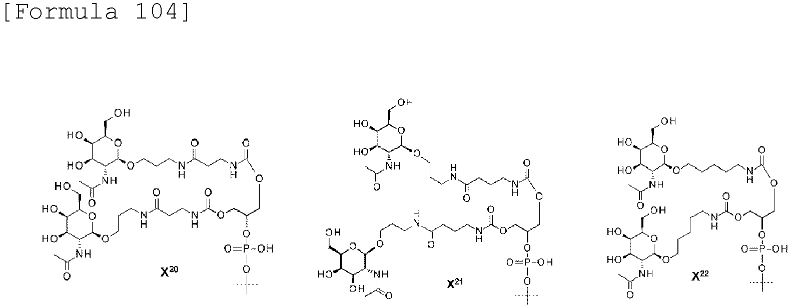

All publications, patents, and patent applications cited herein are incorporated herein by reference in their entirety.

### Industrial Applicability

The present invention can be used for treating glycogen storage disease type Ia.

### Sequence listing free text

SEQ ID Nos: 1 to 48 and 93 to 96 each represent the sequence of an antisense oligonucleotide. Nucleotides constituting the antisense oligonucleotide may be any of natural DNA, natural RNA, DNA/RNA chimera, and modified forms of these, but at least one of them is preferably a modified nucleotide. SEQ ID Nos: 49 to 65 and 67 to 92 each represent the sequence of a primer. SEQ ID No: 66 represents the sequence of a peptide.

## Claims

1. A conjugate of an oligonucleotide having a nucleic acid sequence expected to have a pharmacological effect in hepatic parenchymal cells with a biantennary GalNAc unit, or a pharmaceutically acceptable salt thereof, wherein, in the conjugate, the biantennary GalNAc unit is represented by the following formula and is bound to the 5' end or the 3' end of the oligonucleotide, and
wherein the conjugate is delivered specifically to hepatic parenchymal cells in vivo: 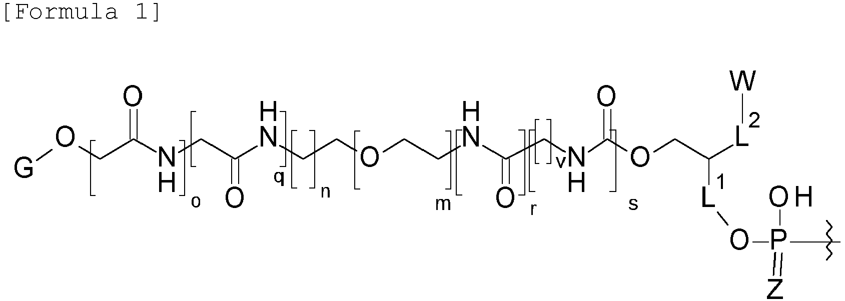 wherein W is a group represented by the following formula: 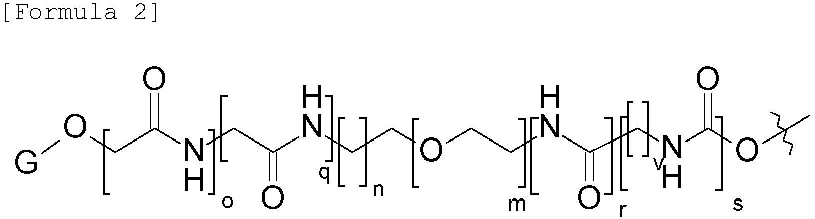 G represents a 5-acetoamido-2-hydroxymethyl-3,4-dihydroxytetrahydropyran-6-yl group (GalNAc); Z represents an oxygen atom or a sulfur atom; one of L¹ and L² represents a methylene group (CH₂), and the other represents no atoms interposed; o, q, r, and s each independently represent 0 or 1; n represents an integer of 1 to 15; m represents an integer of 0 to 5; and v represents 1 to 7, provided that when n is 1, then m is an integer of 0 to 5, and when n is an integer of 2 to 15, then m is 0.

2. The conjugate or a pharmaceutically acceptable salt thereof according to claim 1, wherein o and q in the formula of the biantennary GalNAc unit are each 0.

3. The conjugate or a pharmaceutically acceptable salt thereof according to claim 1 or 2, wherein the biantennary GalNAc unit is bound to the 5' end of the oligonucleotide.

4. The conjugate or a pharmaceutically acceptable salt thereof according to claim 1, wherein the biantennary GalNAc unit is a group represented by any one of the following formulas:

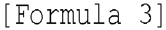

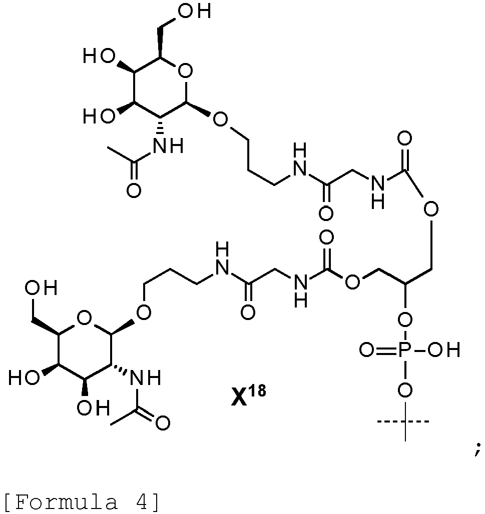

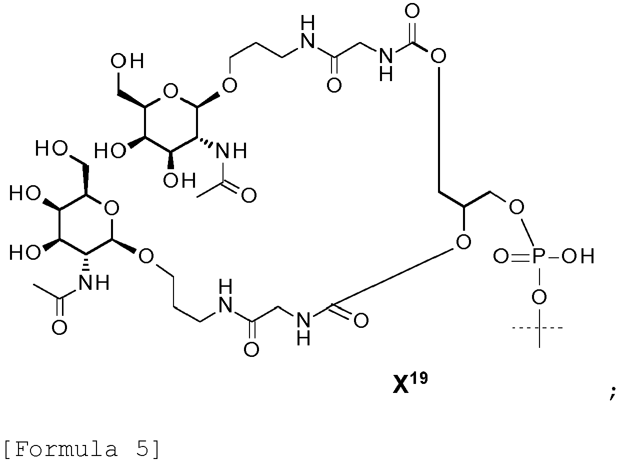

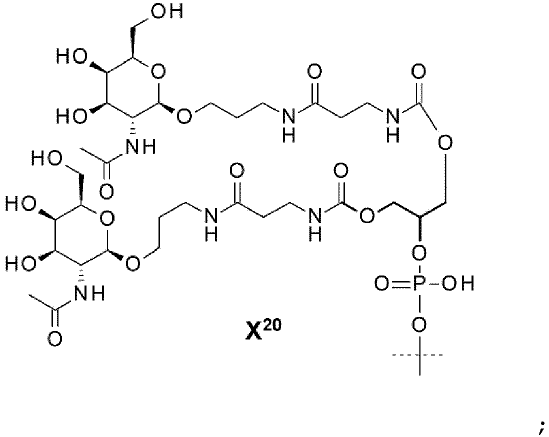

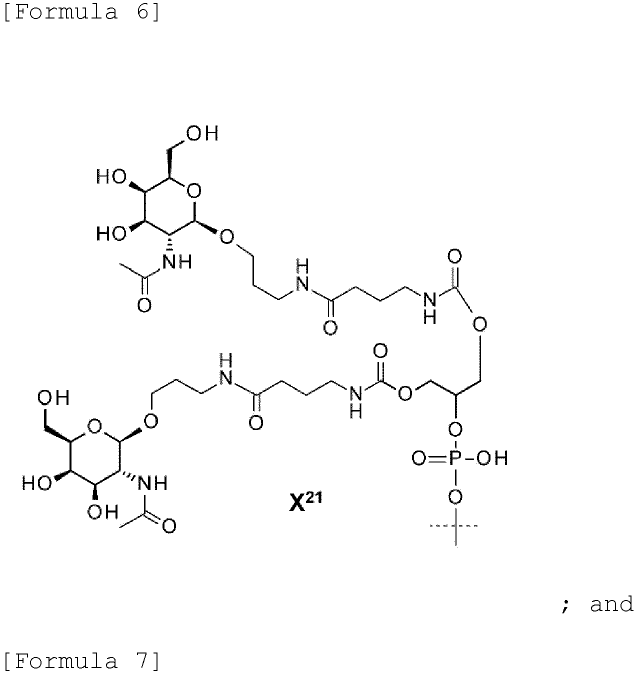

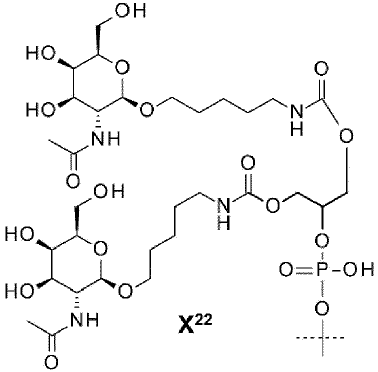

5. A medicament comprising the conjugate or a pharmaceutically acceptable salt thereof according to any one of claims 1 to 4.

6. A method for producing a conjugate of an oligonucleotide with a GalNAc unit, comprising the steps of:
a: synthesizing a conjugate of a structure comprising a hydrophobic protecting group-containing GalNAc unit-phosphate group represented by the following formula bound to the 5' end or the 3' end of the oligonucleotide: 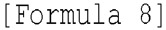 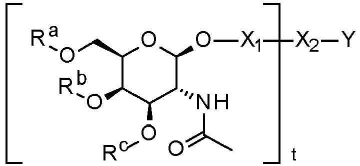 wherein at least one of R^{a}, R^{b}, and R^{c} is a hydrophobic protecting group, and the others are hydrogen atoms or protecting groups to be removed under release conditions; X₁ and X₂ each independently represent a linker structure, which may be branched between X₁ and X₂; t is the number of branched chains in the linker structure and is an integer of 1 to 10; and Y represents a bond which is bound to the oligonucleotide via a phosphate group or a thiophosphate group;
b: passing a solution containing the product of step a through a column filled with a resin having adsorptivity due to hydrophobic interaction, and passing an aqueous washing solution through the column; and
c: eluting the oligonucleotide from the column after step b.

7. The production method according to claim 6, wherein the hydrophobic protecting group is a protecting group to be removed under basic conditions or acidic conditions.

8. The production method according to claim 6, wherein the structure comprising a hydrophobic protecting group-containing GalNAc unit-phosphate group is bound to the 5'-hydroxyl group of the nucleoside located at the 5' end of the oligonucleotide.

9. The production method according to claim 6, wherein R^{a} is a hydrophobic protecting group, R^{b} and R^{c} are each a hydrogen atom, and t is an integer of 1 to 3 in the structure comprising a hydrophobic protecting group-containing GalNAc unit-phosphate group.

10. The production method according to claim 6, wherein the resin having adsorptivity due to hydrophobic interaction is any one selected from styrene-divinylbenzene adsorption resins, styrene-divinylbenzene modified adsorption resins, methacrylic adsorption resins, and phenolic adsorption resins.

11. The production method according to claim 6, wherein the column filled with a resin having adsorptivity due to hydrophobic interaction is an ion exchange column or a reverse phase column.

12. The production method according to claim 6, wherein step a comprises the following steps of:
a1: synthesizing a desired oligonucleotide on a support capable of extending the oligonucleotide in the direction from the 3' end to the 5' end by repeating deprotection of the protecting group of the 5'-hydroxyl group of the nucleoside located at the 5' end and condensation of the nucleoside using the support to extend the chain of the oligonucleotide, wherein protecting groups that undergo deprotection under conditions that allow cleavage of the binding between the 3' end of the oligonucleotide and the support (hereinafter referred to as "release conditions") are selected as the protecting groups other than the protecting group of the 5'-hydroxyl group of the nucleoside used, and a protecting group that does not undergo deprotection under the release conditions is selected as the protecting group of the 5'-hydroxyl group of the nucleoside;
a2: introducing a hydrophobic protecting group-containing GalNAc unit into the 5'-hydroxyl group of the nucleoside located at the 5' end of the oligonucleotide synthesized on the support, wherein a hydrophobic protecting group that does not undergo deprotection under the release conditions is selected; and
a3: treating the support obtained in step a2 with a solution under the release conditions to obtain a solution of a conjugate of a hydrophobic protecting group-containing GalNAc unit with an oligonucleotide.

13. The production method according to claim 6, comprising, after step b and prior to step c, subjecting the hydrophobic protecting group in the conjugate adsorbed on the column to deprotection.

14. The production method according to claim 6, wherein the release conditions are basic, and the hydrophobic protecting group is a protecting group to be removed under acidic conditions.

15. The production method according to claim 6, wherein the structure comprising a hydrophobic protecting group-containing GalNAc unit-phosphate group is represented by any one of the following formulas: 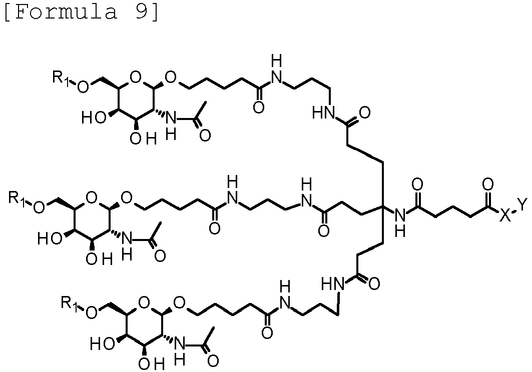 wherein R₁ represents a hydrophobic protecting group to be removed under acidic conditions, X represents a linker structure, Y represents a bond which is bound to the oligonucleotide via a phosphate group or a thiophosphate group, and the same applies below: 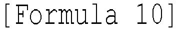 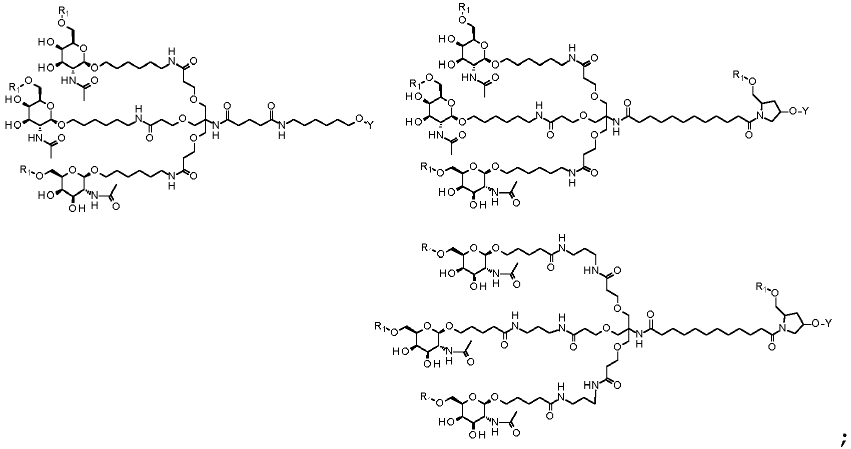 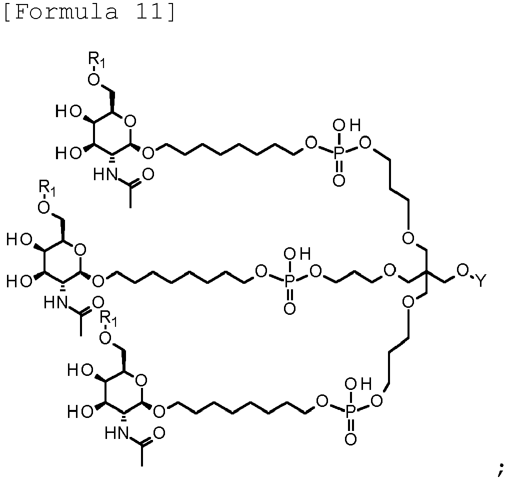 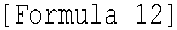 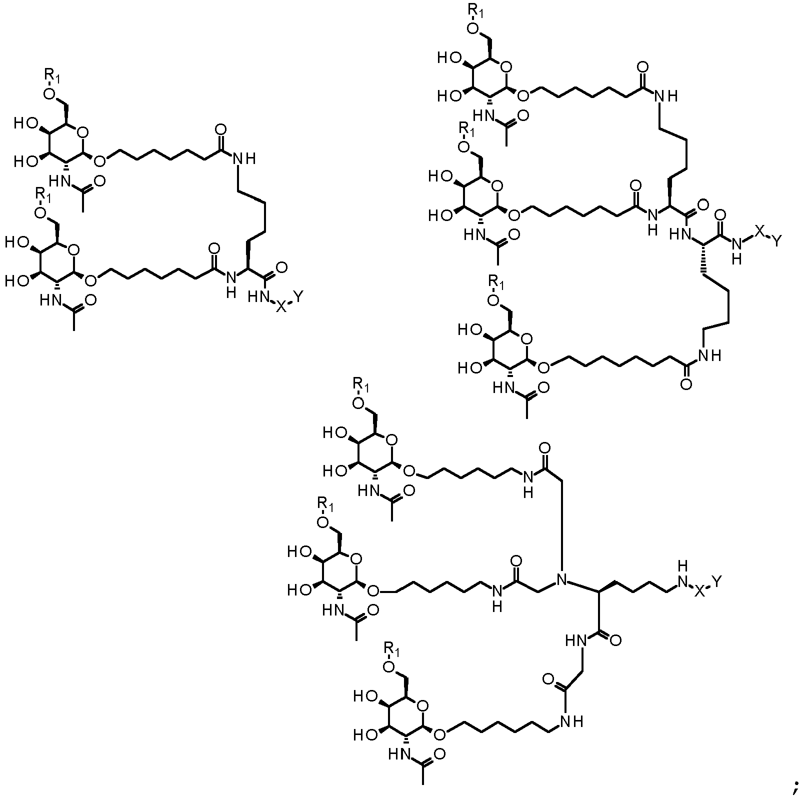 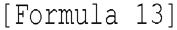 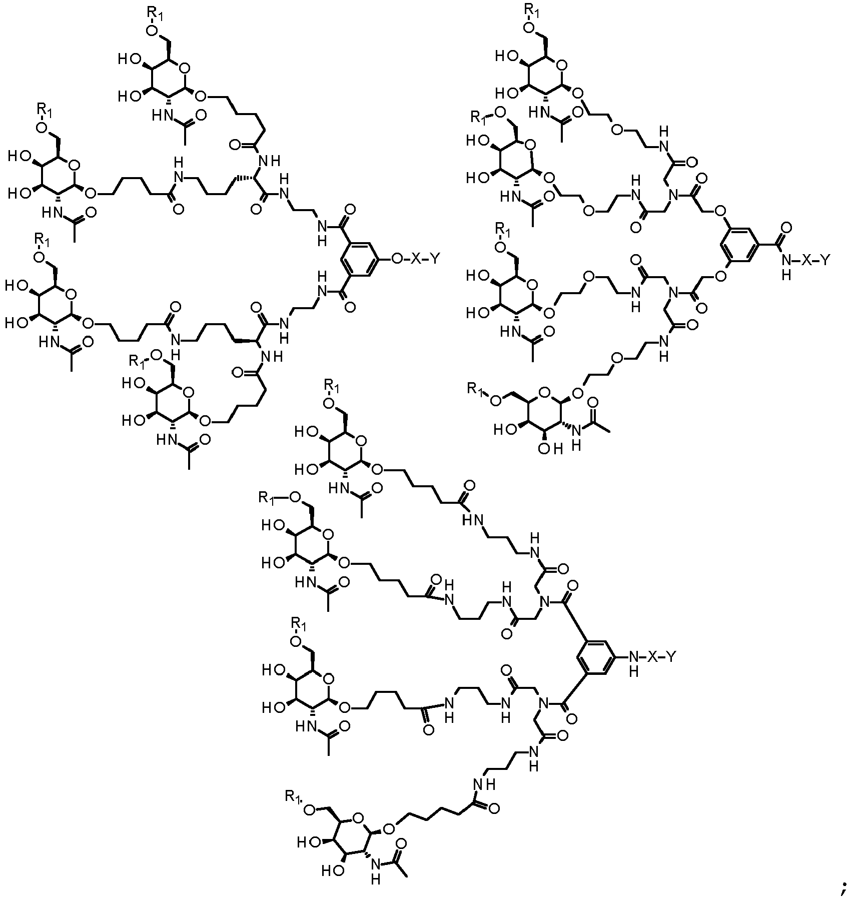 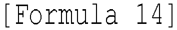 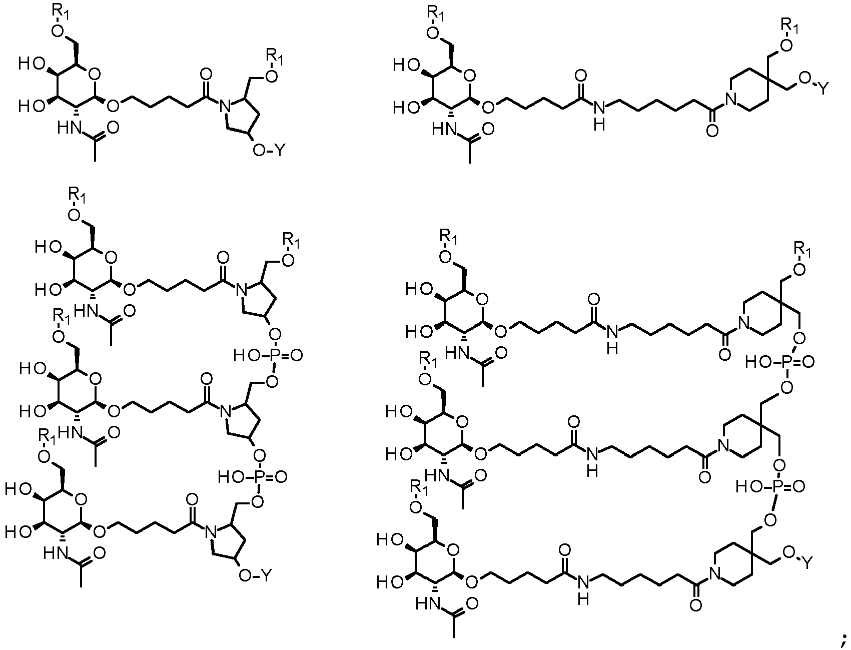 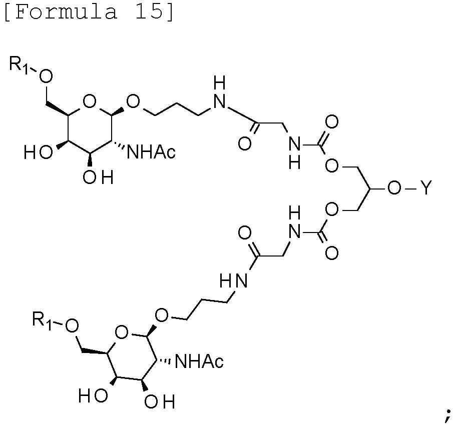 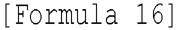 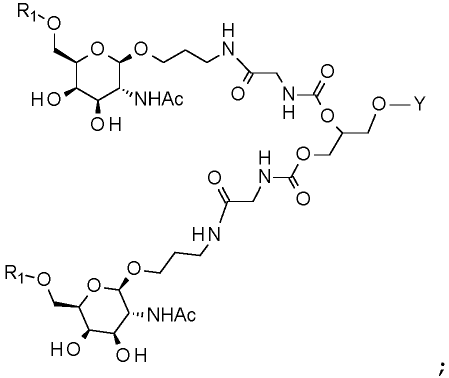 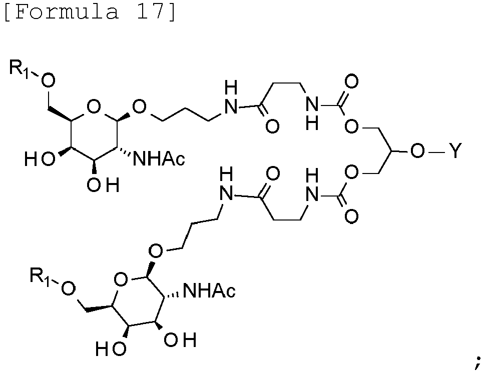 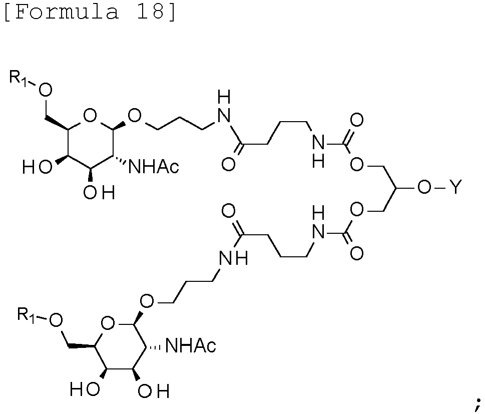 and

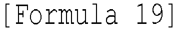

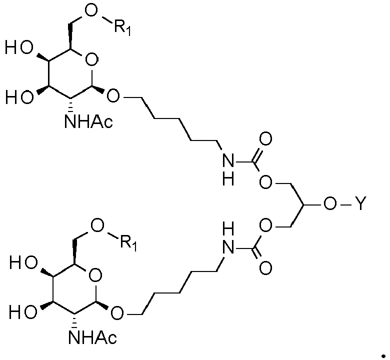

16. A compound represented by the following formula: 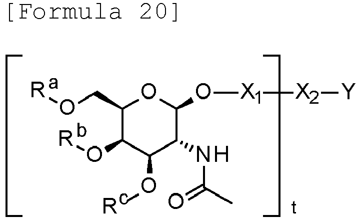 wherein at least one of R^{a}, R^{b}, and R^{c} is a hydrophobic protecting group, and the others are protecting groups to be removed under release conditions; X₁ and X₂ each independently represent a linker structure, which may be branched between X₁ and X₂; t is the number of branched chains in the linker structure and is an integer of 1 to 10; and Y represents a phosphoramidite group or an H-phosphonate group; or a salt thereof.

17. The compound or a salt thereof according to claim 16, wherein R^{a} is a hydrophobic protecting group to be removed under acidic conditions, R^{b} and R^{c} are each an acetyl group, Y represents a phosphoramidite group or an H-phosphonate group, and t is an integer of 1 to 3.

18. The compound or a salt thereof according to claim 16 or 17, represented by any one of the following formulas: 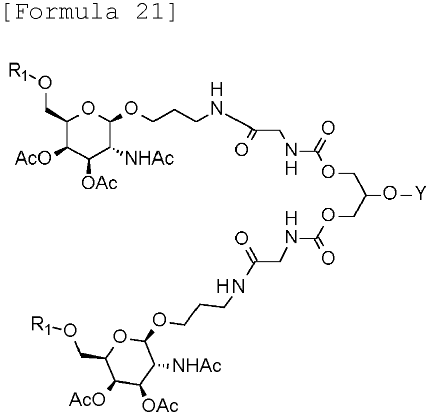 wherein R₁ is a hydrophobic protecting group to be removed under acidic conditions, and Y represents a phosphoramidite group or an H-phosphonate group, and the same applies below: 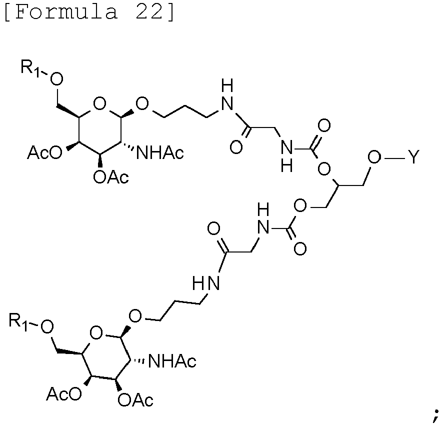 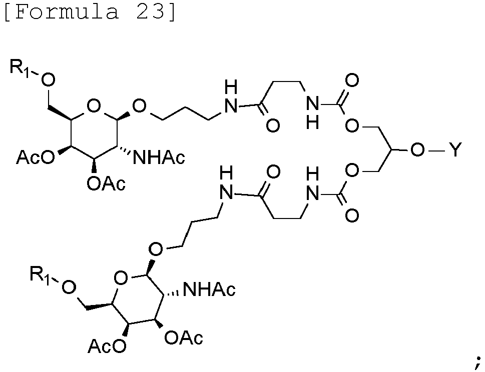 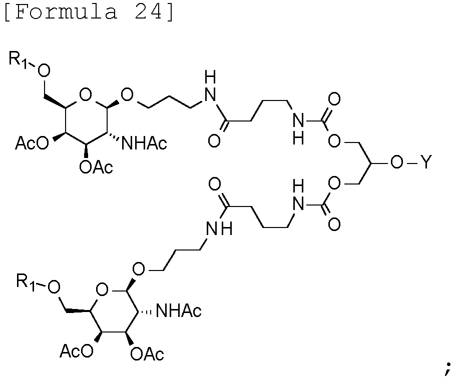 and

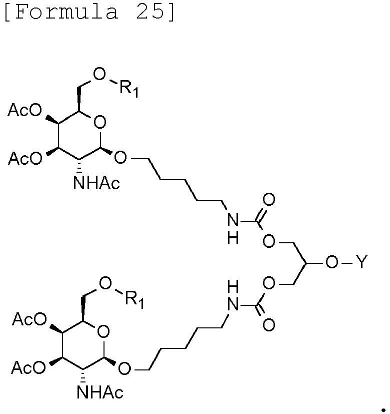

19. The compound or a salt thereof according to claim 16 or 17, represented by any one of the following formulas: 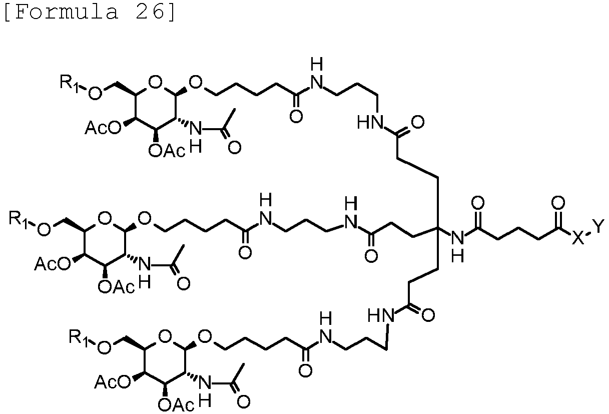 wherein R₁ represents a hydrophobic protecting group to be removed under acidic conditions, X represents a linker structure, and Y represents a phosphoramidite group or an H-phosphonate group, and the same applies below: 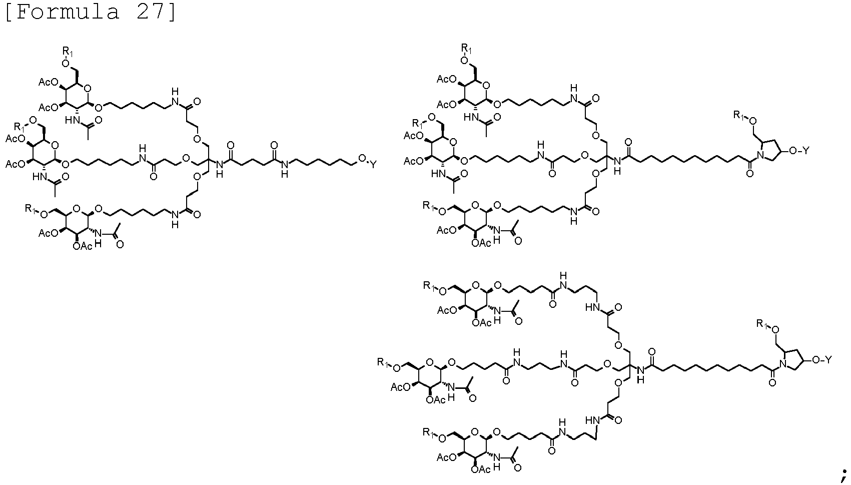 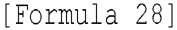 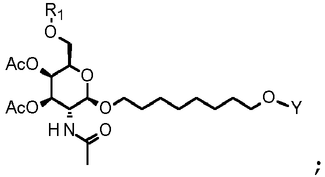 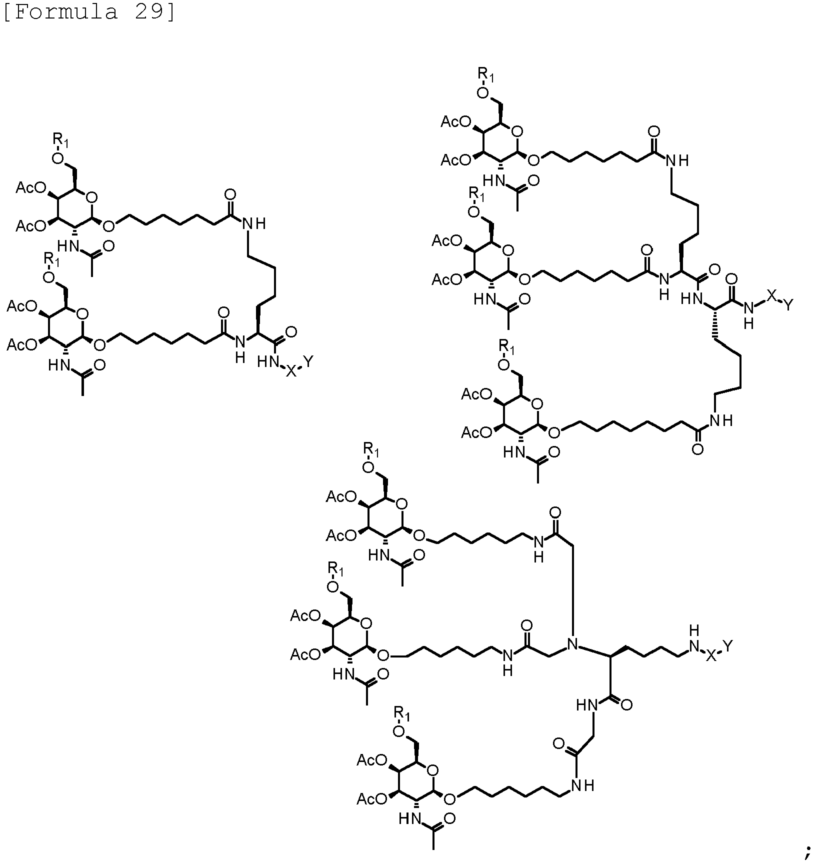 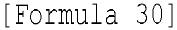 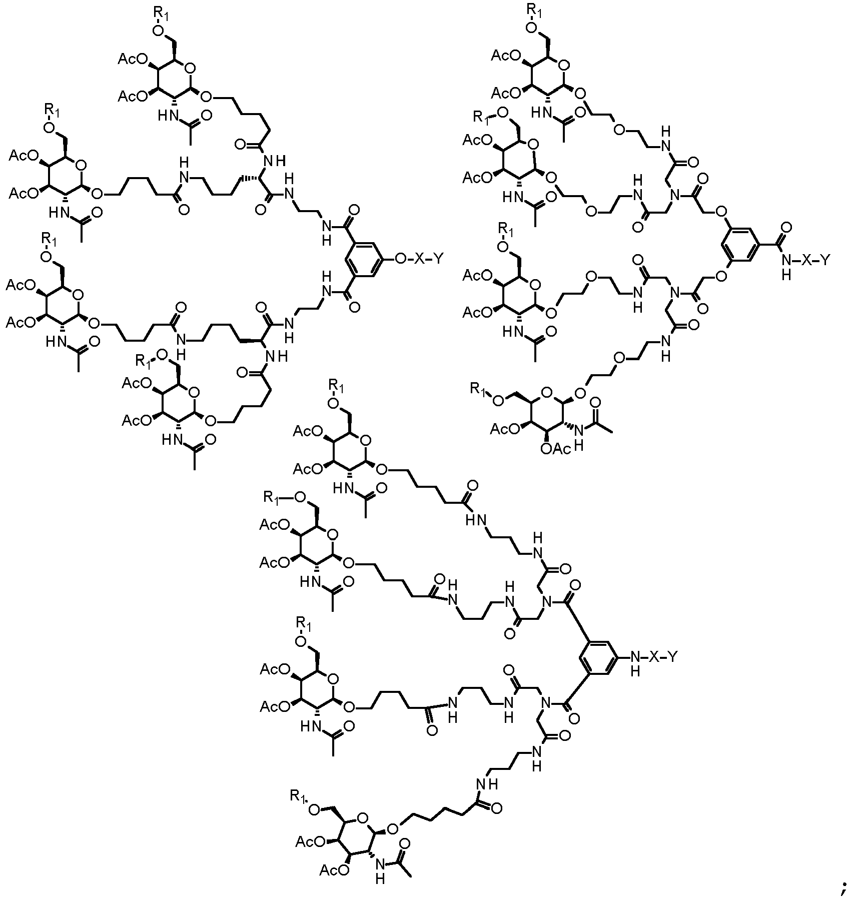 and

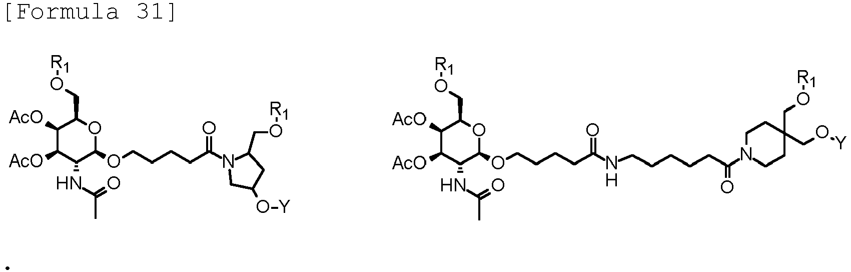

20. The compound or a salt thereof according to any one of claims 16 to 19, wherein the hydrophobic protecting group is an optionally substituted trityl group, and the phosphoramidite group is -P(OCH₂CH₂CN)N(CH(CH₃)₂)₂ or -P(OCH₃)N(CH(CH₃)₂)₂.

21. A conjugate of a structure comprising a hydrophobic protecting group-containing GalNAc unit-phosphate group represented by the following formula bound to the 5' end or the 3' end of an oligonucleotide: 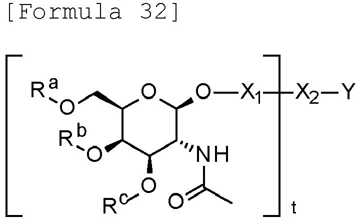 wherein at least one of R^{a}, R^{b}, and R^{c} is a hydrophobic protecting group, and the others are hydrogen atoms or protecting groups to be removed under release conditions; X₁ and X₂ each independently represent a linker structure, which may be branched between X₁ and X₂; t is the number of branched chains in the linker structure and is an integer of 1 to 10; and Y represents a bond which is bound to the oligonucleotide via a phosphate group or a thiophosphate group; or a salt thereof.

22. The conjugate or a salt thereof according to claim 21, wherein the structure comprising a hydrophobic protecting group-containing GalNAc unit-phosphate group is represented by any one of the following formulas: 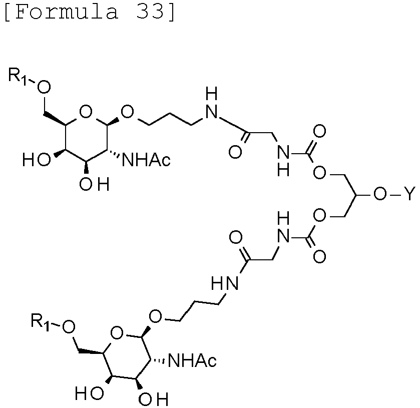 wherein R₁ represents a hydrophobic protecting group to be removed under acidic conditions; Y represents a bond which is bound to the oligonucleotide via a phosphate group or a thiophosphate group, and the same applies below: 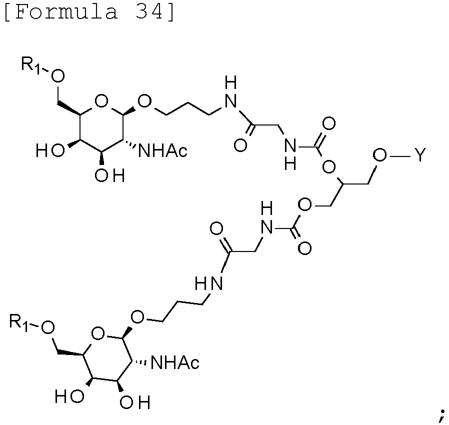 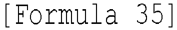 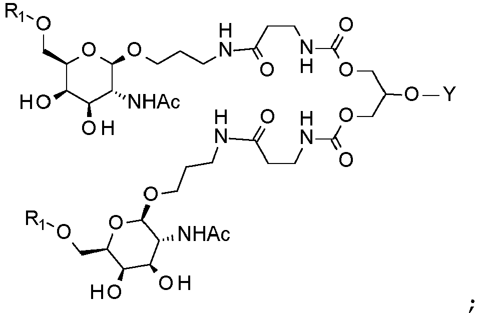 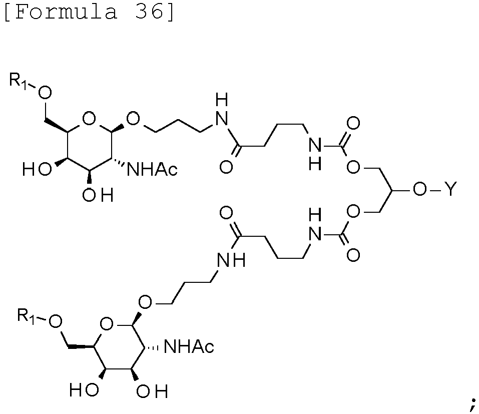 and

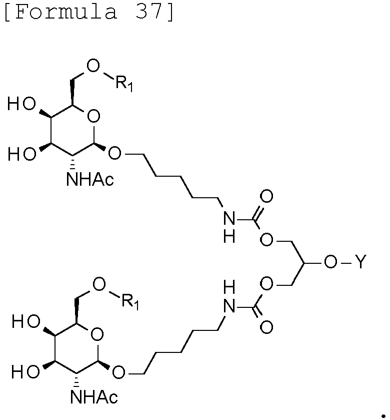

23. The conjugate or a salt thereof according to claim 21, wherein the structure comprising a hydrophobic protecting group-containing GalNAc unit-phosphate group is represented by any one of the following formulas: 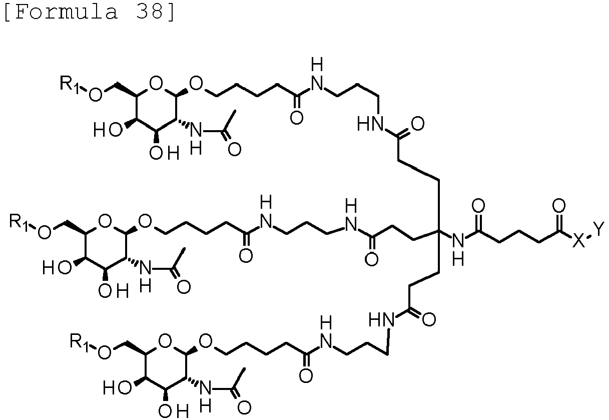 wherein R₁ represents a hydrophobic protecting group to be removed under acidic conditions, X represents a linker structure, Y represents a bond which is bound to the oligonucleotide via a phosphate group or a thiophosphate group, and the same applies below: 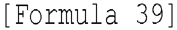 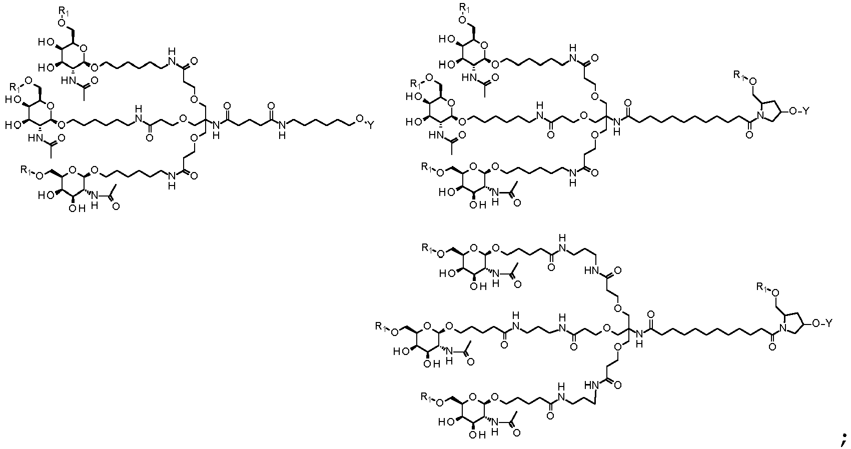 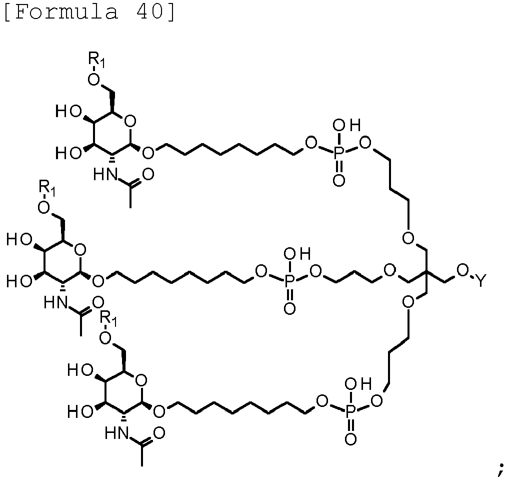 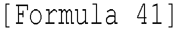 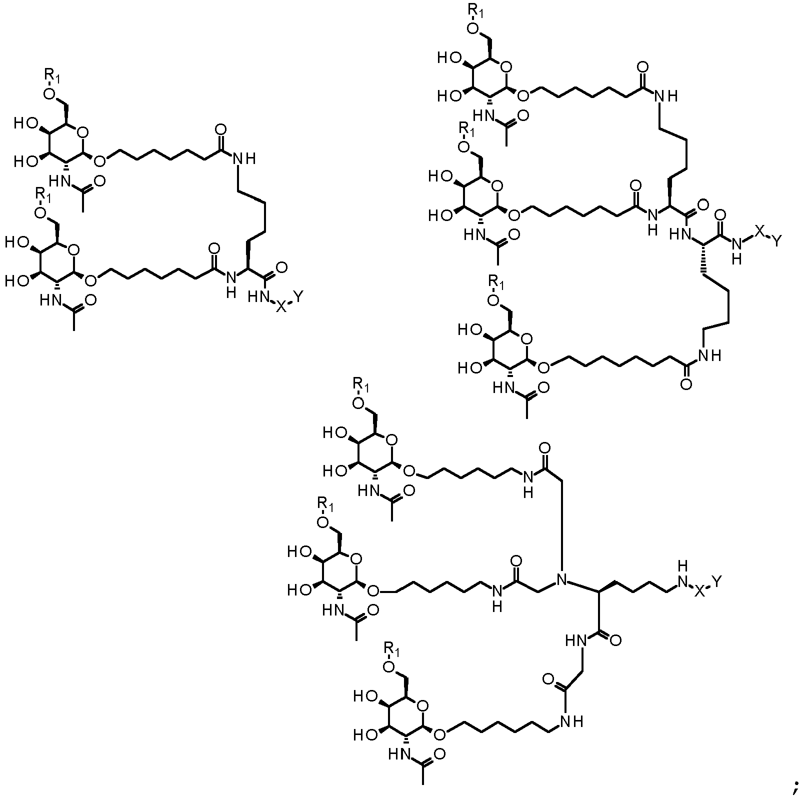 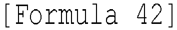 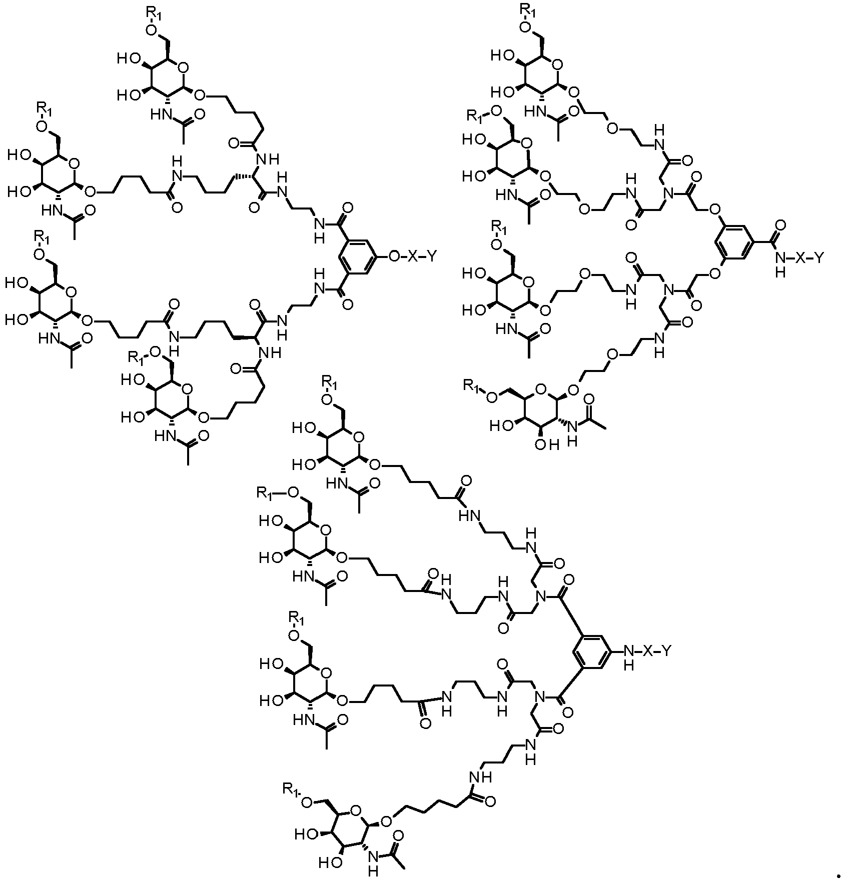 ; and

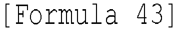

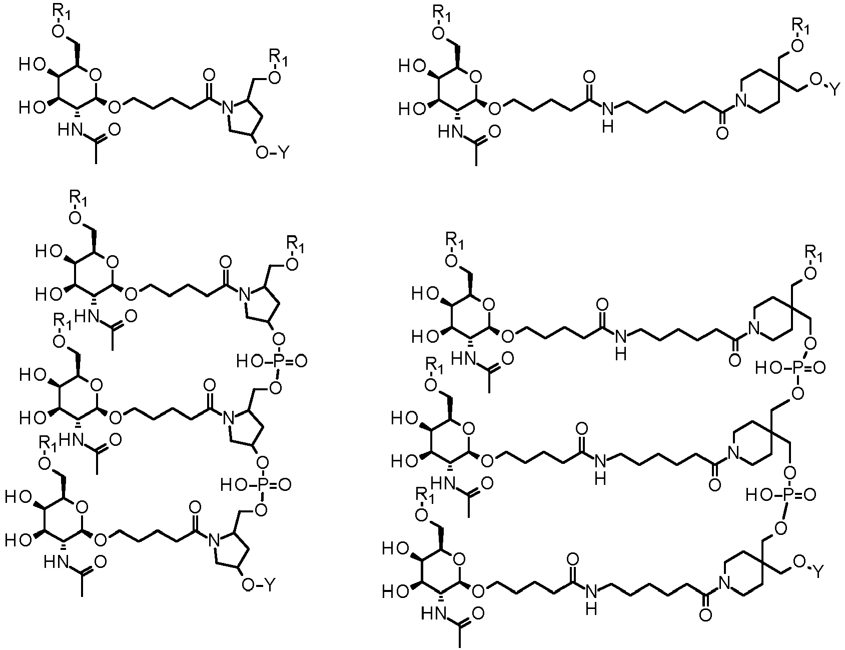

24. The conjugate or a salt thereof according to any one of claims 21 to 23, wherein the hydrophobic protecting group is an optionally substituted trityl group, and Y represents a bond which is bound to the 5'-hydroxyl group of the nucleoside located at the 5' end of the oligonucleotide via a phosphodiester bond or a phosphate thioate bond.


SEQUENCE LISTING

<110>  DAIICHI SANKYO COMPANY, LIMITED

<120>  Conjugate of GalNAc-Oligonucleotide For Delivery To Liver and
Manufacturing Method Thereof

<130>  DSPCT-FP2020

<150>  JP2019-164564
<151>  2019-09-10

<160>  96

<170>  PatentIn version 3.5

<210>  1
<211>  21
<212>  DNA
<213>  Artificial Sequence

<220>
<223>  antisense oligonucleotide

<400>  1
agataaaauc cgauggcgaa g                                                21


<210>  2
<211>  21
<212>  DNA
<213>  Artificial Sequence

<220>
<223>  antisense oligonucleotide

<400>  2
taaaauccga uggcgaagcu g                                                21


<210>  3
<211>  21
<212>  DNA
<213>  Artificial Sequence

<220>
<223>  antisense oligonuleotide

<400>  3
aauccgaugg cgaagcugaa a                                                21


<210>  4
<211>  21
<212>  DNA
<213>  Artificial Sequence

<220>
<223>  antisense oligonucleotide

<400>  4
ccgauggcga agcugaaaag g                                                21

<210>  5
<211>  21
<212>  DNA
<213>  Artificial Sequence

<220>
<223>  antisense oligonucleotide

<400>  5
auggcgaagc ugaaaaggaa g                                                21


<210>  6
<211>  21
<212>  DNA
<213>  Artificial Sequence

<220>
<223>  antisense oligonucleotide

<400>  6
gcgaagcuga aaaggaagaa g                                                21


<210>  7
<211>  21
<212>  DNA
<213>  Artificial Sequence

<220>
<223>  oligonucleotide

<400>  7
aagcugaaaa ggaagaaggu a                                                21


<210>  8
<211>  21
<212>  DNA
<213>  Artificial Sequence

<220>
<223>  antisense oligonucleotide

<400>  8
cugaaaagga agaagguaau g                                                21


<210>  9
<211>  21
<212>  DNA
<213>  Artificial Sequence

<220>
<223>  antisense oligonucleotide

<400>  9
aaaaggaaga agguaaugag a                                                21


<210>  10
<211>  21

<212>  DNA
<213>  Artificial Sequence

<220>
<223>  antisense oligonucleotide

<400>  10
aggaagaagg uaaugagaaa a                                                21


<210>  11
<211>  21
<212>  DNA
<213>  Artificial Sequence

<220>
<223>  antisense oligonucleotide

<400>  11
aagaagguaa ugagaaaata t                                                21


<210>  12
<211>  21
<212>  DNA
<213>  Artificial Sequence

<220>
<223>  antisense oligonucleotide

<400>  12
gauaaaaucc gatggcgaag c                                                21


<210>  13
<211>  21
<212>  DNA
<213>  Artificial Sequence

<220>
<223>  antisense oligonucleotide

<400>  13
auaaaatccg auggcgaagc t                                                21


<210>  14
<211>  21
<212>  DNA
<213>  Artificial Sequence

<220>
<223>  antisense oligonucleotide

<400>  14
aaaauccgat ggcgaagctg a                                                21


<210>  15
<211>  21
<212>  DNA
<213>  Artificial Sequence

<220>
<223>  antisense oligonucleotide

<400>  15
aaatccgaug gcgaagcuga a                                                21


<210>  16
<211>  21
<212>  DNA
<213>  Artificial Sequence

<220>
<223>  antisense oligonucleotide

<400>  16
auccgatggc gaagctgaaa a                                                21


<210>  17
<211>  21
<212>  DNA
<213>  Artificial Sequence

<220>
<223>  antisense oligonucleotide

<400>  17
tccgauggcg aagcugaaaa g                                                21


<210>  18
<211>  21
<212>  DNA
<213>  Artificial Sequence

<220>
<223>  antisense oligonucleotide

<400>  18
cgatggcgaa gctgaaaagg a                                                21


<210>  19
<211>  21
<212>  DNA
<213>  Artificial Sequence

<220>
<223>  antisense oligonucleotide

<400>  19
gauggcgaag cugaaaagga a                                                21


<210>  20
<211>  18
<212>  DNA
<213>  Artificial Sequence

<220>
<223>  antisense oligonucleotide

<400>  20
uaaaauccga tggcgaag                                                    18


<210>  21
<211>  18
<212>  DNA
<213>  Artificial Sequence

<220>
<223>  antisense oligonucleotide

<400>  21
aaaatccgau ggcgaagc                                                    18


<210>  22
<211>  18
<212>  DNA
<213>  Artificial Sequence

<220>
<223>  antisense oligonucleotide

<400>  22
aaauccgaug gcgaagcu                                                    18


<210>  23
<211>  18
<212>  DNA
<213>  Artificial Sequence

<220>
<223>  antisense oligonucleotide

<400>  23
aauccgatgg cgaagctg                                                    18


<210>  24
<211>  18
<212>  DNA
<213>  Artificial Sequence

<220>
<223>  antisense oligonucleotide

<400>  24
atccgauggc gaagcuga                                                    18


<210>  25
<211>  18
<212>  DNA
<213>  Artificial Sequence

<220>
<223>  antisense oligonucleotide

<400>  25
uccgauggcg aagcugaa                                                    18

<210>  26
<211>  18
<212>  DNA
<213>  Artificial Sequence

<220>
<223>  antisense oligonucleotide

<400>  26
ccgatggcga agctgaaa                                                    18


<210>  27
<211>  18
<212>  DNA
<213>  Artificial Sequence

<220>
<223>  antisense oligonucleotide

<400>  27
cgauggcgaa gcugaaaa                                                    18


<210>  28
<211>  18
<212>  DNA
<213>  Artificial Sequence

<220>
<223>  antisense oligonucleotide

<400>  28
gauggcgaag cugaaaag                                                    18


<210>  29
<211>  18
<212>  DNA
<213>  Artificial Sequence

<220>
<223>  antisense oligonucleotide

<400>  29
atggcgaagc tgaaaagg                                                    18


<210>  30
<211>  18
<212>  DNA
<213>  Artificial Sequence

<220>
<223>  antisense oligonucleotide

<400>  30
uggcgaagcu gaaaagga                                                    18


<210>  31
<211>  18

<212>  DNA
<213>  Artificial Sequence

<220>
<223>  antisense oligonucleotide

<400>  31
ggcgaagcug aaaaggaa                                                    18


<210>  32
<211>  18
<212>  DNA
<213>  Artificial Sequence

<220>
<223>  antisense oligonucleotide

<400>  32
gcgaagctga aaaggaag                                                    18


<210>  33
<211>  21
<212>  DNA
<213>  Artificial Sequence

<220>
<223>  antisense oligonucleotide

<400>  33
agataaaauc cgauggcgaa g                                                21


<210>  34
<211>  21
<212>  DNA
<213>  Artificial Sequence

<220>
<223>  antisense oligonucleotide

<400>  34
gcagauaaaa uccgatggcg a                                                21


<210>  35
<211>  21
<212>  DNA
<213>  Artificial Sequence

<220>
<223>  antisense oligonucleotide

<400>  35
cagauaaaat ccgauggcga a                                                21


<210>  36
<211>  18
<212>  DNA
<213>  Artificial Sequence

<220>
<223>  antisense oligonucleotide

<400>  36
cagataaaau ccgauggc                                                    18


<210>  37
<211>  18
<212>  DNA
<213>  Artificial Sequence

<220>
<223>  antisense oligonucleotide

<400>  37
agauaaaauc cgatggcg                                                    18


<210>  38
<211>  18
<212>  DNA
<213>  Artificial Sequence

<220>
<223>  antisense oligonucleotide

<400>  38
gauaaaatcc gauggcga                                                    18


<210>  39
<211>  18
<212>  DNA
<213>  Artificial Sequence

<220>
<223>  antisense oligonucleotide

<400>  39
ataaaauccg auggcgaa                                                    18


<210>  40
<211>  15
<212>  DNA
<213>  Artificial Sequence

<220>
<223>  antisense oligonucleotide

<400>  40
aauccgatgg cgaag                                                       15


<210>  41
<211>  15
<212>  DNA
<213>  Artificial Sequence

<220>
<223>  antisense oligonucleotide

<400>  41
ccgatggcga agctg                                                        15


<210>  42
<211>  16
<212>  DNA
<213>  Artificial Sequence

<220>
<223>  antisense oligonucleotide

<400>  42
aaauccgatg gcgaag                                                       16


<210>  43
<211>  18
<212>  DNA
<213>  Artificial Sequence

<220>
<223>  antisense oligonucleotide

<400>  43
tcaaauccga tggcgaag                                                     18


<210>  44
<211>  15
<212>  DNA
<213>  Artificial Sequence

<220>
<223>  antisense oligonucleotide

<400>  44
atccgauggc gaagc                                                        15


<210>  45
<211>  15
<212>  DNA
<213>  Artificial Sequence

<220>
<223>  antisense oligonucleotide

<400>  45
uccgauggcg aagcu                                                        15


<210>  46
<211>  17
<212>  DNA
<213>  Artificial Sequence

<220>
<223>  antisense oligonucleotide

<400>  46
aaaauccgat ggcgaag                                                      17

<210>  47
<211>  16
<212>  DNA
<213>  Artificial Sequence

<220>
<223>  antisense oligonucleotide

<400>  47
aauccgatgg cgaagc                                                        16


<210>  48
<211>  16
<212>  DNA
<213>  Artificial Sequence

<220>
<223>  antisense oligonucleotide

<400>  48
auccgatggc gaagcu                                                        16


<210>  49
<211>  25
<212>  DNA
<213>  Artificial Sequence

<220>
<223>  primer

<400>  49
atagcagagc aatcaccacc aagcc                                              25


<210>  50
<211>  25
<212>  DNA
<213>  Artificial Sequence

<220>
<223>  primer

<400>  50
attccacgac ggcagaatgg atggc                                              25


<210>  51
<211>  39
<212>  DNA
<213>  Artificial Sequence

<220>
<223>  primer

<400>  51
taccgagctc ggatccacca ccaagcctgg aataactgc                               39


<210>  52
<211>  39

<212>  DNA
<213>  Artificial Sequence

<220>
<223>  primer

<400>  52
ctggactagt ggatcctggc atggttgttg actttaaac                              39


<210>  53
<211>  25
<212>  DNA
<213>  Artificial Sequence

<220>
<223>  primer

<400>  53
tctgggctgt gcagctgaat gtctg                                             25


<210>  54
<211>  25
<212>  DNA
<213>  Artificial Sequence

<220>
<223>  primer

<400>  54
gtaggggatg acactgacgg atgcc                                             25


<210>  55
<211>  23
<212>  DNA
<213>  Artificial Sequence

<220>
<223>  primer

<400>  55
ctggagtcct gtcaggtatg ggc                                               23


<210>  56
<211>  25
<212>  DNA
<213>  Artificial Sequence

<220>
<223>  primer

<400>  56
agctgaaaag gaagaaggta atgag                                             25


<210>  57
<211>  25
<212>  DNA
<213>  Artificial Sequence

<220>
<223>  primer

<400>  57
tcttcctttt cagcttcgcc atcgg                                          25


<210>  58
<211>  22
<212>  DNA
<213>  Artificial Sequence

<220>
<223>  primer

<400>  58
ctgacaggac tccagcaaca ac                                             22


<210>  59
<211>  20
<212>  DNA
<213>  Artificial Sequence

<220>
<223>  primer

<400>  59
ttgtggttgg gattctgggc                                                20


<210>  60
<211>  20
<212>  DNA
<213>  Artificial Sequence

<220>
<223>  primer

<400>  60
atgctgtgga tgtggctgaa                                                20


<210>  61
<211>  19
<212>  DNA
<213>  Artificial Sequence

<220>
<223>  primer

<400>  61
tggcacccag cacaatgaa                                                 19


<210>  62
<211>  25
<212>  DNA
<213>  Artificial Sequence

<220>
<223>  primer

<400>  62
ctaagtcata gtccgcctag aagca                                          25


<210>  63
<211>  24
<212>  DNA
<213>  Artificial Sequence

<220>
<223>  primer

<400>  63
gctgctcatt ttcctcatca agtt                                           24


<210>  64
<211>  24
<212>  DNA
<213>  Artificial Sequence

<220>
<223>  primer

<400>  64
tggatgtggc tgaaagtttc tgta                                           24


<210>  65
<211>  16
<212>  DNA
<213>  Artificial Sequence

<220>
<223>  primer

<400>  65
tcctgtcagg cattgc                                                    16


<210>  66
<211>  20
<212>  PRT
<213>  Artificial Sequence

<220>
<223>  peptide

<400>  66

Trp Cys Glu Gln Pro Glu Trp Val His Ile Asp Thr Thr Pro Phe Ala
1               5                   10                  15


Ser Leu Leu Lys
            20


<210>  67
<211>  25
<212>  DNA
<213>  Artificial Sequence

<220>
<223>  primer

<400>  67
gggaaacatg catgaagccc tgggc                                          25


<210>  68
<211>  25
<212>  DNA
<213>  Artificial Sequence

<220>
<223>  primer

<400>  68
tcccttggta cctcaggaag ctgcc                                          25


<210>  69
<211>  37
<212>  DNA
<213>  Artificial Sequence

<220>
<223>  primer

<400>  69
cgggcccccc ctcgaaaact aggcctgaag agatggc                             37


<210>  70
<211>  39
<212>  DNA
<213>  Artificial Sequence

<220>
<223>  primer

<400>  70
taccgtcgac ctcgagggtt ggccttgatc cctctgcta                           39


<210>  71
<211>  25
<212>  DNA
<213>  Artificial Sequence

<220>
<223>  primer

<400>  71
ggttgagttg atcttctaca tcttg                                          25


<210>  72
<211>  25
<212>  DNA
<213>  Artificial Sequence

<220>
<223>  primer

<400>  72
gcaagagagc cttcaggtag atccc                                          25


<210>  73
<211>  40
<212>  DNA
<213>  Artificial Sequence

<220>
<223>  primer

<400>  73
agttctagag cggccgccca tgcaaaggac taggaacaac                          40


<210>  74
<211>  38
<212>  DNA
<213>  Artificial Sequence

<220>
<223>  primer

<400>  74
accgcggtgg cggccaatgt tgcctgtctt cctcaatc                            38


<210>  75
<211>  31
<212>  DNA
<213>  Artificial Sequence

<220>
<223>  primer

<400>  75
ggccaaccct ggaataactg caagggctct g                                   31


<210>  76
<211>  31
<212>  DNA
<213>  Artificial Sequence

<220>
<223>  primer

<400>  76
ttgcatggtt gttgacttta aacaccgaag a                                   31


<210>  77
<211>  30
<212>  DNA
<213>  Artificial Sequence

<220>
<223>  primer

<400>  77
tcaacaacca tgcaaaggac taggaacaac                                     30

<210>  78
<211>  30
<212>  DNA
<213>  Artificial Sequence

<220>
<223>  primer

<400>  78
attccagggt tggccttgat ccctctgcta                                        30


<210>  79
<211>  22
<212>  DNA
<213>  Artificial Sequence

<220>
<223>  primer

<400>  79
gggatcaagg ccaaccggct gg                                                22


<210>  80
<211>  23
<212>  DNA
<213>  Artificial Sequence

<220>
<223>  primer

<400>  80
taaagtcaac cgccatgcaa agg                                               23


<210>  81
<211>  20
<212>  DNA
<213>  Artificial Sequence

<220>
<223>  primer

<400>  81
tacgtcctct tccccatctg                                                   20


<210>  82
<211>  20
<212>  DNA
<213>  Artificial Sequence

<220>
<223>  primer

<400>  82
ctgacaggac tccagcaaca                                                   20


<210>  83
<211>  28

<212>  DNA
<213>  Artificial Sequence

<220>
<223>  primer

<400>  83
ttccttccaa agcagggact ctctatgt                                          28


<210>  84
<211>  28
<212>  DNA
<213>  Artificial Sequence

<220>
<223>  primer

<400>  84
cttgcagaag gacaagacgt agaagacc                                          28


<210>  85
<211>  28
<212>  DNA
<213>  Artificial Sequence

<220>
<223>  primer

<400>  85
gagtctatat tgagggcagg ctggagtc                                          28


<210>  86
<211>  28
<212>  DNA
<213>  Artificial Sequence

<220>
<223>  primer

<400>  86
tagtctgcct gctcactcaa cctctcct                                          28


<210>  87
<211>  28
<212>  DNA
<213>  Artificial Sequence

<220>
<223>  primer

<400>  87
gagtctatat tgagggcagg ctggagtc                                          28


<210>  88
<211>  28
<212>  DNA
<213>  Artificial Sequence

<220>
<223>  primer

<400>  88
taaatttgac caatgagcac tggaggtc                                          28


<210>  89
<211>  28
<212>  DNA
<213>  Artificial Sequence

<220>
<223>  primer

<400>  89
aaaatcatgt gtatgcgtgc ctttccta                                          28


<210>  90
<211>  20
<212>  DNA
<213>  Artificial Sequence

<220>
<223>  primer

<400>  90
ttgtggttgg gattctgggc                                                   20


<210>  91
<211>  20
<212>  DNA
<213>  Artificial Sequence

<220>
<223>  primer

<400>  91
tccagagtcc acaggaggtc                                                   20


<210>  92
<211>  20
<212>  DNA
<213>  Artificial Sequence

<220>
<223>  primer

<400>  92
gctgtgcagc tgaatgtctg                                                   20


<210>  93
<211>  15
<212>  DNA
<213>  Artificial Sequence

<220>
<223>  antisense oligonucleotide

<400>  93
aatccgatgg cgaag                                                        15


<210>  94
<211>  15
<212>  DNA
<213>  Artificial Sequence

<220>
<223>  antisense oligonucleotide

<400>  94
atccgatggc gaagc                                                        15


<210>  95
<211>  15
<212>  DNA
<213>  Artificial Sequence

<220>
<223>  antisense oligonucleotide

<400>  95
auccgauggc gaagc                                                        15


<210>  96
<211>  14
<212>  DNA
<213>  Artificial Sequence

<220>
<223>  Antisense-oligonucleotide sequence for ApoB mRNA

<400>  96
agcattggta ttca                                                         14